# EUROPEAN PATENT SPECIFICATION

(11) **EP 3 126 395 B1**
(45) Date of publication and mention of the grant of the patent: **24.07.2019**
(21) Application number: 15741892.2
(22) Date of filing: 01.04.2015
(51) Int. Cl.: C07K 16/28, C07K 16/22, C07K 16/24

(54) **MULTISPECIFIC ANTIBODIES**
MULTISPEZIFISCHE ANTIKÖRPER
ANTICORPS MULTI-SPÉCIFIQUES

(30) Priority: 02.04.2014 EP 14163165; 30.07.2014 EP 14179034
(43) Date of publication of application: 08.02.2017
(60) Divisional application: 19180274.3
(73) Proprietor: F. Hoffmann-La Roche AG, 4070 Basel (CH)
(72) Inventor: SCHAEFER, Wolfgang, 68199 Mannheim (DE); KLEIN, Christian, 8906 Bonstetten (CH); IMHOF-JUNG, Sabine, 82152 Planegg (DE); KLOSTERMANN, Stefan, 82061 Neuried (DE); MOLHOJ, Michael, 80686 Muenchen (DE); REGULA, Joerg Thomas, 81377 Muenchen (DE)
(74) Representative: Burger, Alexander
(86) International application number: PCT/EP2015/057165
(87) International publication number: WO 2015/150447

(56) References cited:
- WO-A1-2013/150043
- WO-A1-2013/174873
- WO-A1-2014/081955
- CHRISTIAN KLEIN ET AL: "Progress in overcoming the chain association issue in bispecific heterodimeric IgG antibodies", MABS, vol. 4, no. 6, 1 November 2012 (2012-11-01), pages 653-663, XP055106060, ISSN: 1942-0862, DOI: 10.4161/mabs.21379
- SEBASTIAN FENN ET AL: "Crystal Structure of an Anti-Ang2 CrossFab Demonstrates Complete Structural and Functional Integrity of the Variable Domain", PLOS ONE, vol. 8, no. 4, 1 April 2013 (2013-04-01), page e61953, XP055106055, ISSN: 1932-6203, DOI: 10.1371/journal.pone.0061953

## Description

The present invention relates to novel multispecific antibodies, their manufacture and use.

### Background of the Invention

Engineered proteins, such as bi- or multispecific antibodies capable of binding two or more antigens are known in the art. Such multispecific binding proteins can be generated using cell fusion, chemical conjugation, or recombinant DNA techniques.

A wide variety of recombinant multispecific antibody formats have been developed in the recent past, e.g. tetravalent bispecific antibodies by fusion of, e.g. an IgG antibody format and single chain domains (see e.g. Coloma, M.J., et. al., Nature Biotech. 15 (1997) 159-163; WO 2001/077342; and Morrison, S.L., Nature Biotech. 25 (2007) 1233-1234).

Also several other new formats wherein the antibody core structure (IgA, IgD, IgE, IgG or IgM) is no longer retained such as dia-, tria- or tetrabodies, minibodies, several single chain formats (scFv, Bis-scFv), which are capable of binding two or more antigens, have been developed (Holliger, P., et. al, Nature Biotech. 23 (2005) 1126-1136; Fischer, N., and Léger, O., Pathobiology 74 (2007) 3-14; Shen, J., et. al., J. Immunol. Methods 318 (2007) 65-74; Wu, C., et al., Nature Biotech. 25 (2007) 1290-1297).

All such formats use linkers either to fuse the antibody core (IgA, IgD, IgE, IgG or IgM) to a further binding protein (e.g. scFv) or to fuse e.g. two Fab fragments or scFv (Fischer, N., and Léger, O., Pathobiology 74 (2007) 3-14). While it is obvious that linkers have advantages for the engineering of bispecific antibodies, they may also cause problems in therapeutic settings. Indeed, these foreign peptides might elicit an immune response against the linker itself or the junction between the protein and the linker. Furthermore, the flexible nature of these peptides makes them more prone to proteolytic cleavage, potentially leading to poor antibody stability, aggregation and increased immunogenicity. In addition one may want to retain effector functions, such as e.g. complement-dependent cytotoxicity (CDC) or antibody dependent cellular cytotoxicity (ADCC), which are mediated through the Fc-part by maintaining a high degree of similarity to naturally occurring antibodies. Thus, ideally, one should aim at developing bispecific antibodies that are very similar in general structure to naturally occurring antibodies (like IgA, IgD, IgE, IgG or IgM) with minimal deviation from human sequences.

In one approach bispecific antibodies that are very similar to natural antibodies have been produced using the quadroma technology (see Milstein, C., and Cuello, A.C., Nature 305 (1983) 537-540) based on the somatic fusion of two different hybridoma cell lines expressing murine monoclonal antibodies with the desired specificities of the bispecific antibody. Because of the random pairing of two different antibody heavy and light chains within the resulting hybrid-hybridoma (or quadroma) cell line, up to ten different antibody species are generated of which only one is the desired, functional bispecific antibody. Due to the presence of mispaired byproducts, and significantly reduced production yields, sophisticated purification procedures are required (see e.g. Morrison, S.L., Nature Biotech. 25 (2007) 1233-1234). In general the same problem of mispaired by-products remains if recombinant expression techniques are used.

An approach to circumvent the problem of mispaired byproducts, which is known as 'knobs-into-holes', aims at forcing the pairing of two different antibody heavy chains by introducing mutations into the CH3 domains to modify the contact interface. On one chain bulky amino acids were replaced by amino acids with short side chains to create a 'hole'. Conversely, amino acids with large side chains were introduced into the other CH3 domain, to create a 'knob'. By coexpressing these two heavy chains (and two identical light chains, which have to be appropriate for both heavy chains), high yields of heterodimer formation ('knob-hole') versus homodimer formation ('hole-hole' or 'knob-knob') was observed (Ridgway, J.B., et al., Protein Eng. 9 (1996) 617-621; and WO 96/027011). The percentage of heterodimer could be further increased by remodeling the interaction surfaces of the two CH3 domains using a phage display approach and the introduction of a disulfide bridge to stabilize the heterodimers (Merchant, A.M., et al., Nature Biotech. 16 (1998) 677-681; Atwell, S., et al., J. Mol. Biol. 270 (1997) 26-35). New approaches for the knobs-into-holes technology are described in e.g. in EP 1 870 459 A1. Although this format appears very attractive, no data describing progression towards the clinic are currently available. One important constraint of this strategy is that the light chains of the two parent antibodies have to be identical to prevent mispairing and formation of inactive molecules. Thus this technique is not appropriate as a basis for easily developing recombinant, tri-or tetraspecific antibodies against three or four antigens starting from two antibodies against the first and the second antigen, as either the heavy chains of these antibodies and/or the identical light chains have to be optimized first and then further antigen binding peptides against the third and fourth antigen have to be added.

WO 2006/093794 relates to heterodimeric protein binding compositions. WO 99/37791 describes multipurpose antibody derivatives. Morrison, S.L., et al., J. Immunol. 160 (1998) 2802-2808 refers to the influence of variable region domain exchange on the functional properties of IgG.

WO 2013/02362 relates to heterodimerized polypeptides. WO 2013/12733 relates to polypeptides comprising heterodimeric Fc regions. WO 2012/131555 relates to engineered hetero-dimeric immunoglobulins. EP 2647707 relates to engineered hetero-dimeric immunoglobulins.

WO 2013/026835 relates to bispecific, Fc free antibodies with a domain crossover. WO 2009/080251, WO 2009/080252, WO 2009/080253, WO 2009/080254 and Schaefer, W. et al, PNAS, 108 (2011) 11187-1191 relate to bivalent, bispecific IgG antibodies with a domain crossover.

The multispecific antibodies with VH/VL replacement/exchange in one binding to prevent light chain mispairing (CrossMab^{VH-VL}) which are described in WO2009/080252, (see also Schaefer, W. et al, PNAS, 108 (2011) 11187-1191) clearly reduce the byproducts caused by the mismatch of a light chain against a first antigen with the wrong heavy chain against the second antigen (compared to approaches without such domain exchange). However their preparation is not completely free of side products. The main side product is based on a Bence-Jones -type interaction -see also Schaefer, W. et al, PNAS, 108 (2011) 11187-1191; in Fig. S1I of the Supplement; and Klein, C. et al, MABS, vol. 4 no. 6 (2012) 653-663).

Therefore there is still a need for further reduction of such side products improve e.g. the yield of such bispecific antibodies.

### Summary of the Invention

The invention relates to a multispecific antibody of human IgG1, human IgG2, human IgG3 or human IgG4 subclass, comprising:
a) the first light chain and the first heavy chain of a first antibody which specifically binds to a first antigen; and
b) the second light chain and the second heavy chain of a second antibody which specifically binds to a second antigen, and wherein the variable domains VL and VH in the second light chain and second heavy chain of the second antibody are replaced by each other; and
wherein in the constant domain CL of the first light chain under a) the amino acid at position 124 is substituted independently by lysine (K) or arginine (R), and wherein in the constant domain CH1 of the first heavy chain under a) the amino acid at position 147 or the amino acid at position 213 is substituted independently by glutamic acid (E) or aspartic acid (D) (numbering according to Kabat EU index).

A further embodiment of the invention is a method for the preparation of a multispecific antibody according to the invention
comprising the steps of
A) transforming a host cell with vectors comprising nucleic acid molecules encoding
   a) the first light chain and the first heavy chain of a first antibody which specifically binds to a first antigen; and
   b) the second light chain and the second heavy chain of a second antibody which specifically binds to a second antigen, wherein the variable domains VL and VH in the second light chain and second heavy chain of the second antibody are replaced by each other; and
   wherein in the constant domain CL of the first light chain under a) the amino acid at position 124 is substituted independently by lysine (K) or arginine (R), and wherein in the constant domain CH1 of the first heavy chain under a) the amino acid at position 147 or the amino acid at position 213 is substituted independently by glutamic acid (E) or aspartic acid (D) (numbering according to Kabat EU index).
B) culturing the host cell under conditions that allow synthesis of said antibody molecule; and
C) recovering said antibody molecule from said culture.

A further embodiment of the invention is a nucleic acid encoding the amino acid sequences of a multispecific antibody according to the invention.

A further embodiment of the invention are expression vectors containing the nucleic acid according to the invention capable of expressing said nucleic acid in a host cell.

A further embodiment of the invention is a host cell comprising a vector according to the invention.

A further embodiment of the invention is a composition, e.g. a pharmaceutical or a diagnostic composition, of the antibody according to the invention.

A further embodiment of the invention is a pharmaceutical composition comprising an antibody according to the invention and at least one pharmaceutically acceptable excipient.

A further embodiment of the invention is a method for the treatment of a patient in need of therapy, characterized by administering to the patient a therapeutically effective amount of an antibody according to the invention.

According to the invention, the ratio of a desired multispecific antibody compared to undesired main side Bence Jones-type products can be improved by the introduction of substitutions of charged amino acids with the opposite charges at specific amino acid positions in the CH1 and CL domains.

### Description of the Figures

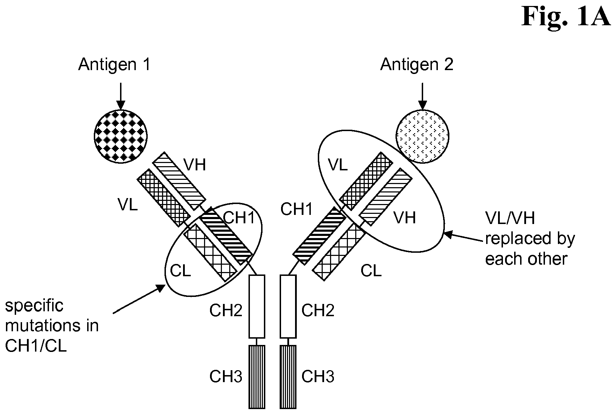

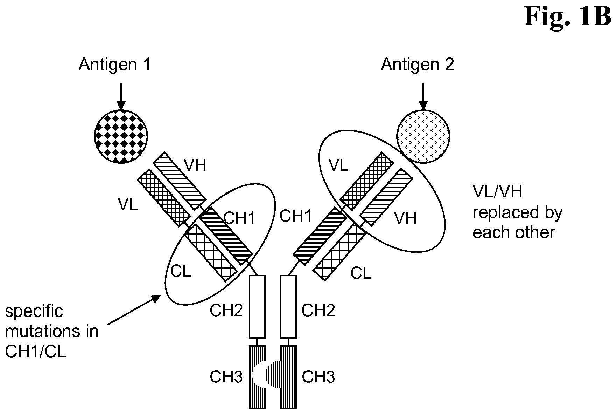

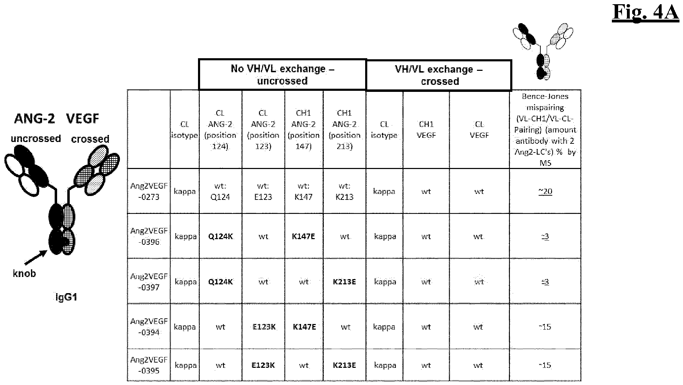

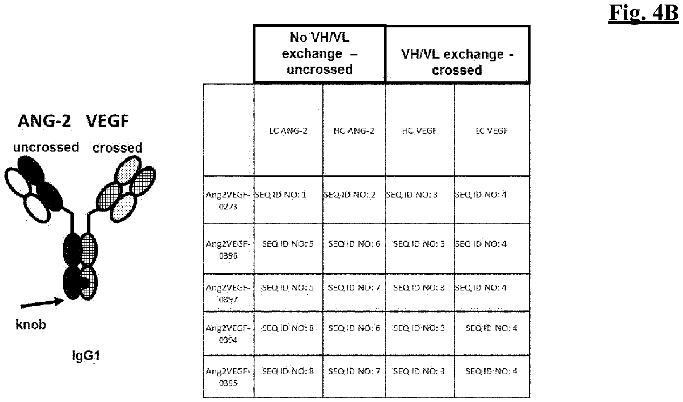

- **Figure 1**: Some examples of multispecific antibodies according to the invention with VH/VL domain replacement in one antibody binding arm and specific mutations in one CH1/CL domain interface:
at least the amino acid at position 124 of the CL domain is substituted independently by lysine (K), arginine (R) or Histidine (H) (numbering according to Kabat), and at least the amino acid at position 147 of the CH1 domain or the amino acid at position 213 of the CH1 domain is substituted independently by glutamic acid (E), or aspartic acid (D) (numbering according to Kabat EU index).
**Figure 1A****:** VH/VL domain replacement in one antibody binding arm and specific mutations in the CH1/CL domain interface of the other antibody binding arm.
**Figure 1B****:** VH/VL domain replacement in one antibody binding arm and specific mutations in the CH1/CL domain interface of the other antibody binding arm, and modifications of the CH3/CH3 domain interface to enforce heavy chain heterodimerization (like e.g. knobs-into-holes technology or alternative heterodimerization technologies like e.g. substitution of charged amino acids with their respective opposite charge).

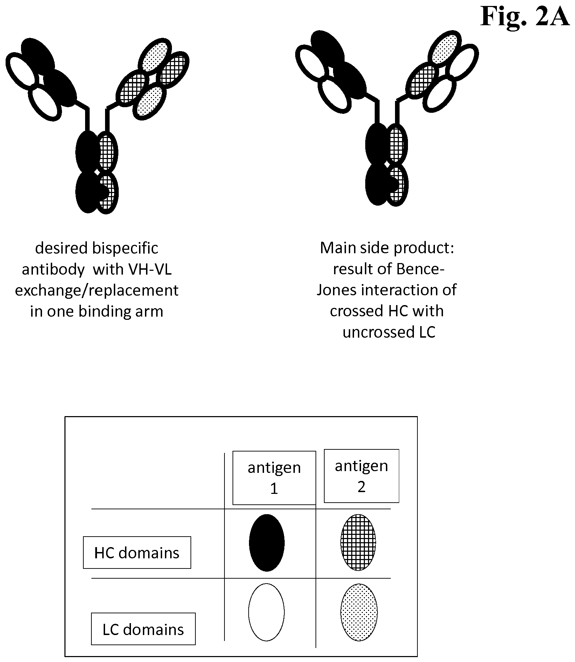

- **Figure 2A**: Example of multispecific antibody with VH/VL domain replacement in one antibody binding arm and **without** mutations in one CH1/CL domain interface (left side) and the main side product of this multispecific antibody (due to VL-VL Bence jones-type domain interaction). Other possible variants as potential side products were not detected neither by mass spectrometry directly; nor by mass spectrometry after plasmin or LysC digestion by analyzing the Fab fragments thereof.

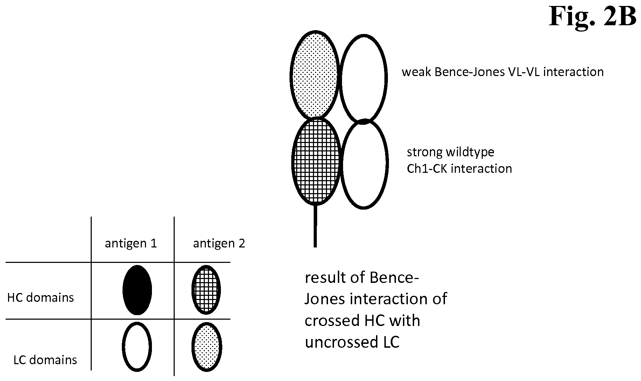

- **Figure 2B**: Origin of the main side product of multispecific antibody with VH/VL domain replacement in one antibody binding arm and **without** mutations in one CH1/CL domain interface (due to VL-VL Bence jones-type domain interaction).

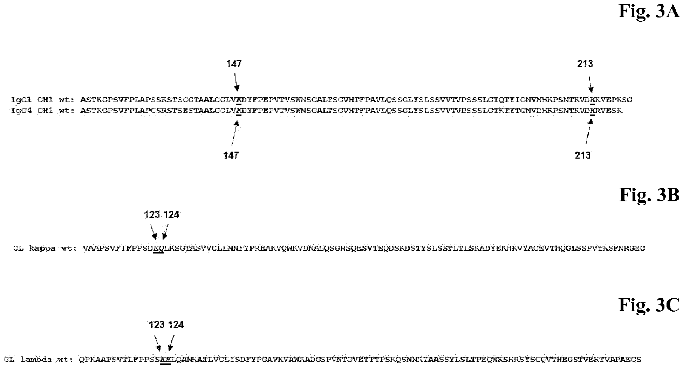

- **Figure 3**: **Figure 3A****:** wild type (wt) amino acid sequences in CH1 domain (two IgG isotypes are shown) with underlined and highlighted amino acid positions 147 and 213 (numbering according to Kabat EU index).
**Figure 3B****:** wild type (wt) amino acid sequences in the CL domain of kappa isotype with underlined and highlighted amino acid positions 124 and 123 (numbering according to Kabat).
**Figure 3C****:** wild type (wt) amino acid sequences in the CL domain of lambda isotype with underlined and highlighted amino acid positions 124 and 123 (numbering according to Kabat).
- **Figure 4**: **Figure 4A****:** Reduction of main Bence-Jones-type side product by single charged amino acids substitutions according to the invention in the CH1/CL interface.
   Examples of anti-*Ang2-VEGF* multispecific antibodies according to the invention with VH/VL domain exchange/replacement (*CrossMAb^{Vh-VL}*).
   Comparison of wild type (wt) and different combinations of single charged amino acids substitutions
   1) wildtype (wt) anti-*Ang2-VEGF CrossMAb^{Vh-VL}* multispecific antibody without specific amino acid substitutions in the CH1/CL interface,
   2) anti-*Ang2-VEGF* multispecific antibodies according to the invention i) with substitutions at position 124 of the CL domain is, and at position 147 of the CH1 domain (numbering according to Kabat EU index) or ii) with substitutions at position 124 of the CL domain is, and at position 213 of the CH1 domain (numbering according to Kabat EU index),
   3) other anti-*Ang2-VEGF CrossMAb^{Vh-VL}* multispecific antibodies with substitutions at different positions
**Figure 4B****:** Sequences (SEQ ID NOs) of the multispecific antibodies for which the results are shown in Figure 4A.

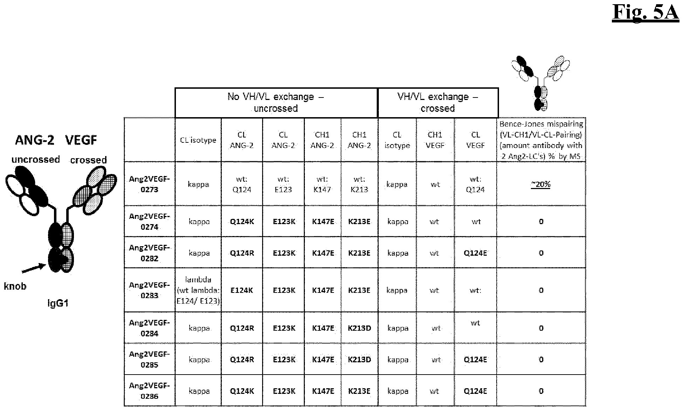

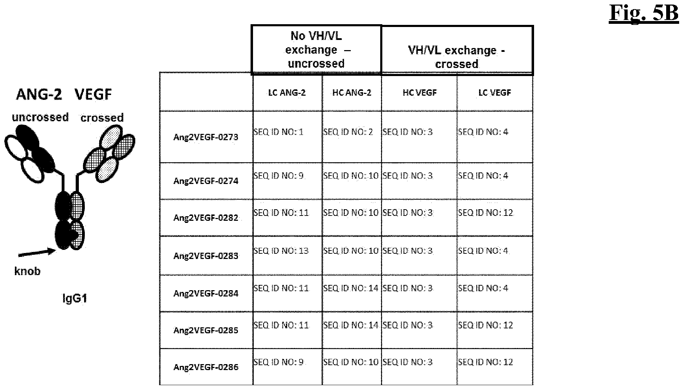

- **Figure 5**: **Figure 5A**:Reduction of main Bence-Jones-type side product by different charged amino acids substitutions in the CH1/CL interface.
   Examples of anti-*Ang2-VEGF* multispecific antibodies according to the invention with VH/VL domain exchange/replacement (*CrossMAb^{Vh-VL}*).
   Comparison of wild type (wt) and different combinations of charged amino acids substitutions
**Figure 5B****:** Sequences (SEQ ID NOs) of the multispecific antibodies for which the results are shown in Figure 5A.

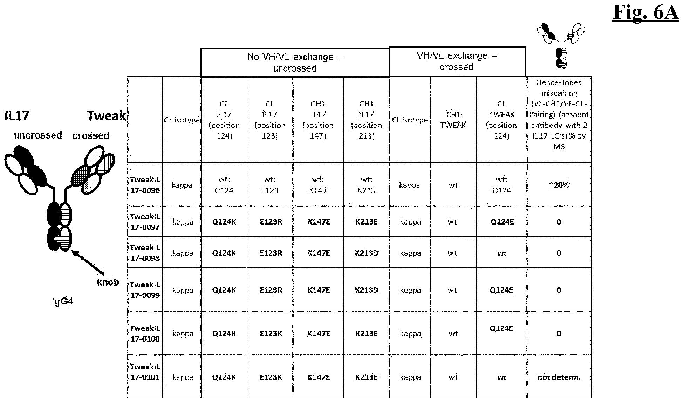

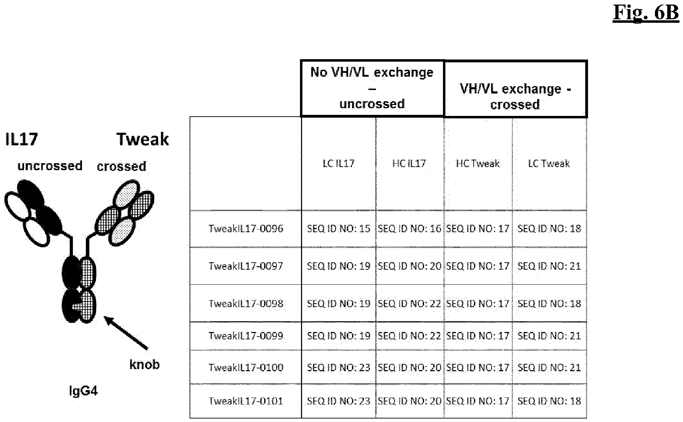

- **Figure 6**: **Figure 6A**:Reduction of main Bence-Jones-type product side by different charged amino acids substitutions in the CH1/CL interface.
   Examples of anti-*IL-17*/*TWEAK* multispecific antibodies according to the invention with VH/VL domain exchange/replacement (*CrossMAb^{Vh-VL}*).
   Comparison of wild type (wt) and different combinations of charged amino acids substitutions
**Figure 6B****:** Sequences (SEQ ID NOs) of the multispecific antibodies for which the results are shown in Figure 6A.

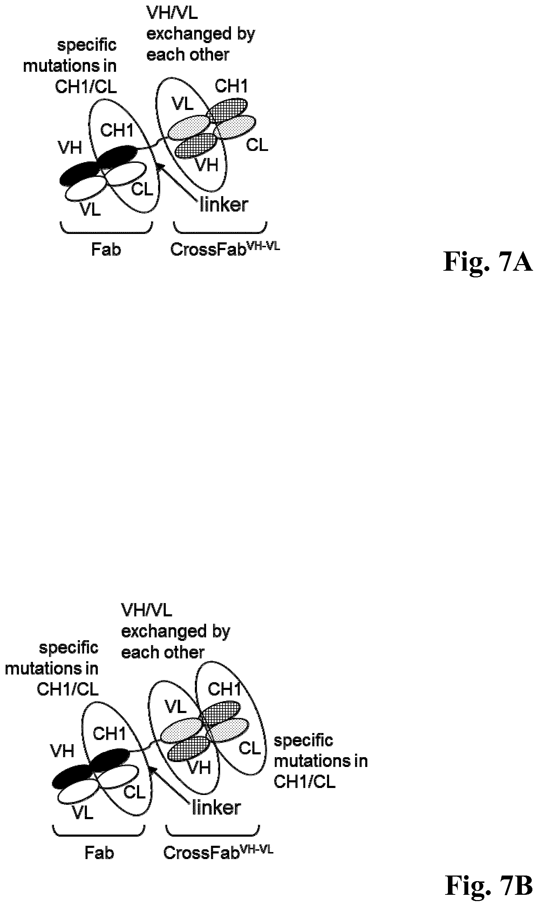

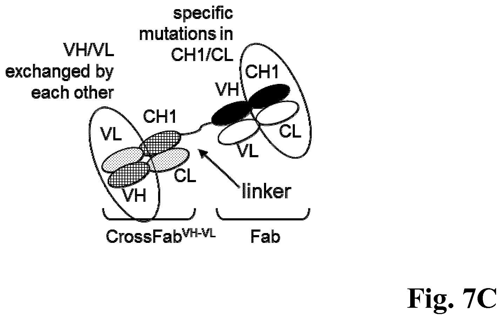

- **Figure 7**: Some examples of bivalent multispecific antibodies according to the invention with VH/VL domain replacement in one antibody binding arm and specific mutations in one CH1/CL domain interface, wherein the multispecific antibdodies are devoid of an Fc fragment (Fab-CrossFab^{VH-VL} format and CrossFab^{VH-VL}-Fab): at least the amino acid at position 124 of the CL domain is substituted independently by lysine (K) ot arginine (R), and at least the amino acid at position 147 of the CH1 domain or the amino acid at position 213 of the CH1 domain is substituted independently by glutamic acid (E), or aspartic acid (D) (numbering according to Kabat EU index).
**Figure 7A****:** VH/VL domain replacement in one antibody binding arm and specific mutations in the CH1/CL domain interface of the other antibody binding arm.
**Figure 7B****:** VH/VL domain replacement in one antibody binding arm with specific mutation in the CH1/CL domain interface of the same antibody binding arm; and further specific mutations in the CH1/CL domain interface of the other antibody binding arm.
**Figure 7C****:** VH/VL domain replacement in one antibody binding arm and specific mutations in the CH1/CL domain interface of the other antibody binding arm.

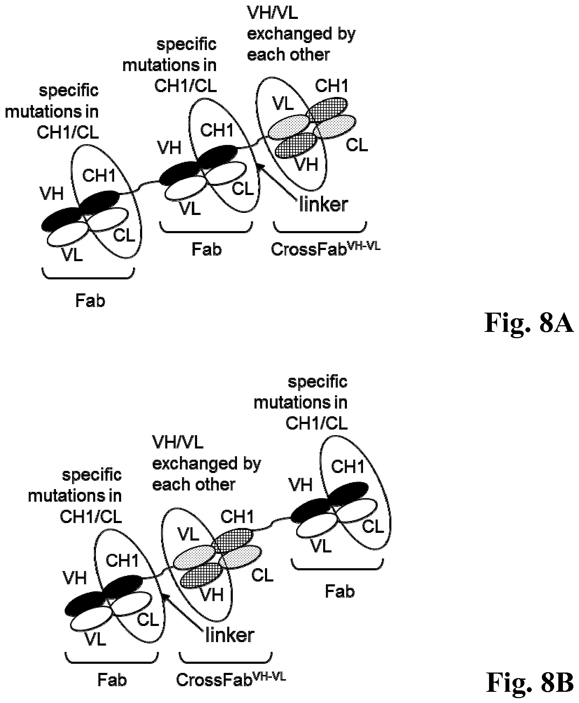

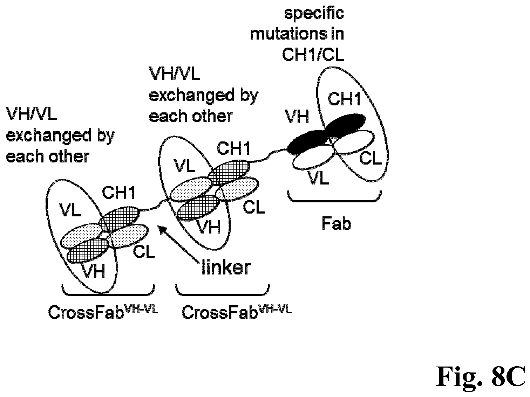

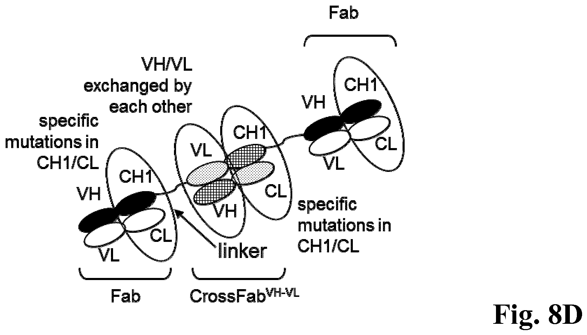

- **Figure 8**: Some examples of trivalent multispecific antibodies according to the invention with VH/VL domain replacement in one antibody binding arm and specific mutations in one CH1/CL domain interface, wherein the multispecific antibdodies are devoid of an Fc fragment (Fab-Fab-CrossFab^{VH-VL} format):
at least the amino acid at position 124 of the CL domain is substituted independently by lysine (K) or arginine (R) (numbering according to Kabat), and
at least the amino acid at position 147 of the CH1 domain or the amino acid at position 213 of the CH1 domain is substituted independently by glutamic acid (E), or aspartic acid (D) (numbering according to Kabat EU index).

**Figure 8A****, B, C:** VH/VL domain replacement in one antibody binding arm and specific mutations in the CH1/CL domain interface of the other antibody binding arms.
**Figure 8D****:** VH/VL domain replacement in one antibody binding arm with specific mutation in the CH1/CL domain interface of the same antibody binding arm; and further specific mutations in the CH1/CL domain interface of the other antibody binding arms.

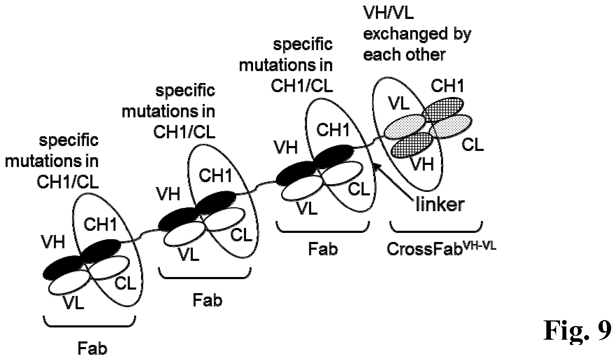

- **Figure 9**: Example of tetravalent multispecific antibodies according to the invention with VH/VL domain replacement in one antibody binding arm and specific mutations in one CH1/CL domain interface, wherein the multispecific antibdodies are devoid of an Fc fragment (Fab-Fab-CrossFab^{VH-VL} format):
at least the amino acid at position 124 of the CL domain is substituted independently by lysine (K) or arginine (R) (numbering according to Kabat), and
at least the amino acid at position 147 of the CH1 domain or the amino acid at position 213 of the CH1 domain is substituted independently by glutamic acid (E), or aspartic acid (D) (numbering according to Kabat EU index):
   VH/VL domain replacement in one antibody binding arm and specific mutations in the CH1/CL domain interface of the other antibody binding arms.

### Detailed Description of the Invention

Multispecific antibodies with a domain replacement/exchange in one binding arm (CrossMabVH-VL) are described in detail in WO2009/080252 and Schaefer, W. et al, PNAS, 108 (2011) 11187-1191. They clearly reduce the byproducts caused by the mismatch of a light chain against a first antigen with the wrong heavy chain against the second antigen (compared to approaches without such domain exchange). However, their preparation is not completely free of side products. The main side product is based on a Bence-Jones -type interaction -see also Schaefer, W. et al, PNAS, 108 (2011) 11187-1191; in Fig. S1I of the Supplement).

An approach for further reduction of such side products to improve the yield of such multispecific antibodies (i.e. multispecific antibodies, which comprise a VH/VL domain replacement/exchange only in the binding arm(s) of one antigen specificity, whereas the binding arm(s) of the other antigen specificity does not comprise a VH/VL domain replacement/exchange but rather is of a wild-type antibody domain arrangement as indicated in **Fig. 1**) was identified by the introduction of substitutions of charged amino acids with the opposite charge at specific amino acid positions in the CH1 and CL domains.

Therefore, the invention relates to a multispecific antibody of human IgG1, human IgG2, human IgG3 or human IgG4 subclass, comprising:
a) a first light chain and a first heavy chain of a first antibody which specifically binds to a first antigen; and
b) a second light chain and a second heavy chain of a second antibody which specifically binds to a second antigen, and wherein the variable domains VL and VH in the second light chain and second heavy chain of the second antibody are replaced by each other; and
wherein in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 147 or the amino acid at position 213 is substituted independently by glutamic acid (E) or aspartic acid (D) (numbering according to Kabat EU index).

In accordance with the concept of the invention, the antibody according to the invention comprises the modifications as indicated above only in the constant domain CL of the first light chain and in the constant domain CH1 of the first heavy chain.

Thus, for said second antibody which specifically binds to a second antigen comprised in a multispecific antibody according to the invention the following applies:
- within the light chain the variable light chain domain VL is replaced by the variable heavy chain domain VH of said antibody; and
- within the heavy chain the variable heavy chain domain VH is replaced by the variable light chain domain VL of said antibody; and
- the constant domains CL and CH1 in the second light chain and second heavy chain of the second antibody are not replaced by each other (remain unexchanged).

Thus for said antibody which specifically binds to a first antigen comprised in a multispecific antibody according to the invention the following applies:
- within said first light chain derived from said first antibody the sequential arrangement of the domains of the light chain (CL-VL) remains unaltered; and
- within said first heavy chain derived from said first antibody the sequential arrangement of the domains of the heavy chain (e.g. CH1-VH or CH3-CH2-CH1-VH) remains unaltered (therefore said antibody which specifically binds to the first antibody does not include a domain exchange, particularly not an exchange of VH/VL).

In other words, said antibody which specifically binds to a first antigen comprised in a multispecific antibody according to the invention comprises:
- a first light chain derived from said first antibody comprising a sequential arrangement of the domains of the light chain of VL-CL (from N-terminal to C-terminal direction); and
- a first heavy chain derived from said first antibody comprising a sequential arrangement of the domains of the heavy chain of CH1-VH (from from N-terminal to C-terminal direction) (in one embodiment the first heavy chain comprises a sequential arrangement of the domains of the heavy chain of CH3-CH2-CH1-VH from N-terminal to C-terminal direction).

The "light chain of an antibody" as used herein is a polypeptide comprising in N-terminal to C-terminal direction an antibody light chain variable domain (VL), and an antibody light chain constant domain (CL), abbreviated as VL-CL.

The "heavy chain of an antibody" as used herein is a polypeptide comprising in N-terminal to C-terminal direction an antibody heavy chain variable domain (VH) and an antibody constant heavy chain domain 1 (CHI).

In one embodiment of the invention the heavy chain of the multispecific antibody includes in N-terminal to C-terminal direction an antibody heavy chain variable domain (VH) and an antibody constant heavy chain domain 1 (CHI) and is devoid of heavy chain constant domains CH2 and CH3, thus abbreviated as VH-CH1. In one embodiment multispecific antibodies according to the invention comprise at least two Fab fragments, wherein the first Fab fragment comprises at least one antigen binding site specific for a first antigen; and the second Fab fragment comprises at least one antigen binding site specific for a second antigen, wherein in the second Fab fragment the variable domains VL and VH in the second light chain and second heavy chain are replaced by each other; and wherein the multispecific antibody is devoid of an Fc domain; and wherein in the constant domain CL of the light chain of the first Fab fragment the amino acid at position 124 is substituted independently by lysine (K) or arginine (R) (numbering according to Kabat), and wherein in the constant domain CH1 of the heavy chain of the first Fab fragment the amino acid at position 147 or the amino acid at position 213 is substituted independently by glutamic acid (E) or aspartic acid (D) (numbering according to Kabat EU index).

As used herein, "Fab fragment" refers to an antibody fragment comprising a light chain fragment comprising a variable VL domain and a constant domain of a light chain (CL), and a variable VH domain and a first constant domain (CHI) of a heavy chain. The multispecific antibodies according to this embodiment comprise at least two Fab fragments, wherein the variable regions of the heavy and light chain of the second Fab fragment are exchanged. Due to the exchange of the variable regions, said second Fab fragment is also referred to as "cross-Fab fragment" or "xFab fragment" or "crossover Fab fragment". In said second Fab fragment wherein the variable regions of the Fab heavy and light chain are exchanged, the crossover Fab molecule comprises a modified heavy chain composed of the light chain variable region (VL) and the heavy chain constant region (CHI), and a modified light chain composed of the heavy chain variable region (VH) and the light chain constant region (CL). This crossover Fab molecule is also referred to as CrossFab^{VH/VL}.

The term "Fc domain" is used herein to define a C-terminal region of an immunoglobulin heavy chain that contains at least a portion of the constant region. For example in natural antibodies, the Fc domain is composed of two identical protein fragments, derived from the second and third constant domains of the antibody's two heavy chains in IgG, IgA and IgD isotypes; IgM and IgE Fc domains contain three heavy chain constant domains (CH domains 2-4) in each polypeptide chain. "Devoid of the Fc domain" as used herein means that the bispecific antibodies of the invention do not comprise a CH2, CH3 and CH4 domain; i.e. the constant heavy chain consists solely of one or more CH1 domains.

In one embodiment the first and second Fab fragments are connected via a peptide linker. The term "peptide linker" as used herein denotes a peptide with amino acid sequences, which is preferably of synthetic origin. In one embodiment a peptide linker is used to connect one of the Fab fragments to the C- or N-terminus of the other Fab fragment in order to form a multispecific antibody according to the invention. In one preferred embodiment said peptide linker is a peptide with an amino acid sequence with a length of at least 5 amino acids, in one embodiment with a length of 5 to 100, in a further embodiment of 10 to 50 amino acids. In one embodiment said peptide linker is (GxS)ₙ or (GxS)ₙGₘ with G = glycine, S = serine, and (x = 3, n= 3, 4, 5 or 6, and m= 0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said peptide linker is (G₄S)₂. The peptide linker is used to connect the first and the second Fab fragment. In one embodiment the first Fab fragment is connected to the C- or N- terminus of the second Fab fragment.

In another preferred embodiment of the invention the heavy chain of an antibody comprises in N-terminal to C-terminal direction an antibody heavy chain variable domain (VH), an antibody constant heavy chain domain 1 (CHI), an antibody heavy chain constant domain 2 (CH2), and an antibody heavy chain constant domain 3 (CH3), abbreviated as VH-CH1-CH2-CH3.

In case the multispecific antibody comprises the domains VH-CH1-CH2-CH3 in each heavy chain, an additional aspect of the invention is to further improve the ratio of a desired multispecific antibody compared to undesired side products can be by modifications of the first and second CH3 domain of said the multispecific antibody to increase the heterodimerisation of both heavy chains containing these first and second CH3 domain.

There exist several approaches for CH3-modifications to enforce the heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically in all such approaches the first CH3 domain and the second CH3 domains are both engineered in a complementary manner so that each CH3 domain (or the heavy chain comprising it) cannot longer homodimerize with itself but is forced to heterodimerize with the complementary engineered other CH3 domain (so that the first and second CH3 domain heterodimerize and no homdimers between the two first or the two second CH3 domains are formed). These different approaches for improved heavy chain heterodimerization are contemplated as different alternatives in combination with the heavy-light chain modifications (VH and VL exchange/replacement in one binding arm and the introduction of substitutions of charged amino acids with opposite charges in the CH1/CL interface) in the multispecific antibodies according to the invention which reduce light chain mispairing and Bence-Jones type side products.

In one embodiment of the invention (in case the multispecific antibody comprises CH3 domains in the heavy chains) the CH3 domains of said multispecific antibody according to the invention are altered to support heterodimerization by
- substituting at least one amino acid of the CH3 domain of the first heavy chain, and
- substituting at least one amino acid of the CH3 domain of the second heavy chain, wherein said amino acid is facing the at least one amino acid of the CH3 domain of the first heavy chain within the tertiary structure of the multispecific antibody,
   wherein the respective amino acids within the CH3 domains of the first and second heavy chain, respectively, are either
- substituted such that amino acids of opposite side chain charges are introduced into the opposing heavy chains, or
- substituted such that amino acids with large and small side chain volumes are introduced into the opposing heavy chains, whereby a protuberance is created by an amino acid with a large side chain volume in one CH3 domain, which is positionable in a cavity located within the other CH3 domain, wherein the cavity is created by an amino acid with a small side chain volume.

In one preferred embodiment of the invention (in case the multispecific antibody comprises CH3 domains in the heavy chains) the CH3 domains of said multispecific antibody according to the invention are altered by the "knob-into-hole" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681; and WO 98/ 050431. In this method the interaction surfaces of the two CH3 domains are altered to increase the heterodimerisation of both heavy chains containing these two CH3 domains. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

Thus in one embodiment of the invention said multispecific antibody (comprises a CH3 domain in each heavy chain and) is further characterized in that the first CH3 domain of the first heavy chain of the antibody under a) and the second CH3 domain of the second heavy chain of the antibody under b) each meet at an interface which comprises an original interface between the antibody CH3 domains, wherein said interface is altered to promote the formation of the multispecific antibody, wherein the alteration is characterized in that:
i) the CH3 domain of one heavy chain is altered, so that within the original interface of the CH3 domain of the one heavy chain that meets the original interface of the CH3 domain of the other heavy chain within the multispecific antibody, an amino acid residue is replaced with an amino acid residue having a larger side chain volume, thereby generating a protuberance within the interface of the CH3 domain of the one heavy chain which is positionable in a cavity within the interface of the CH3 domain of the other heavy chain; and
ii) the CH3 domain of the other heavy chain is altered, so that within the original interface of the CH3 domain of the other heavy chain that meets the original interface of the CH3 domain of the one heavy chain within the multispecific antibody an amino acid residue is replaced with an amino acid residue having a smaller side chain volume, thereby generating a cavity within the interface of the CH3 domain of the other heavy chain within which a protuberance within the interface of the CH3 domain of the one heavy chain is positionable.

In one embodiment of the invention said amino acid residue having a larger side chain volume is selected from the group consisting of arginine (R), phenylalanine (F), tyrosine (Y) and tryptophan (W).

In one embodiment of the invention said amino acid residue having a smaller side chain volume is selected from the group consisting of alanine (A), serine (S), threonine (T) and valine (V).

In one aspect of the invention both CH3 domains are further altered by the introduction of cysteine (C) as amino acid in the corresponding positions of each CH3 domain such that a disulfide bridge between both CH3 domains can be formed. Thus according to this aspect of the invention, the CH3 domain of the one heavy chain is further altered so that within the original interface of the CH3 domain of the one heavy chain that meets the original interface of the CH3 domain of the other heavy chain within the multispecific antibody, an amino acid residue is replaced by a cysteine (C) residue, and the CH3 domain of the other heavy chain is further altered so that within the original interface of the CH3 domain of the other heavy chain that meets the original interface of the CH3 domain of the one heavy chain within the multispecific antibody, an amino acid residue is replaced by a cysteine (C) residue, such that a disulfide bridge between both CH3 domains can be formed via the introduced cysteine residues.

In one preferred embodiment, said multispecific antibody comprises an amino acid T366W mutation in one CH3 domain of the "knob chain" and amino acid T366S, L368A, Y407V mutations in the other CH3 domain of the "hole chain". An additional interchain disulfide bridge between the CH3 domains can also be used (Merchant, A.M., et al., Nature Biotech. 16 (1998) 677-681), e.g. by introducing an amino acid Y349C mutation into the CH3 domain of the "hole chain"; and an amino acid E356C mutation or an amino acid S354C mutation into the CH3 domain of the "knobs chain".

In one preferred embodiment, said multispecific antibody (which comprises a CH3 domain in each heavy chain) comprises amino acid S354C and T366W mutations in one CH3 domain and amino acid Y349C, T366S, L368A and Y407V mutations in the other of the two CH3 domains (with the additional amino acid S354C mutation in one CH3 domain and the additional amino acid Y349C mutation in the other CH3 domain forming an interchain disulfide bridge) (numberings according to Kabat EU index).

Other techniques for CH3-modifications to enforce the heterodimerization are contemplated as alternatives of the invention and described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291.

In one embodiment the heterodimerization approach described in EP 1 870 459A1 is used alternatively. This approach is based on the introduction of substitutions/mutations of charged amino acids with the opposite charge at specific amino acid positions of the in the CH3/CH3 domain interface between both heavy chains. One preferred embodiment for said multispecific antibodies are amino acid R409D and K370E mutations in the CH3 domain of one heavy chain and amino acid D399K and E357K mutations in the CH3 domain of the other heavy chain of the multispecific antibody (numberings according to Kabat EU index).

In another embodiment said multispecific antibody comprises an amino acid T366W mutation in the CH3 domain of the "knobs chain" and amino acid T366S, L368A and Y407V mutations in the CH3 domain of the "hole chain"; and additionally comprises amino acid R409D and K370E mutations in the CH3 domain of the "knobs chain" and amino acid D399K and E357K mutations in the CH3 domain of the "hole chain".

In another embodiment said multispecific antibody comprises amino acid S354C and T366W mutations in of the CH3 domain of one heavy chain and amino acid Y349C, T366S, L368A and Y407V mutations in the CH3 domain of the other heavy chain; or said multispecific antibody comprises amino acid Y349C and T366W mutations in the CH3 domain of one heavy chain and amino acid S354C, T366S, L368A and Y407V mutations in the CH3 domain of the other heavy chain and additionally comprises amino acid R409D and K370E mutations in the CH3 domain of the "knobs chain" and amino acid D399K and E357K mutations in the CH3 domain of the "hole chain".

In one embodiment the heterodimerization approach described in WO2013/157953 is used alternatively. In one embodiment the CH3 domain of one heavy chain comprises an amino acid T366K mutation and the CH3 domain of the other heavy chain comprises an amino acid L351D mutation. In a further embodiment the CH3 domain of the one heavy chain further comprises an amino acid L351K mutation. In a further embodiment the CH3 domain of the other heavy chain further comprises an amino acid mutation selected from Y349E, Y349D and L368E (in one embodiment L368E).

In one embodiment the heterodimerization approach described in WO2012/058768 is used alternatively. In one embodiment the CH3 domain of one heavy chain comprises amino acid L351Y and Y407A mutations and the CH3 domain of the other heavy chain comprises amino acid T366A and K409F mutations. In a further embodiment the CH3 domain of the other heavy chain further comprises an amino acid mutation at position T411, D399, S400, F405, N390 or K392. In one embodiment said amino acid mutation is selected from the group consisting of
a) T411N, T411R, T411Q, T411K, T411D, T411E and T411W,
b) D399R, D399W, D399Y and D399K,
c) S400E, S400D, S400R and S400K,
d) F405I, F405M, F405T, F405S, F405V and F405W,
e) N390R, N390K and N390D,
f) K392V, K392M, K392R, K392L, K392F and K392E.

In a further embodiment the CH3 domain of one heavy chain comprises amino acid L351Y and Y407A mutations and the CH3 domain of the other heavy chain comprises amino acid T366V and K409F mutations. In a further embodiment the CH3 domain of one heavy chain comprises an amino acid Y407A mutation and the CH3 domain of the other heavy chain comprises amino acid T366A and K409F mutations. In a further embodiment the CH3 domain of the other heavy chain further comprises amino acid K392E, T411E, D399R and S400R mutations.

In one embodiment the heterodimerization approach described in WO2011/143545 is used alternatively. In one embodiment the amino acid modification according to WO2011/143545 is introduced in the CH3 domain of the heavy chain at a position selected from the group consisting of 368 and 409.

In one embodiment the heterodimerization approach described in WO2011/090762 which also uses the knob-into-hole technology described above is used alternatively. In one embodiment the CH3 domain of one heavy chain comprises an amino acid T366W mutation and the CH3 domain of the other heavy chain comprises an amino acid Y407A mutation. In one embodiment the CH3 domain of one heavy chain comprises an amino acid T366Y mutation and the CH3 domain of the other heavy chain comprises an amino acid Y407T mutation.

In one embodiment the multispecific antibody is of IgG2 isotype and the heterodimerization approach described in WO2010/129304 is used alternatively.

In one embodiment the heterodimerization approach described in WO2009/089004 is used alternatively. In one embodiment the CH3 domain of one heavy chain comprises an amino acid substitution of K392 or N392 with a negatively-charged amino acid (in one embodiment glutamic acid (E) or aspartic acid (D); in a further embodiment a K392D or N392D mutation) and the CH3 domain of the other heavy chain comprises an amino acid substitution of D399, E356, D356, or E357 with a positively-charged amino acid (in one embodiment Lysine (K) or arginine (R), in a further embodiment a D399K, E356K, D356K or E357K substitution; and in an even further embodiment a D399K or E356K mutation). In a further embodiment the CH3 domain of the one heavy chain further comprises an amino acid substitution of K409 or R409 with a negatively-charged amino acid (in one embodiment glutamic acid (E) or aspartic acid (D); in a further embodiment a K409D or R409D mutation). In a further embodiment the CH3 domain of the one heavy chain further or alternatively comprises an amino acid substitution of K439 and/or K370 with a negatively-charged amino acid (in one embodiment glutamic acid (E) or aspartic acid (D)).

In one embodiment the heterodimerization approach described in WO2007/147901 is used alternatively. In one embodiment the CH3 domain of one heavy chain comprises amino acid K253E, D282K and K322D mutations and the CH3 domain of the other heavy chain comprises amino acid D239K, E240K and K292D mutations.

In one embodiment the heterodimerization approach described in WO2007/110205 is used alternatively.

The terms "binding site" or "antigen-binding site" as used herein denotes the region(s) of an antibody molecule to which a ligand (e.g. the antigen or antigen fragment of it) actually binds and which is derived from an antibody. The antigen-binding site includes antibody heavy chain variable domains (VH) and/or an antibody light chain variable domain (VL), or pairs of VH/VL.

The antigen-binding sites that specifically bind to the desired antigen can be derived a) from known antibodies to the antigen or b) from new antibodies or antibody fragments obtained by de novo immunization methods using inter alia either the antigen protein or nucleic acid or fragments thereof, or by phage display.

An antigen-binding site of an antibody of the invention can contain six complementarity determining regions (CDRs) which contribute in varying degrees to the affinity of the binding site for antigen. There are three heavy chain variable domain CDRs (CDRH1, CDRH2 and CDRH3) and three light chain variable domain CDRs (CDRL1, CDRL2 and CDRL3). The extent of CDR and framework regions (FRs) is determined by comparison to a compiled database of amino acid sequences in which those regions have been defined according to variability among the sequences. Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

Antibody specificity refers to selective recognition of the antibody for a particular epitope of an antigen. Natural antibodies, for example, are monospecific. The term "monospecific" antibody as used herein denotes an antibody that has one or more binding sites each of which bind to the same epitope of the same antigen.

Multispecific antibodies are e.g. bispecific, tri- or tetraspecific antibodies. Bispecific antibodies are antibodies which have two different antigen-binding specificities. Trispecific antibodies, accordingly, are antibodies which have three different antigen-binding specificities. Tetraspecific antibodies are antibodies which have four different antigen-binding specificities. In one preferred embodiment of the invention the multispecific antibody is a bispecific antibody.

If an antibody has more than one specificity, the recognized epitopes may be associated with a single antigen or with more than one antigen.

The term "valent" as used within the current application denotes the presence of a specified number of binding sites in an antibody molecule. A natural antibody for example has two binding sites and is bivalent. As such, the term "trivalent" denotes the presence of three binding sites in an antibody molecule.

In one preferred embodiment of the invention the antibodies of the invention comprise immunoglobulin constant regions of one or more immunoglobulin classes. Immunoglobulin classes include IgG, IgM, IgA, IgD, and IgE isotypes and, in the case of IgG and IgA, their subtypes. In one preferred embodiment, an antibody of the invention has a constant domain structure of an IgG type antibody. The terms "monoclonal antibody" or "monoclonal antibody composition" as used herein refers to a preparation of antibody molecules of a single amino acid composition.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S.L., et al., Proc. Natl. Acad. Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole, et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The "variable domain" (variable domain of a light chain (VL), variable domain of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain an antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991).

As used herein, the terms "binding", "that/which specifically binds", and "specifically binding" refer to the binding of the antibody to an epitope of the antigen in an in vitro assay, preferably in an plasmon resonance assay (BIAcore®, GE-Healthcare Uppsala, Sweden) with purified wild-type antigen. The affinity of the binding is defined by the terms kₐ (rate constant for the association of the antibody from the antibody/antigen complex), k_{D} (dissociation constant), and K_{D} (k_{D}/kₐ). In one embodiment "binding" or "that/which specifically binds to" means a binding affinity (K_{D}) of 10⁻⁸ mol/l or less, in one embodiment 10⁻⁸ M to 10⁻¹³ mol/l. Thus, a multispecific antibody according to the invention specifically binds to each antigen for which it is specific with a binding affinity (K_{D}) of 10⁻⁸ mol/l or less, in one embodiment with a binding affinity (K_{D}) of 10⁻⁸ to 10⁻¹³ mol/l. In one embodiment the multispecific antibody specifically binds to its antigen with a binding affinity (K_{D}) of 10⁻⁹ to 10⁻¹³ mol/l.

Binding of the antibody to the FcyRIII can be investigated by a BIAcore® assay (GE-Healthcare Uppsala, Sweden). The affinity of the binding is defined by the terms kₐ (rate constant for the association of the antibody from the antibody/antigen complex), k_{D} (dissociation constant), and K_{D} (k_{D}/ka).

The term "epitope" includes any polypeptide determinant capable of specific binding to an antibody. In certain embodiments, epitope determinants include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

In certain embodiments, an antibody is said to specifically bind an antigen when it preferentially recognizes its target antigen in a complex mixture of proteins and/or macromolecules.

In a further embodiment the multispecific antibody according to the invention is characterized in that said antibody is of human IgG1 subclass, or of human IgG1 subclass with the mutations L234A and L235A (numbering according to Kabat EU index).

In a further embodiment the multispecific antibody according to the invention is characterized in that said antibody is of human IgG2 subclass.

In a further embodiment the multispecific antibody according to the invention is characterized in that said antibody is of human IgG3 subclass.

In a further embodiment the multispecific antibody according to the invention is characterized in that said antibody is of human IgG4 subclass or, of human IgG4 subclass with the additional mutation S228P (numbering according to Kabat EU index).

In a further embodiment the multispecific antibody according to the invention is characterized in that it is of human IgG1 or human IgG4 subclass.

In a further embodiment the multispecific antibody according to the invention is characterized in being of human IgG1 subclass with the mutations L234A and L235A (numbering according to Kabat EU index).

In a further embodiment the multispecific antibody according to the invention is characterized in being of human IgG1 subclass with the mutations L234A, L235A and P329G (numbering according to Kabat EU index).

In a further embodiment the multispecific antibody according to the invention is characterized in being of human IgG4 subclass with the mutations S228P and L235E (numbering according to Kabat EU index).

In a further embodiment the multispecific antibody according to the invention is characterized in being of human IgG4 subclass with the mutations S228P, L235E and P329G (numbering according to Kabat EU index).

It has now been found that the multispecific antibodies according to the invention have improved characteristics, such as biological or pharmacological activity, pharmacokinetic properties or toxicity. They can be used e.g. for the treatment of diseases, such as cancer.

The term "constant region" as used within the current applications denotes the sum of the domains of an antibody other than the variable region. The constant region is not involved directly in binding of an antigen, but exhibit various effector functions. Depending on the amino acid sequence of the constant region of their heavy chains, antibodies are divided in the classes: IgA, IgD, IgE, IgG and IgM, and several of these may be further divided into subclasses, such as IgG1, IgG2, IgG3, and IgG4, IgA1 and IgA2. The heavy chain constant regions that correspond to the different classes of antibodies are called α, δ, ε, γ, and µ, respectively. The light chain constant regions (CL) which can be found in all five antibody classes are called κ (kappa) and λ (lambda). The "constant domains" as used herein are from human origin which is from a constant heavy chain region of a human antibody of the subclass IgG1, IgG2, IgG3, or IgG4 and/or a constant light chain kappa or lambda region. Such constant domains and regions are well known in the state of the art and e.g. described by Kabat, et al., Sequences of Proteins of Immunological Interest, 5th ed., Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CHI, Hinge, CH2 and CH3, which is herein further clarified by referring to "numbering according to Kabat EU index" in this case).

While antibodies of the IgG4 subclass show reduced Fc receptor (FcyRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, and His435 (numberings according to Kabat EU index) are residues which, if altered, provide also reduced Fc receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434).

In one embodiment an antibody according to the invention has a reduced FcR binding compared to an IgG1 antibody. Thus, the parent antibody is in regard to FcR binding of IgG4 subclass or of IgG1 or IgG2 subclass with a mutation in S228, L234, L235 and/or D265, and/ or contains the PVA236 mutation (numberings according to Kabat EU index). In one embodiment the mutations in the parent antibody are S228P, L234A, L235A, L235E and/or PVA236 (numberings according to Kabat EU index). In another embodiment the mutations in the parent antibody are in IgG4 S228P and in IgG1 L234A and L235A (numberings according to Kabat EU index).

The constant region of an antibody is directly involved in ADCC (antibody-dependent cell-mediated cytotoxicity) and CDC (complement-dependent cytotoxicity). Complement activation (CDC) is initiated by binding of complement factor C1q to the constant region of most IgG antibody subclasses. Binding of C1q to an antibody is caused by defined protein-protein interactions at the so called binding site. Such constant region binding sites are known in the state of the art and described e.g. by Lukas, T.J., et al., J. Immunol. 127 (1981) 2555-2560; Bunkhouse, R. and Cobra, J.J., Mol. Immunol. 16 (1979) 907-917; Burton, D.R., et al., Nature 288 (1980) 338-344; Thomason, J.E., et al., Mol. Immunol. 37 (2000) 995-1004; Idiocies, E.E., et al., J. Immunol. 164 (2000) 4178-4184; Hearer, M., et al., J. Virol. 75 (2001) 12161-12168; Morgan, A., et al., Immunology 86 (1995) 319-324; and EP 0 307 434. Such constant region binding sites are, e.g., characterized by the amino acids L234, L235, D270, N297, E318, K320, K322, P331, and P329 (numbering according to Kabat EU index).

The term "antibody-dependent cellular cytotoxicity (ADCC)" refers to lysis of human target cells by an antibody according to the invention in the presence of effector cells. ADCC is measured preferably by the treatment of a preparation of antigen expressing cells with an antibody according to the invention in the presence of effector cells such as freshly isolated PBMC or purified effector cells from buffy coats, like monocytes or natural killer (NK) cells or a permanently growing NK cell line.

The term "complement-dependent cytotoxicity (CDC)" denotes a process initiated by binding of complement factor C1q to the Fc part of most IgG antibody subclasses. Binding of C1q to an antibody is caused by defined protein-protein interactions at the so called binding site. Such Fc part binding sites are known in the state of the art (see above). Such Fc part binding sites are, e.g., characterized by the amino acids L234, L235, D270, N297, E318, K320, K322, P331, and P329 (numbering according to Kabat EU index). Antibodies of subclass IgG1, IgG2, and IgG3 usually show complement activation including C1q and C3 binding, whereas IgG4 does not activate the complement system and does not bind C1q and/or C3.

Cell-mediated effector functions of monoclonal antibodies can be enhanced by engineering their oligosaccharide component as described in Umana, P., et al., Nature Biotechnol. 17 (1999) 176-180, and US 6,602,684. IgG1 type antibodies, the most commonly used therapeutic antibodies, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M., R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32). Umana, P., et al., Nature Biotechnol. 17 (1999) 176-180 and WO 99/54342 showed that overexpression in Chinese hamster ovary (CHO) cells of β(1,4)-N-acetylglucosaminyltransferase III ("GnTIII"), a glycosyltransferase catalyzing the formation of bisected oligosaccharides, significantly increases the in vitro ADCC activity of antibodies. Alterations in the composition of the Asn297 carbohydrate or its elimination affect also binding to FcyR and C1q (Umana, P., et al., Nature Biotechnol. 17 (1999) 176-180; Davies, J., et al., Biotechnol. Bioeng. 74 (2001) 288-294; Mimura, Y., et al., J. Biol. Chem. 276 (2001) 45539-45547; Radaev, S., et al., J. Biol. Chem. 276 (2001) 16478-16483; Shields, R.L., et al., J. Biol. Chem. 276 (2001) 6591-6604; Shields, R.L., et al., J. Biol. Chem. 277 (2002) 26733-26740; Simmons, L.C., et al., J. Immunol. Methods 263 (2002) 133-147).

Methods to enhance cell-mediated effector functions of monoclonal antibodies are reported e.g. in WO 2005/018572, WO 2006/116260, WO 2006/114700, WO 2004/065540, WO 2005/011735, WO 2005/027966, WO 1997/028267, US 2006/0134709, US 2005/0054048, US 2005/0152894, WO 2003/035835, WO 2000/061739.

In one preferred embodiment of the invention, the multispecific antibody is glycosylated (if it comprises an Fc part of IgG1, IgG2, IgG3 or IgG4 subclass, preferably of IgG1 or IgG3 subclass) with a sugar chain at Asn297 whereby the amount of fucose within said sugar chain is 65% or lower (numbering according to Kabat EU index). In another embodiment is the amount of fucose within said sugar chain is between 5% and 65%, preferably between 20% and 40%. "Asn297" according to the invention means amino acid asparagine located at about position 297 in the Fc region. Based on minor sequence variations of antibodies, Asn297 can also be located some amino acids (usually not more than ±3 amino acids) upstream or downstream of position 297, i.e. between position 294 and 300. In one embodiment the glycosylated antibody according to the invention the IgG subclass is of human IgG1 subclass, of human IgG1 subclass with the mutations L234A and L235A or of IgG3 subclass. In a further embodiment the amount of N-glycolylneuraminic acid (NGNA) is 1% or less and/or the amount of N-terminal alpha-1,3-galactose is 1% or less within said sugar chain. The sugar chain preferably exhibits the characteristics of N-linked glycans attached to Asn297 of an antibody recombinantly expressed in a CHO cell.

The term "the sugar chains show characteristics of N-linked glycans attached to Asn297 of an antibody recombinantly expressed in a CHO cell" denotes that the sugar chain at Asn297 of the parent antibody according to the invention has the same structure and sugar residue sequence except for the fucose residue as those of the same antibody expressed in unmodified CHO cells, e.g. as those reported in WO 2006/103100.

The term "NGNA" as used within this application denotes the sugar residue N-glycolylneuraminic acid.

Glycosylation of human IgG1 or IgG3 occurs at Asn297 as core fucosylated biantennary complex oligosaccharide glycosylation terminated with up to two Gal residues. Human constant heavy chain regions of the IgG1 or IgG3 subclass are reported in detail by Kabat, E., A., et al., Sequences of Proteins of Immunological Interest, 5th Ed. Public Health Service, National Institutes of Health, Bethesda, MD. (1991), and by Brüggemann, M., et al., J. Exp. Med. 166 (1987) 1351-1361; Love, T., W., et al., Methods Enzymol. 178 (1989) 515-527. These structures are designated as G0, G1 (α-1,6- or α-1,3-), or G2 glycan residues, depending from the amount of terminal Gal residues (Raju, T., S., Bioprocess Int. 1 (2003) 44-53). CHO type glycosylation of antibody Fc parts is e.g. described by Routier, F., H., Glycoconjugate J. 14 (1997) 201-207. Antibodies which are recombinantly expressed in non-glycomodified CHO host cells usually are fucosylated at Asn297 in an amount of at least 85%. The modified oligosaccharides of the antibody may be hybrid or complex. Preferably the bisected, reduced/not-fucosylated oligosaccharides are hybrid. In another embodiment, the bisected, reduced/not-fucosylated oligosaccharides are complex.

According to the invention "amount of fucose" means the amount of said sugar within the sugar chain at Asn297, related to the sum of all glycostructures attached to Asn297 (e.g. complex, hybrid and high mannose structures) measured by MALDI-TOF mass spectrometry and calculated as average value. The relative amount of fucose is the percentage of fucose-containing structures related to all glycostructures identified in an N-Glycosidase F treated sample (e.g. complex, hybrid and oligo- and high-mannose structures, resp.) by MALDI-TOF.

Antibodies according to the invention may bind to a variety of antigens. In one embodiment of the invention, neither the first antigen nor the second antigen is an activating T cell antigen. In one embodiment of the invention, neither the first antigen nor the second antigen is CD3. In one embodiment, the antibody does not specifically bind to an activating T cell antigen. In one embodiment, the antibody does not specifically bind to CD3.

In one embodiment of the invention the first or the second antigen is human TWEAK. In one embodiment of the invention the first or the second antigen is human IL17. In one embodiment of the invention the first antigen is human TWEAK and the second antigen is human IL17. In one embodiment of the invention the first antigen is human IL17 and the second antigen is human TWEAK.

Human TWEAK (UniProtKB O43508, TNF-related weak inducer of apoptosis) is a cell surface associated type II transmembrane protein. TWEAK is described in Chicheportiche, Y., et al., J. Biol. Chem. 272 (1997) 32401-32410; Marsters, S.A., et al., Curr. Biol. 8 (1998) 525-528; Lynch, C.N., et al., J. Biol. Chem. 274 (1999) 8455-8459. The active form of TWEAK is a soluble homotrimer. Human and murine TWEAK show 93 % sequence identity in receptor binding domain. The TWEAK receptor Fn14 (fibroblast growth factor inducible 14 kDa protein) is a 129 aa type I transmembane protein consisting of one single cystein rich domain in ligand binding domain. Signaling of TWEAK occurs via NF-KB pathway activation. TWEAK mRNA is expressed in a variety of tissues and found in most major organs like heart, brain, skeletal muscle, and pancreas, tissues related to the immune system like spleen, lymph nodes, and thymus. Fn14 mRNA has been detected in heart, brain, lung, placenta, vascular EC and smooth muscle cells. TWEAK-null and Fnl4-null knockout mice are viable, healthy and fertile and have more natural killer cells and display an enhanced innate inflammatory response. TWEAK is involved in apoptosis, proliferation, angiogenesis, ischemic penumbra, cerebral edema, multiple sclerosis.

Human IL-17 (also named IL17-A; CTLA-8, Swiss Prot Q16552, IL17) is a pro-inflammatory cytokine produced by a subset of helper T cells (called Th17) that has been implicated in the pathogenesis of MS. IL-17A plays a role in the induction of other inflammatory cytokines, chemokines and adhesion molecules. Treatment of animals with IL-17A neutralizing antibodies decreases disease incidence and severity in autoimmune encephalomyelitis (Komiyama, Y. et al., J. Immunol. 177 (2006) 566-573). IL-17A is over-expressed in the cerebrospinal fluid of MS patients (Hellings, P.W. et al., Am. J. Resp. Cell Mol. Biol. 28 (2003) 42-50; Matusevicius, D. et al., Multiple Sclerosis 5 (1999) 101-104; WO 2005/051422). In addition, IL-17A neutralizing antibodies reduce severity and incidence of mouse RA model of collagen induced arthritis, and high levels of IL-17A can be detected in the synovial fluid of inflamed joints from RA patients (Ziolkowska, M. et al., J. Immunol. 164 (2000) 2832-2838; Kotake, S., et al., J. Clin. Invest. 103 (1999) 1345-1352; Hellings, P.W. et al., Am. J. Resp. Cell Mol. Biol. 28 (2003) 42-50).

The antibody according to the invention is produced by recombinant means. Thus, one aspect of the current invention is a nucleic acid encoding the antibody according to the invention and a further aspect is a cell comprising said nucleic acid encoding an antibody according to the invention. Methods for recombinant production are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective modified light and heavy chains are inserted into expression vectors by standard methods. Expression is performed in appropriate prokaryotic or eukaryotic host cells like CHO cells, NS0 cells, SP2/0 cells, HEK293 cells, COS cells, PER.C6 cells, yeast, or E.coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

Antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a cleaved variant heavy chain). This may be the case where the final two C-terminal amino acids of the heavy chain are glycine (G446) and lysine (K447, numbering according to Kabat EU index).

Therefore, amino acid sequences of heavy chains including CH3 domains are denoted herein without C-terminal glycine-lysine dipeptide if not indicated otherwise.

In one embodiment, an antibody comprising a heavy chain including a CH3 domain, as specified herein, comprises an additional C-terminal glycine-lysine dipeptide (G446 and K447, numbering according to EU index of Kabat). In one embodiment, an antibody comprising a heavy chain including a CH3 domain, as specified herein, comprises an additional C-terminal glycine residue (G446, numbering according to EU index of Kabat).

Compositions of the invention, such as the pharmaceutical compositions described herein, comprise a population of antibodies of the invention. The population of antibodies may comprise antibodies having a full-length heavy chain and antibodies having a cleaved variant heavy chain. The population of antibodies may consist of a mixture of antibodies having a full-length heavy chain and antibodies having a cleaved variant heavy chain, wherein at least 50%, at least 60%, at least 70%, at least 80% or at least 90% of the antibodies have a cleaved variant heavy chain.

In one embodiment, a composition comprising a population of antibodies of the invention comprises an antibody comprising a heavy chain including a CH3 domain, as specified herein, with an additional C-terminal glycine-lysine dipeptide (G446 and K447, numbering according to EU index of Kabat). In one embodiment, a composition comprising a population of antibodies of the invention comprises an antibody comprising a heavy chain including a CH3 domain, as specified herein, with an additional C-terminal glycine residue (G446, numbering according to EU index of Kabat).

In one embodiment, such a composition comprises a population of antibodies comprised of antibodies comprising a heavy chain including a CH3 domain, as specified herein; antibodies comprising a heavy chain including a CH3 domain, as specified herein, with an additional C-terminal glycine residue (G446, numbering according to EU index of Kabat); and antibodies comprising a heavy chain including a CH3 domain, as specified herein, with an additional C-terminal glycine-lysine dipeptide (G446 and K447, numbering according to EU index of Kabat).

The multispecific antibodies according to the invention are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

Amino acid sequence variants (or mutants) of the multispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and antigen binding, but may further improve the yield of the recombinant production, protein stability or facilitate the purification. In certain embodiments, antibody variants having one or more conservative amino acid substitutions are provided.

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table 1 - Amino acids with specific properties**

| **Amino Acid** | **3-Letter** | **1-Letter** | **Side-chain polarity** | **Side-chain charge (pH 7.4)** |
|---|---|---|---|---|
| Alanine | Ala | A | nonpolar | neutral |
| Arginine | Arg | R | basic polar | **positive** |
| Asparagine | Asn | N | polar | neutral |
| Aspartic acid | Asp | D | acidic polar | **negative** |
| Cysteine | Cys | C | nonpolar | neutral |
| Glutamic acid | Glu | E | acidic polar | **negative** |
| Glutamine | Gln | Q | polar | neutral |
| Glycine | Gly | G | nonpolar | neutral |
| Histidine | His | H | basic polar | **positive** (10%) neutral (90%) |
| Isoleucine | Ile | I | nonpolar | neutral |
| Leucine | Leu | L | nonpolar | neutral |
| Lysine | Lys | K | basic polar | **positive** |
| Methionine | Met | M | nonpolar | neutral |
| Phenylalanine | Phe | F | nonpolar | neutral |
| Proline | Pro | P | nonpolar | neutral |
| Serine | Ser | S | polar | neutral |
| Threonine | Thr | T | polar | neutral |
| Tryptophan | Trp | W | nonpolar | neutral |
| Tyrosine | Tyr | Y | polar | neutral |
| Valine | Val | V | nonpolar | neutral |

The term "host cell" as used in the current application denotes any kind of cellular system which can be engineered to generate the antibodies according to the current invention. In one embodiment HEK293 cells and CHO cells are used as host cells. As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

A nucleic acid is "operably linked" when it is placed in a functional relationship with another nucleic acid sequence. For example, DNA for a pre-sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

Purification of antibodies is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

One aspect of the invention is a pharmaceutical composition comprising an antibody according to the invention and at least one pharmaceutically acceptable excipient. Disclosed herein is the use of an antibody according to the invention for the manufacture of a pharmaceutical composition. Also disclosed herein is a method for the manufacture of a pharmaceutical composition comprising an antibody according to the invention.

As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term cancer as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, the carrier preferably is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, F.N, et al., PNAS 69 (1972) 7110 et seq.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product.

### In the following specific embodiments of the invention are listed:

2. A multispecific antibody of human IgG1, human IgG2, human IgG3 or human IgG4 subclass, comprising:
   a) a first light chain and a first heavy chain of a first antibody which specifically binds to a first antigen; and
   b) a second light chain and a second heavy chain of a second antibody which specifically binds to a second antigen, and wherein the variable domains VL and VH in the second light chain and second heavy chain of the second antibody are replaced by each other; and
   wherein
   in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 147 or the amino acid at position 213 is substituted independently by glutamic acid (E) or aspartic acid (D) (numbering according to Kabat EU index).
2. The multispecific antibody according to embodiment 1,
   wherein in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 147 is substituted independently by glutamic acid (E) or aspartic acid (D) (numbering according to Kabat EU index).
3. The multispecific antibody according to embodiment 2,
   wherein in the constant domain CL of the first light chain under a) the amino acid at position 124 is substituted independently by lysine (K) or arginine (R) (numbering according to Kabat), and the amino acid at position 123 is substituted independently by lysine (K), or arginine (R) (numbering according to Kabat); and wherein in the constant domain CH1 of the first heavy chain under a) the amino acid at position 147 is substituted independently by glutamic acid (E) or aspartic acid (D) (numbering according to Kabat EU index) and the amino acid at position 213 is substituted independently by glutamic acid (E) or aspartic acid (D) (numbering according to Kabat EU index).
4. The multispecific antibody according to embodiment 1,
   wherein in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 213 is substituted independently by glutamic acid (E) or aspartic acid (D) (numbering according to Kabat EU index).
5. The multispecific antibody according to embodiment 1,
   wherein in the constant domain CL of the second light chain under b) the amino acid at position 124 is substituted independently by lysine (K) or arginine (R) (numbering according to Kabat); and wherein in the constant domain CH1 of the second heavy chain under b) the amino acid at position 147 or the amino acid at position 213 is substituted independently by glutamic acid (E) or aspartic acid (D) (numbering according to Kabat EU index).
6. The multispecific antibody according to embodiment 3,
   wherein in the constant domain CL of the first light chain under a) the amino acid at position 124 is substituted by lysine (K) (numbering according to Kabat) and the amino acid at position 123 is substituted by lysine (K) (numbering according to Kabat),
   and wherein in the constant domain CH1 of the first heavy chain under a) the amino acid at position 147 is substituted by glutamic acid (E) (numbering according to Kabat EU index) and the amino acid at position 213 is substituted by glutamic acid (E) (numbering according to Kabat EU index).
7. The multispecific antibody according to any one of the preceding embodiments, wherein the constant domains CL of the first light chain under a) and the second light chain under b) are of kappa isotype.
8. The multispecific antibody according to any one of embodiments 1 to 6, wherein the constant domain CL of the first light chain under a) is of lambda isotype and the constant domain CL of the second light chain under b) is of kappa isotype.
9. The multispecific antibody according to any one of embodiments 1 to 6, wherein the constant domains CL of the first light chain under a) and the second light chain under b) are of lambda isotype.
10. The multispecific antibody according to any one of the preceding embodiments wherein in the constant domain CL of the second light chain under b), in which the amino acid at position 124 is not substituted independently by lysine (K), arginine (R) or histidine (H) and which is of kappa isotype, the amino acid at position 124 is substituted independently by glutamic acid (E) or aspartic acid (D) (in one preferred embodiment by glutamic acid (E)) (numbering according to Kabat).
11. The antibody according to any one of the preceding embodiments, characterized in that a first CH3 domain of the first heavy chain of the antibody under a) and a second CH3 domain of the second heavy chain of the antibody under b) each meet at an interface which comprises an original interface between the antibody CH3 domains, wherein said interface is altered to promote the formation of the multispecific antibody, wherein the alteration is characterized in that:
   i) the CH3 domain of one heavy chain is altered, so that within the original interface of the CH3 domain of the one heavy chain that meets the original interface of the CH3 domain of the other heavy chain within the multispecific antibody, an amino acid residue is replaced with an amino acid residue having a larger side chain volume, thereby generating a protuberance within the interface of the CH3 domain of the one heavy chain which is positionable in a cavity within the interface of the CH3 domain of the other heavy chain; and
   ii) the CH3 domain of the other heavy chain is altered, so that within the original interface of the CH3 domain of the other heavy chain that meets the original interface of the CH3 domain of the one heavy chain within the multispecific antibody an amino acid residue is replaced with an amino acid residue having a smaller side chain volume, thereby generating a cavity within the interface of the CH3 domain of the other heavy chain within which a protuberance within the interface of the CH3 domain of the one heavy chain is positionable.
12. The antibody according to embodiment 11, characterized in that the said amino acid residue having a larger side chain volume is selected from the group consisting of arginine (R), phenylalanine (F), tyrosine (Y) and tryptophan (W), and said amino acid residue having a smaller side chain volume is selected from the group consisting of alanine (A), serine (S), threonine (T) and valine (V).
13. The antibody according to embodiments 11 or 12, characterized in that both CH3 domains are further altered by the introduction of cysteine (C) as amino acid in the corresponding positions of each CH3 domain such that a disulfide bridge between both CH3 domains can be formed.
14. A multispecific antibody according to any one of the preceding embodiments wherein the antibody is bispecific.
15. A multispecific antibody according to any one of the preceding embodiments that specifically binds to human TWEAK and that specifically binds to human IL17, wherein
   A) the multispecific antibody comprises a variable heavy chain domain (VH) of SEQ ID NO:24, and a variable light chain domain (VL) of SEQ ID NO:25; and
   B) the multispecific antibody comprises a variable heavy chain domain (VH) of SEQ ID NO:26, and a variable light chain domain (VL) of SEQ ID NO:27.
16. A bispecific antibody that comprises
   a) a first light chain and a first heavy chain of a first antibody which specifically binds to a human TWEAK, which comprises a variable heavy chain domain (VH) of SEQ ID NO:24, and a variable light chain domain (VL) of SEQ ID NO:25; and
   b) the second light chain and the second heavy chain of a second antibody which specifically binds to a human IL-17, which comprises a variable heavy chain domain (VH) of SEQ ID NO:26, and a variable light chain domain (VL) of SEQ ID NO:27; wherein the variable domains VL and VH in the second light chain and second heavy chain of the second antibody are replaced by each other; and
   wherein in the constant domain CL of the first light chain under a) the amino acid at position 124 is substituted independently by lysine (K) or arginine (R), and wherein in the constant domain CH1 of the first heavy chain under a) the amino acid at position 147 or the amino acid at position 213 is substituted independently by glutamic acid (E) or aspartic acid (D) (numbering according to Kabat EU index).
17. The bispecific antibody according to embodiment 17, wherein
   in the constant domain CL of the first light chain under a) the amino acid at position 124 is substituted independently by lysine (K) or arginine (R) (numbering according to Kabat), and the amino acid at position 123 is substituted independently by lysine (K) or arginine (R) (numbering according to Kabat); and wherein in the constant domain CH1 of the first heavy chain under a) the amino acid at position 147 is substituted independently by glutamic acid (E) or aspartic acid (D) (numbering according to Kabat EU index) and the amino acid at position 213 is substituted independently by glutamic acid (E) or aspartic acid (D) (numbering according to Kabat EU index).
18. An antibody according to any one of embodiments 15 to 17 for use in the treatment of cancer, or inflammatory diseases, autoimmune diseases, rheumatoid arthritis, psoratic arthritis, muscle diseases, e.g. muscular dystrophy, multiple sclerosis, chronic kidney diseases, bone diseases, e.g. bone degeneration in multiple myeloma, systemic lupus erythematosus, lupus nephritis, and vascular injury.
19. Use of an antibody according to any one of embodiments 15 to 17 for manufacture of a medicament for the treatment of cancer, or inflammatory diseases, autoimmune diseases, rheumatoid arthritis, psoratic arthritis, muscle diseases, e.g. muscular dystrophy, multiple sclerosis, chronic kidney diseases, bone diseases, e.g. bone degeneration in multiple myeloma, systemic lupus erythematosus, lupus nephritis, and vascular injury.
20. The multispecific antibody according to any one of embodiments 1 to 17, characterized in that it is of human IgG1 or human IgG4 subclass.
21. The multispecific antibody according to any one of embodiments 1 to 17 and 20, characterized in being of human IgG1 subclass with the mutations L234A and L235A (numbering according to Kabat EU index).
22. The multispecific antibody according to any one of embodiments 1 to 17 and 20 to 21, characterized in being of human IgG1 subclass with the mutations L234A, L235A and P329G (numbering according to Kabat EU index).
23. The multispecific antibody according to any one of preceding embodiments 1 to 17 and 20, characterized in being of human IgG4 subclass with the mutations S228P and L235E (numbering according to Kabat EU index).
24. The multispecific antibody according to any one of embodiments 1 to 17, and 20 to 23, characterized in being of human IgG4 subclass with the mutations S228P, L235E and P329G (numbering according to Kabat EU index).
25. A method for the preparation of a multispecific antibody according to any one of embodiments 1 to 17 and 20 to 23,
   comprising the steps of
   A) transforming a host cell with vectors comprising nucleic acid molecules encoding
      a) the first light chain and the first heavy chain of a first antibody which specifically binds to a first antigen; and
      b) the second light chain and the second heavy chain of a second antibody which specifically binds to a second antigen, and wherein the variable domains VL and VH in the second light chain and second heavy chain of the second antibody are replaced by each other; and
      wherein
      in the constant domain CL of the first light chain under a) the amino acid at position 124 is substituted independently by lysine (K), arginine (R) or histidine (H) (numbering according to Kabat) (in one preferred embodiment independently by lysine (K) or arginine (R)), and wherein in the constant domain CH1 of the first heavy chain under a) the amino acid at position 147 or the amino acid at position 213 is substituted independently by glutamic acid (E) or aspartic acid (D) (numbering according to Kabat EU index);
   B) culturing the host cell under conditions that allow synthesis of said antibody molecule; and
   C) recovering said antibody molecule from said culture.
26. Nucleic acid encoding the amino acid sequences of a multispecific antibody according to any one of embodiments 1 to 17 and 20 to 23.
27. Expression vector containing the nucleic acid according to embodiment 26 capable of expressing said nucleic acid in a host cell.
28. A host cell comprising a vector according to embodiment 27.
29. A pharmaceutical composition comprising an antibody according to any one of embodiments 1 to 17 and 20 to 23 and at least one pharmaceutically acceptable excipient.
30. The multispecific antibody according to any one of embodiments 1 to 10, and 14 to 17, wherein the antibody comprises at least two Fab fragments, wherein the first Fab fragment comprises at least one antigen binding site specific for a first antigen; and the second Fab fragment comprises at least one antigen binding site specific for a second antigen, wherein in the second Fab fragment the variable domains VL and VH in the second light chain and second heavy chain are replaced by each other; and wherein the multispecific antibody is devoid of an Fc domain.
31. The multispecific antibody according to embodiment 30, wherein the antibody comprises two to four Fab fragments.
32. The multispecific antibody according to embodiment 30 or 31, wherein the antibody specifically binds to human Ang-2 and VEGF.
33. A method of producing an antibody comprising culturing the host cell of embodiment 32 so that the antibody is produced.
34. The method of embodiment 33, further comprising recovering the antibody from the host cell.

The following examples, sequence listing and figures are provided to aid the understanding of the present invention, the true scope of which is set forth in the appended claims.

**Description of the Amino acid Sequences**

| | |
|---|---|
| **SEQ ID NO:1** | light chain (LC) <Ang-2> wild type (wt) |
| **SEQ ID NO:2** | heavy chain (HC) <Ang-2> wild type (wt) |
| **SEQ ID NO:3** | heavy chain (HC) <VEGF> with VH-VL exchange wild type (wt) |
| **SEQ ID NO:4** | light chain (LC) <VEGF> with VH-VL exchange wild type (wt) |
| **SEQ ID NO:5** | light chain (LC) <Ang-2> with Q124K substitution |
| **SEQ ID NO:6** | heavy chain (HC) <Ang-2> with K147E substitution |
| **SEQ ID NO:7** | heavy chain (HC) <Ang-2> with K213E substitution |
| **SEQ ID NO:8** | light chain (LC) <Ang-2> with E123K substitution |
| **SEQ ID NO:9** | light chain (LC) <Ang-2> with Q124K substitution and E123K substitution |
| **SEQ ID NO:10** | heavy chain (HC) <Ang-2> with K147E substitution and K213E substitution |
| **SEQ ID NO:11** | light chain (LC) <Ang-2> with Q124R substitution and E123K substitution |
| **SEQ ID NO:12** | light chain (LC) <VEGF> with Q124E substitution |
| **SEQ ID NO:13** | light chain (LC) <Ang-2> with E124K substitution and E123K substitution |
| **SEQ ID NO:14** | heavy chain (HC) <Ang-2> with K147E substitution and K213D substitution |
| **SEQ ID NO:15** | light chain (LC) <IL-17> wild type (wt) |
| **SEQ ID NO:16** | heavy chain (HC) <IL-17> wild type (wt) |
| **SEQ ID NO:17** | heavy chain (HC) <TWEAK> with VH-VL exchange wild type (wt) |
| **SEQ ID NO:18** | light chain (LC) <TWEAK> with VH-VL exchange wild type (wt) |
| **SEQ ID NO:19** | light chain (LC) <IL-17> with Q124K substitution and E123R substitution |
| **SEQ ID NO:20** | heavy chain (HC) <IL-17> with K147E substitution and K213E substitution |
| **SEQ ID NO:21** | light chain (LC) <TWEAK> with Q124E substitution |
| **SEQ ID NO:22** | heavy chain (HC) <IL-17> with K147E substitution and K213D substitution |
| **SEQ ID NO:23** | light chain (LC) <IL-17> with Q124K substitution and E123K substitution |
| **SEQ ID NO:24** | variable heavy chain domain VH <TWEAK > 305-HC4 |
| **SEQ ID NO:25** | variable light chain domain VL <TWEAK>305-LC2 |
| **SEQ ID NO:26** | variable heavy chain domain VH <IL-17> HC136 |
| **SEQ ID NO:27** | variable light chain domain VL <IL-17> LC136 |
| **SEQ ID NO: 28** | heavy chain (HC) <TWEAK> with VH-VL exchange wild type (wt) (comprising terminal GK dipeptide) |
| **SEQ ID NO: 29** | heavy chain (HC) <IL-17> with K147E substitution and K213E substitution (comprising terminal GK dipeptide) |
| **SEQ ID NO: 30** | heavy chain (HC) <IL-17> with K147E substitution and K213D substitution (comprising terminal GK dipeptide) |
| **SEQ ID NO: 31** | heavy chain (HC) <Ang-2> wild type (wt) (comprising terminal GK dipeptide) |
| **SEQ ID NO: 32** | heavy chain (HC) <VEGF> with VH-VL exchange wild type (wt) (comprising terminal GK dipeptide) |
| **SEQ ID NO: 33** | heavy chain (HC) <Ang-2> with K147E substitution (comprising terminal GK dipeptide) |
| **SEQ ID NO: 34** | heavy chain (HC) <Ang-2> with K213E substitution (comprising terminal GK dipeptide) |
| **SEQ ID NO: 35** | heavy chain (HC) <Ang-2> with K147E substitution and K213E substitution (comprising terminal GK dipeptide) |
| **SEQ ID NO: 36** | heavy chain (HC) <Ang-2> with K147E substitution and K213D substitution (comprising terminal GK dipeptide) |
| **SEQ ID NO: 37** | heavy chain (HC) <IL-17> wild type (wt) (comprising terminal GK dipeptide) |
| **SEQ ID NO: 38** | Fab₂-CrossFab heavy chain (HC) including two heavy chains (HC) <Ang-2> wild type (wt) coupled to one heavy chain (HC) <VEGF> with VH-VL exchange wild type (wt) via glycine-serine-linkers |
| **SEQ ID NO: 39** | Fab₂-CrossFab heavy chain (HC) including two heavy chains (HC) <Ang-2> with K147E and K213E substitutions coupled to one heavy chain (HC) <VEGF> with VH-VL exchange wild type (wt) via glycine-serine-linkers |
| **SEQ ID NO: 40** | CrossFab-Fab heavy chain (HC) including one heavy chain (HC) <VEGF> with VH-VL exchange wild type (wt) coupled to one heavy chain (HC) <Ang-2> with K147E and K213E substitutions via glycine-serine-linkers |
| **SEQ ID NO: 41** | CrossFab-Fab heavy chain (HC) including one heavy chain (HC) <VEGF> with VH-VL exchange wild type (wt) coupled to one heavy chain (HC) <Ang-2> with K147E and K213E substitutions via glycine-serine-linkers |
| **SEQ ID NO: 42** | 2 CrossFab₂-Fab heavy chain (HC) including two heavy chains (HC) <VEGF> with VH-VL exchange wild type (wt) coupled to one heavy chain (HC) <Ang-2> wild type (wt) via glycine-serinee-linkers |
| **SEQ ID NO: 43** | CrossFab₂-Fab heavy chain (HC) including two heavy chains (HC) <VEGF> with VH-VL exchange wild type (wt) coupled to one heavy chain (HC) <Ang-2> with K147E and K231E substitutions via glycine-serine-linkers |
| **SEQ ID NO: 44** | heavy chain (HC) <VEGF> with VH-VL exchange with K147E substitution |
| **SEQ ID NO: 45** | light chain (LC) <VEGF> with VH-VL exchange with Q124K substitution |
| **SEQ ID NO: 46** | heavy chain (HC) <VEGF> with VH-VL exchange with K147E, and K213E substitution |
| **SEQ ID NO: 47** | light chain (LC) <VEGF> with VH-VL exchange with E123K, and Q124K substitution |

### Examples

### Materials & general methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the numbering systems according to Kabat (Kabat, E.A., et al., Sequences of Proteins of Immunological Interest, 5th ed., Public Health Service, National Institutes of Health, Bethesda, MD (1991)) as defined above.

### Recombinant DNA techniques

Standard methods were used to manipulate DNA as described in Sambrook, J. et al., Molecular Cloning: A laboratory manual; Cold Spring Harbor Laboratory Press, Cold Spring Harbor, New York, 1989. The molecular biological reagents were used according to the manufacturer's instructions.

### Gene synthesis

Desired gene segments were prepared from oligonucleotides made by chemical synthesis. The 600 - 1800 bp long gene segments, which were flanked by singular restriction endonuclease cleavage sites, were assembled by annealing and ligating oligonucleotides including PCR amplification and subsequently cloned via the indicated restriction sites e.g. KpnI/ SacI or AscI/PacI into a pPCRScript (Stratagene) based pGA4 cloning vector. The DNA sequences of the subcloned gene fragments were confirmed by DNA sequencing. Gene synthesis fragments were ordered according to given specifications at Geneart (Regensburg, Germany).

### DNA sequence determination

DNA sequences were determined by double strand sequencing performed at MediGenomix GmbH (Martinsried, Germany) or Sequiserve GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 was used for sequence creation, mapping, analysis, annotation and illustration.

### Expression vectors

For the expression of the described antibodies, variants of expression plasmids for transient expression (e.g. in HEK293 EBNA or HEK293-F) cells based either on a cDNA organization with or without a CMV-Intron A promoter or on a genomic organization with a CMV promoter were applied.

Beside the antibody expression cassette the vectors contained:
- an origin of replication which allows replication of this plasmid in *E. coli*, and
- a β-lactamase gene which confers ampicillin resistance in *E. coli.*

The transcription unit of the antibody gene was composed of the following elements:
- unique restriction site(s) at the 5' end
- the immediate early enhancer and promoter from the human cytomegalovirus,
- followed by the Intron A sequence in the case of the cDNA organization,
- a 5'-untranslated region of a human antibody gene,
- an immunoglobulin heavy chain signal sequence,
- the human antibody chain (wildtype or with domain exchange) either as cDNA or as genomic organization with the immunoglobulin exon-intron organization
- a 3' untranslated region with a polyadenylation signal sequence, and
- unique restriction site(s) at the 3' end.

The fusion genes comprising the antibody chains as described below were generated by PCR and/or gene synthesis and assembled by known recombinant methods and techniques by connection of the according nucleic acid segments e.g. using unique restriction sites in the respective vectors. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

Multispecific antibodies were expressed by transient co-transfection of the respective expression plasmids in adherently growing HEK293-EBNA or in HEK29-F cells growing in suspension as described below.

### Transient transfections in HEK293-EBNA system

Multispecific antibodies were expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear antigen; American type culture collection deposit number ATCC # CRL-10852, Lot. 959 218) cultivated in DMEM (Dulbecco's modified Eagle's medium, Gibco®) supplemented with 10% Ultra Low IgG FCS (fetal calf serum, Gibco®), 2 mM L-Glutamine (Gibco®), and 250 µg/ml Geneticin (Gibco®). For transfection FuGENE™ 6 Transfection Reagent (Roche Molecular Biochemicals) was used in a ratio of FuGENE™ reagent (µl) to DNA (µg) of 4:1 (ranging from 3:1 to 6:1). Proteins were expressed from the respective plasmids using a molar ratio of (modified and wildtype) light chain and heavy chain encoding plasmids of 1:1 (equimolar) ranging from 1:2 to 2:1, respectively. Cells were fed at day 3 with L-Glutamine ad 4 mM, Glucose [Sigma] and NAA [Gibco®]. Multispecific antibody containing cell culture supernatants were harvested from day 5 to 11 after transfection by centrifugation and stored at -20°C. General information regarding the recombinant expression of human immunoglobulins in e.g. HEK293 cells is given in: Meissner, P. et al., Biotechnol. Bioeng. 75 (2001) 197-203.

### Transient transfections in HEK293-F system

Multispecific antibodies were generated by transient transfection with the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serum-free FreeStyle™ 293 expression medium (Invitrogen) were transfected with a mix of the four expression plasmids and 293fectin™ or fectin (Invitrogen). For 2 L shake flask (Corning) HEK293-F cells were seeded at a density of 1.0E^{∗}6 cells/mL in 600 mL and incubated at 120 rpm, 8% CO2. The day after the cells were transfected at a cell density of ca. 1.5E^{∗}6 cells/mL with ca. 42 mL mix of A) 20 mL Opti-MEM (Invitrogen) with 600 µg total plasmid DNA (1 µg/mL) encoding the heavy or modified heavy chain, respectively and the corresponding light chain in an equimolar ratio and B) 20 ml Opti-MEM + 1.2 mL 293 fectin or fectin (2 µl/mL). According to the glucose consumption glucose solution was added during the course of the fermentation. The supernatant containing the secreted antibody was harvested after 5-10 days and antibodies were either directly purified from the supernatant or the supernatant was frozen and stored.

### Protein determination

The protein concentration of purified antibodies and derivatives was determined by determining the optical density (OD) at 280 nm, using the molar extinction coefficient calculated on the basis of the amino acid sequence according to Pace, et al., Protein Science, 1995, 4, 2411-1423.

### Antibody concentration determination in supernatants

The concentration of antibodies and derivatives in cell culture supernatants was estimated by immunoprecipitation with Protein A Agarose-beads (Roche). 60 µL Protein A Agarose beads were washed three times in TBS-NP40 (50 mM Tris, pH 7.5, 150 mM NaCl, 1% Nonidet-P40). Subsequently, 1 -15 mL cell culture supernatant were applied to the Protein A Agarose beads pre-equilibrated in TBS-NP40. After incubation for at 1 hour at room temperature the beads were washed on an Ultrafree-MC-filter column (Amicon) once with 0.5 mL TBS-NP40, twice with 0.5 mL 2x phosphate buffered saline (2xPBS, Roche) and briefly four times with 0.5 mL 100 mM Na-citrate pH 5,0. Bound antibody was eluted by addition of 35 µl NuPAGE® LDS Sample Buffer (Invitrogen). Half of the sample was combined with NuPAGE® Sample Reducing Agent or left unreduced, respectively, and heated for 10 min at 70°C. Consequently, 5-30 µl were applied to a 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.

The concentration of antibodies and derivatives in cell culture supernatants was quantitatively measured by affinity HPLC chromatography. Briefly, cell culture supernatants containing antibodies and derivatives that bind to Protein A were applied to an Applied Biosystems Poros A/20 column in 200 mM KH2PO4, 100 mM sodium citrate, pH 7.4 and eluted from the matrix with 200 mM NaCl, 100 mM citric acid, pH 2,5 on an Agilent HPLC 1100 system. The eluted protein was quantified by UV absorbance and integration of peak areas. A purified standard IgG1 antibody served as a standard.

Alternatively, the concentration of antibodies and derivatives in cell culture supernatants was measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcγ> BI (Dianova) at 0.1 µg/mL for 1 hour at room temperature or alternatively overnight at 4°C and subsequently washed three times with 200 µL/well PBS, 0.05% Tween (PBST, Sigma). 100 µL/well of a dilution series in PBS (Sigma) of the respective antibody containing cell culture supernatants was added to the wells and incubated for 1-2 hour on a microtiterplate shaker at room temperature. The wells were washed three times with 200 µL/well PBST and bound antibody was detected with 100 µl F(ab')2<hFcγ>POD (Dianova) at 0.1 µg/mL as the detection antibody for 1-2 hours on a microtiterplate shaker at room temperature. Unbound detection antibody was washed away three times with 200 µL/well PBST and the bound detection antibody was detected by addition of 100 µL ABTS/well. Determination of absorbance was performed on a Tecan Fluor Spectrometer at a measurement wavelength of 405 nm (reference wavelength 492 nm).

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VH/VL exchange (VH/VL CrossMabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Determination of binding and binding affinity of multispecific antibodies to the respective antigens using surface plasmon resonance (SPR) (BIACORE)

Binding of the generated antibodies to the respective antigens (e.g ANG2 and VEGF) is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C) Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

### Exammple 1A

### Production and expression of multispecific antibodies which bind to Angiopoietin-2 (ANG2) and VEGF with VH/VL domain exchange/replacement (CrossMAb^{Vh-VL}) in one binding arm and with single charged amino acid substitutions in the CH1/CL interface

In a first example multispecific antibodies which binds to human Angiopoietin-2 (ANG2) and human VEGF were generated as described in the general methods section by classical molecular biology techniques and is expressed transiently in HEK293 cells as described above. A general scheme of these respective multispecific, antibodies is given in Figures 1A to C. For comparison also the wild type (wt) VH/VL domain exchange/replacement antibodies with no substitution in the CH1/CL interface was prepared. Also other alternative substitutions in close proximity in the CH1CL interface (mentioned e.g. in EP 2647707) were used for comparison. The multispecific antibodies were expressed using expression plasmids containing the nucleic acids encoding the amino acid sequences depicted in Table 2a.

**Table 2a: Amino acid sequences of light chains (LC) and heavy chains (HC) of anti-Ang2-VEGF multispecific antibodies Ang2VEGF-0273, Ang2VEGF-0396,Ang2VEGF-0397, Ang2VEGF-0394, Ang2VEGF-0395 with VH/VL domain exchange/replacement (CrossMAb^{Vh-VL}): wild type (wt) and different combinations of single charged amino acids substitutions**

| **Antibody** | **LC ANG-2** | **HC ANG-2** | **HC VEGF** | **LC VEGF** |
|---|---|---|---|---|
| Ang2VEGF-0273 | SEQ ID NO: 1 | SEQ ID NO: 2 | SEQ ID NO: 3 | SEQ ID NO: 4 |
| Ang2VEGF-0396 | SEQ ID NO: 5 | SEQ ID NO: 6 | SEQ ID NO: 3 | SEQ ID NO: 4 |
| Ang2VEGF-0397 | SEQ ID NO: 5 | SEQ ID NO: 7 | SEQ ID NO: 3 | SEQ ID NO: 4 |
| Ang2VEGF-0394 | SEQ ID NO: 8 | SEQ ID NO: 6 | SEQ ID NO: 3 | SEQ ID NO: 4 |
| Ang2VEGF-0395 | SEQ ID NO: 8 | SEQ ID NO: 7 | SEQ ID NO: 3 | SEQ ID NO: 4 |

For all constructs knobs into holes heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above)

### Example 1B

### Purification and characterization of multispecific antibodies which bind to Angiopoietin-2 (ANG2) and VEGF with VH/VL domain exchange/replacement (CrossMAb^{Vh-VL}) in one binding arm and with single charged amino acid substitutions in the CH1/CL interface

The multispecific antibodies expressed above were purified from the supernatant by a combination of Protein A affinity chromatography and size exclusion chromatography. All multispecific antibodies can be produced in good yields and are stable.

The obtained products were characterized for identity by mass spectrometry and analytical properties such as purity by SDS-PAGE, monomer content and stability

### Mass spectrometry

The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

Results are shown in Table 2b and Figure 4a.

**Table 2b: Reduction of main Bence-Jones-type side product by single charged amino acids substitutions according to the invention in the CH1/CL interface**

| | **CL ANG-2 (position 124)** | **CL ANG-2 (position 123)** | **CH1 ANG-2 (position 147)** | **CH1 ANG-2 (position 213)** | **CH1 VEGF** | **CL VEGF** | **Main side product (Bence-Jones type mispairing) % by MS** |
|---|---|---|---|---|---|---|---|
| Ang2VEGF-0273 | wt: Q124 | wt: E123 | wt: K147 | wt: K213 | wt | wt | **∼20** |
| Ang2VEGF-0396 | **Q124K** | wt | **K147E** | wt | wt | wt | **_{∼}3** |
| Ang2VEGF-0397 | **Q124K** | wt | wt | **K213E** | wt | wt | **_{∼}3** |
| Ang2VEGF-0394 | wt | **E123K** | **K147E** | wt | wt | wt | **_{∼}15** |
| Ang2VEGF-0395 | wt | **E123K** | wt | **K213E** | wt | wt | **_{∼}15** |

Results in Table 2b and Figure 4a show that with the substitutions of single charged amino acids with the opposite charge in the CH1 and CL domains according to the invention/as described for the invention (CL:Q124K and CH1:K147E pair; or CL:Q124K and CH1:K213E pair) the main side product (Bence-Jones type mispairing) is strongly reduced when compared to the wild type multispecific antibody without such substitutions (∼ 17% reduction). With other substitutions in close proximity (CL:Q123K and CH1:K147E pair; or CL:Q123K and CH1 :K213E pair) only a slight reduction of the main side product compared to the wild type multispecific antibody without such substitutions (∼ 5% reduction).

### Example 1C

### Antigen binding properties of multispecific antibodies which bind to Angiopoietin-2 (ANG2) and VEGF with VH/VL domain exchange/replacement (CrossMAb^{Vh-vL}) in one binding arm and with single charged amino acid substitutions in the CH1/CL interface

Binding of the multispecific antibodies of the previous examples 1A and 1B to their respective target antigens, i.e. ANG2 and VEGF, was assessed by Biacore®.

### VEGF binding was assessed according to the following procedure:

Binding of indicated antibodies to human VEGFA-121 was investigated by surface plasmon resonance using a BIACORE® T200 instrument (GE Healthcare). Around 10000 (RU) of anti His antibody (1 µg/ml anti His antibody; Order Code: 28995056; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

VEFGA-121-His was captured by injecting a 0.5 µg/ml solution for 30 sec at a flow of 5 µl/min. The association was measured by injection of the indicated antibodies in various concentrations in solution for 180 sec at a flow of 30 µl/min starting with 1000 nM in 1:3 serial dilutions. The dissociation phase was monitored for up to 600 sec and triggered by switching from the sample solution to running buffer. The surface was regenerated by 60 sec washing with a Glycine pH 1.5 solution at a flow rate of 30 µl/min. Bulk refractive index differences were corrected by subtracting the response obtained from a anti His antibody surface. Blank injections are also subtracted (= double referencing). For calculation of K_{D} and other kinetic parameters the Langmuir 1:1 model was used.

### Ang-2 binding was assessed according to the following procedure:

Binding of indicated antibodies to human Ang-2-RBD-Fc was investigated by surface plasmon resonance using a BIACORE® T200 instrument (GE Healthcare). Around 8000 (RU) of goat anti human F(ab')₂ (10 µg/ml anti human F(ab)'₂; Order Code: 28958325; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The bispecific antibody was captured by injecting a 5 nM solution for 25 sec at a flow of 5 µl/min. The association was measured by injection of human Ang2-RBD-Fc in various concentrations in solution for 120 sec at a flow of 30 µl/min starting with 100 nM in 1:3 serial dilutions. The dissociation phase was monitored for up to 180 sec and triggered by switching from the sample solution to running buffer. The surface was regenerated by 60 sec washing with a Glycine pH 2.1 solution at a flow rate of 30 µl/min. Bulk refractive index differences were corrected by subtracting the response obtained from a goat anti human F(ab')₂ surface. Blank injections are also subtracted (= double referencing). For calculation of apparent K_{D} the Langmuir 1:1 model was used.

As comparative example, a reference antibody specifically binding to Ang2 and VEGF comprising a VH/VL domain exchange/replacement but lacking charged amino acid substitutions (Ang2VEGF-0273 antibody of **Table 2b**) was assessed in parallel.

Results are indicated in **Tables 2c and 2d**.

**Table 2c: Affinity for VEGF of indicated antibodies**

| **Sample** | **KD (nM)** |
|---|---|
| Ang2VEGF-0273 | 6 |
| Ang2VEGF-0396 | 3 |
| Ang2VEGF-0397 | 4 |
| Ang2VEGF-0394 | 3 |
| Ang2VEGF-0395 | 4 |

**Table 2d: Affinity for Ang2 of indicated antibodies**

| **Sample** | **KD (nM)** |
|---|---|
| Ang2VEGF-0273 | 15 |
| Ang2VEGF-0396 | 17 |
| Ang2VEGF-0397 | 14 |
| Ang2VEGF-0394 | 12 |
| Ang2VEGF-0395 | 15 |

All tested antibodies specifically bind to both targets, Ang2 and VEGF, and exhibit an antigen affinity in the nanomolar range.

### Example 1D

### Stability of multispecific antibodies which bind to Angiopoietin-2 (ANG2) and VEGF with VH/VL domain exchange/replacement (CrossMAb^{Vh-VL}) in one binding arm and with single charged amino acid substitutions in the CH1/CL interface

In order to assess stability of the antibody constructs, thermal stability as well as aggregation onset temperatures were assessed according to the following procedure.

Samples of the indicated antibodies were prepared at a concentration of 1 mg/mL in 20 mM Histidine/Histidine chloride, 140 mM NaCl, pH 6.0, transferred into a 10 µL micro-cuvette array and static light scattering data as well as fluorescence data upon excitation with a 266 nm laser were recorded with an Optim1000 instrument (Avacta Inc.), while the samples were heated at a rate of 0.1 °C/min from 25°C to 90°C.

The aggregation onset temperature (T_{agg}) is defined as the temperature at which the scattered light intensity starts to increase. The melting temperature (Tₘ) is defined as the inflection point in a fluorescence intensity vs. wavelength graph.

Results are shown in **Table 2e**.

**Table 2e: Aggregation onset temperature (T_{agg}) and melting temperature (Tₘ) of indicated antibodies**

| **Sample** | **T_{agg} (°C)** | **Tₘ (°C)** |
|---|---|---|
| Ang2VEGF-0273 | 56,0 | 61,3 |
| Ang2VEGF-0396 | 56,9 | 62,0 |
| Ang2VEGF-0397 | 56,0 | 61,7 |
| Ang2VEGF-0394 | 56,9 | 62,2 |
| Ang2VEGF-0395 | 56,8 | 62,1 |

### Example 1E

### Production yield of multispecific antibodies which bind to Angiopoietin-2 (ANG2) and VEGF with VH/VL domain exchange/replacement (CrossMAb^{Vh-VL}) in one binding arm and with single charged amino acid substitutions in the CH1/CL interface

Production yields of the indicated multispecific antibodies were assessed after Protein A purification (ProtA). Results are shown in **Table 2f**.

**Table 2e: Production yields [mg/L supernatant] of indicated antibodies**

| **Sample** | **ProtA** |
|---|---|
| Ang2VEGF-0273 | 65 |
| Ang2VEGF-0396 | 80.8 |
| Ang2VEGF-0397 | 68.4 |
| Ang2VEGF-0394 | 79.2 |
| Ang2VEGF-0395 | 93.6 |

### Example 2A

### Production and expression of multispecific antibodies which bind to Angiopoietin-2 (ANG2) and VEGF with VH/VL domain exchange/replacement (CrossMAb^{Vh-VL}) in one binding arm and with different charged amino acid substitutions in the CH1/CL interface

In a first example multispecific antibodies which binds to human Angiopoietin-2 (ANG2) and human VEGF were generated as described in the general methods section by classical molecular biology techniques and is expressed transiently in HEK293 cells as described above. A general scheme of these respective multispecific, antibodies is given in Figures 1A to C. For comparison also the wild type (wt) VH/VL domain exchange/replacement antibodies with no substitution in the CH1/CL interface was prepared. The multispecific antibodies were expressed using expression plasmids containing the nucleic acids encoding the amino acid sequences depicted in Table 3a.

**Table 3a: Amino acid sequences of light chains (LC) and heavy chains (HC) of anti-Ang2-VEGF multispecific antibodies Ang2VEGF-0273, Ang2VEGF-0274, Ang2VEGF-0282, Ang2VEGF-0283, Ang2VEGF-0284, Ang2VEGF-0285, Ang2VEGF-0286 with VH/VL domain exchange/replacement (CrossMAb^{Vh-VL}) : wild type (wt) and different combinations of charged amino acids substitutions**

| **Antibody** | **LC ANG-2** | **HC ANG-2** | **HC VEGF** | **LC VEGF** |
|---|---|---|---|---|
| Ang2VEGF-0273 | SEQ ID NO: 1 | SEQ ID NO: 2 | SEQ ID NO: 3 | SEQ ID NO: 4 |
| Ang2VEGF-0274 | SEQ ID NO: 9 | SEQ ID NO: 10 | SEQ ID NO: 3 | SEQ ID NO: 4 |
| Ang2VEGF-0282 | SEQ ID NO: 11 | SEQ ID NO: 10 | SEQ ID NO: 3 | SEQ ID NO: 12 |
| Ang2VEGF-0283 | SEQ ID NO: 13 | SEQ ID NO: 10 | SEQ ID NO: 3 | SEQ ID NO: 4 |
| Ang2VEGF-0284 | SEQ ID NO: 11 | SEQ ID NO: 14 | SEQ ID NO: 3 | SEQ ID NO: 4 |
| Ang2VEGF-0285 | SEQ ID NO: 11 | SEQ ID NO: 14 | SEQ ID NO: 3 | SEQ ID NO: 12 |
| Ang2VEGF-0286 | SEQ ID NO: 9 | SEQ ID NO: 10 | SEQ ID NO: 3 | SEQ ID NO: 12 |

For all constructs knobs into holes heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 2B

### Purification and characterization of multispecific antibodies which bind to Angiopoietin-2 (ANG2) and VEGF with VH/VL domain exchange/replacement (CrossMAb^{Vh-VL}) in one binding arm and with different charged amino acid substitutions in the CH1/CL interface

The multispecific antibodies expressed above were purified from the supernatant by a combination of Protein A affinity chromatography and size exclusion chromatography. All multispecific antibodies can be produced in good yields and are stable.

The obtained products were characterized for identity by mass spectrometry and analytical properties such as purity by SDS-PAGE, monomer content and stability

### Mass spectrometry

The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

Results are shown in Table 3b and Figure 5a.

**Table 3b: Reduction of main Bence-Jones-type side product by single charged amino acids substitutions according to the invention in the CH1/CL interface**

| | **CL ANG-2** | **CL ANG-2** | **CH1 ANG-2** | **CH1 ANG-2** | **CH1 VEGF** | **CL VEGF** | **Main side product (Bence-Jones type mispairing) % by MS** |
|---|---|---|---|---|---|---|---|
| Ang2VEGF-0273 | wt: Q124 (kappa) | wt: E123 | wt: K147 | wt: K213 | wt | wt: Q124 | **∼20%** |
| Ang2VEGF-0274 | **Q124K** | **E123K** | **K147E** | **K213E** | wt | wt | **0** |
| Ang2VEGF-0282 | **Q124R** | **E123K** | **K147E** | **K213E** | wt | **Q124E** | **0** |
| Ang2VEGF-0283 | **E124K** (lambda) | **E123K** | **K147E** | **K213E** | wt | wt: | **0** |
| Ang2VEGF-0284 | **Q124R** | **E123K** | **K147E** | **K213D** | wt | wt | **0** |
| Ang2VEGF-0285 | **Q124R** | **E123K** | **K147E** | **K213D** | wt | **Q124E** | **0** |
| Ang2VEGF-0286 | **Q124K** | **E123K** | **K147E** | **K213E** | wt | **Q124E** | **0** |

Results in Table 3b and Figure 5a show that with the double substitutions of charged amino acids with the opposite charge in the CH1 and CL domains according to the invention/as described for the invention (CL:Q124K/E123K and CH1:K147E/K213E; CL:Q124R/E123K and CH1:K147E/K213E; CL:Q124R/E123K and CH1:K147E/K213D) the main side product (Bence-Jones type mispairing) is completely removed when compared to the wild type multispecific antibody without such substitutions. This is independent of the further single substitution Q124E in the CL domain of the other binding arm, which does not influence the expression nor side product profile.

### Example 2C

### Antigen binding properties of multispecific antibodies which bind to Angiopoietin-2 (ANG2) and VEGF with VH/VL domain exchange/replacement (CrossMAb^{Vh-VL}) in one binding arm and with different charged amino acid substitutions in the CH1/CL interface

Binding of the multispecific antibodies of the previous examples 2A and 2B to their respective target antigens, i.e. ANG2 and VEGF, was assessed by Biacore® as outlined in example 1C.

As comparative example, the reference antibody specifically binding to Ang2 and VEGF comprising a VH/VL domain exchange/replacement but lacking charged amino acid substitutions (Ang2VEGF-0273 antibody of **Table 2b**) was assessed in parallel.

Results are indicated in **Tables 3c and 3d.**

**Table 3c: Affinity for VEGF of indicated antibodies**

| **Sample** | **KD (nM)** |
|---|---|
| Ang2VEGF-0273 | 6 |
| Ang2VEGF-0274 | 3 |
| Ang2VEGF-0282 | 4 |
| Ang2VEGF-0283 | 4 |
| Ang2VEGF-0284 | 4 |
| Ang2VEGF-0285 | 4 |
| Ang2VEGF-0286 | 4 |

**Table 3d: Affinity for Ang2 of indicated antibodies**

| **Sample** | **KD (nM)** |
|---|---|
| Ang2VEGF-0273 | 15 |
| Ang2VEGF-0274 | 17 |
| Ang2VEGF-0282 | 14 |
| Ang2VEGF-0283 | 15 |
| Ang2VEGF-0284 | 13 |
| Ang2VEGF-0285 | 14 |
| Ang2VEGF-0286 | 12 |

All tested antibodies specifically bind to both targets, Ang2 and VEGF, and exhibit an antigen affinity in the nanomolar range.

### Example 2D

### Stability of multispecific antibodies which bind to Angiopoietin-2 (ANG2) and VEGF with VH/VL domain exchange/replacement (CrossMAb^{Vh-VL}) in one binding arm and with single charged amino acid substitutions in the CH1/CL interface

In order to assess stability of the antibody constructs, thermal stability as well as aggregation onset temperatures were assessed as outlined in example 1D.

Results are shown in **Table 3e**.

**Table 3e: Aggregation onset temperature (T_{agg}) and melting temperature (Tₘ) of indicated antibodies**

| **Sample** | **T_{agg} (°C)** | **Tₘ (°C)** |
|---|---|---|
| Ang2VEGF-0273 | 56,0 | 61,3 |
| Ang2VEGF-0274 | 53,5 | 58,9 |
| Ang2VEGF-0282 | 56,9 | 61,4 |
| Ang2VEGF-0283 | 56,3 | 61,0 |
| Ang2VEGF-0284 | 56,3 | 61,1 |
| Ang2VEGF-0285 | 56,3 | 61,1 |
| Ang2VEGF-0286 | 56,3 | 61,6 |

### Example 3A

### Production and expression of multispecific antibodies which bind to IL-17 and TWEAK with VH/VL domain exchange/replacement (CrossMAb^{Vh-VL}) in one binding arm and with different charged amino acid substitutions in the CH1/CL interface

In a first example multispecific antibodies which binds to human IL-17 and human TWEAK were generated as described in the general methods section by classical molecular biology techniques and expressed transiently in HEK293 cells as described above. A general scheme of these respective multispecific, antibodies is given in Figures 1A to C. For comparison also the wild type (wt) VH/VL domain exchange/replacement antibodies with no substitution in the CH1/CL interface was prepared. The multispecific antibodies were expressed using expression plasmids containing the nucleic acids encoding the amino acid sequences depicted in Table 4a.

**Table 4a: Amino acid sequences of light chains (LC) and heavy chains (HC) of anti-TWEAK-IL17 multispecific antibodies TweakIL17-0096, TweakIL17-0097, TweakIL17-0098, TweakIL17-0099, TweakIL17-0100, TweakIL17-0101 with VH/VL domain exchange/replacement (CrossMAb^{Vh-VL}): wild type (wt) and different combinations of charged amino acids substitutions**

| **Antibody** | **LC IL17** | **HC IL17** | **HC TWEAK** | **LC TWEAK** |
|---|---|---|---|---|
| TweakIL17-0096 | SEQ ID NO: 15 | SEQ ID NO: 16 | SEQ ID NO: 17 | SEQ ID NO: 18 |
| TweakIL17-0097 | SEQ ID NO: 19 | SEQ ID NO: 20 | SEQ ID NO: 17 | SEQ ID NO: 21 |
| TweakIL17-0098 | SEQ ID NO: 19 | SEQ ID NO: 22 | SEQ ID NO: 17 | SEQ ID NO: 18 |
| TweakIL17-0099 | SEQ ID NO: 19 | SEQ ID NO: 22 | SEQ ID NO: 17 | SEQ ID NO: 21 |
| TweakIL17-0100 | SEQ ID NO: 23 | SEQ ID NO: 20 | SEQ ID NO: 17 | SEQ ID NO: 21 |
| TweakIL17-0101 | SEQ ID NO: 23 | SEQ ID NO: 20 | SEQ ID NO: 17 | SEQ ID NO: 18 |

For all constructs knobs into holes heterodimerization technology was used with a typical knob (T366W) substitution in the first CH3 domain and the corresponding hole susbstitutions (T366S, L368A and Y407V) in the second CH3 domain (as well as two additional introduced cysteine residues S354C/Y349'C) (contained in the respective corresponding heavy chain (HC) sequences depicted above).

### Example 3B

### Purification and characterization of multispecific antibodies which bind to IL-17 and TWEAK with VH/VL domain exchange/replacement (CrossMAb^{Vh-VL}) in one binding arm and with different charged amino acid substitutions in the CH1/CL interface

The multispecific antibodies expressed above were purified from the supernatant by a combination of Protein A affinity chromatography and size exclusion chromatography. All multispecific antibodies can be produced in good yields and are stable.

The obtained products were characterized for identity by mass spectrometry and analytical properties such as purity by SDS-PAGE, monomer content and stability

### Mass spectrometry

The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

Results are shown in Table 4b and Figure 6a.

**Table 4b: Reduction of main Bence-Jones-type side product by single charged amino acids substitutions according to the invention in the CH1/CL interface**

| | **CL IL17 (positio n 124)** | **CL IL17 (position 123)** | **CH1 IL17 (position 147)** | **CH1 IL17 (positi on 213)** | **CH1 TWEAK** | **CL TWEAK (position 124)** | **Main side product (Bence-Jones type mispairing) % by MS** |
|---|---|---|---|---|---|---|---|
| **TweakIL17-0096** | wt: Q124 | wt: E123 | wt: K147 | wt: K213 | wt | wt: Q124 | **∼20%** |
| **TweakIL17-0097** | **Q124K** | **E123R** | **K147E** | **K213E** | wt | **Q124E** | **0** |
| **TweakIL17-0098** | **Q124K** | **E123R** | **K147E** | **K213D** | wt | **wt** | **0** |
| **TweakIL17-0099** | **Q124K** | **E123R** | **K147E** | **K213D** | wt | **Q124E** | **0** |
| **TweakIL17-0100** | **Q124K** | **E123K** | **K147E** | **K213E** | wt | **Q124E** | **0** |
| **TweakIL17-0101** | **Q124K** | **E123K** | **K147E** | **K213E** | wt | **wt** | **not determ.** |

Results in Table 2b and Figure 6a show that with the double substitutions of charged amino acids with the opposite charge in the CH1 and CL domains according to the invention/as described for the invention (CL:Q124K/E123R and CH1:K147E/K213E; CL:Q124K/E123R and CH1:K147E/K213D; CL:Q124K/E123K and CH1:K147E/K213E) the main side product (Bence-Jones type mispairing) is completely removed when compared to the wild type multispecific antibody without such substitutions. This is independent of the further single substitution Q124E in the CL domain of the other binding arm, which does not influence the expression nor side product profile.

### Example 4A

### Production and expression of bivalent and trivalent multispecific antibodies which bind to Ang2 and VEGF, wherein the antibodies are devoid of an Fc fragments and include a VH/VL domain exchange/replacement in one binding arm and one or more charged amino acid substitutions in the CH1/CL interface

In a further example multispecific antibodies which bind to human Ang2 and human VEGF were generated as described in the general methods section by classical molecular biology techniques and expressed transiently in HEK293 cells as described above. The generated antibodies included in the binding arm specifically binding to VEGF a Fab fragment with a VH/VL domain exchange and in another binding arm specifically binding to Ang2 a Fab fragment without domain exchanges, while the multispecific antibody is devoid of an Fc fragment. Accordingly, the first light chain is derived from an antibody specifically binding to human Ang2 and comprises from N-terminal to C-terminal direction the domains VL-CL. The heavy chains of the first (anti-Ang2) and the second (anti-VEGF) antibody are connected via a glycin-serin peptide linker. In the heavy chain of the antibody specifically binding to VEGF the original variable domain VH is replaced by the variable domain VL derived from the anti-VEGF antibody. Thus, the polypeptide comprising the heavy chains of the anti-Ang2 and anti-VEGF antibodies comprises from N-terminal to C-terminal direction the domains VH(Ang2)-CH1(Ang2)-linker-VL(VEGF)-CH1(VEGF). In the light chain specifically binding to human VEGF, the original variable domain VL is replaced by the variable domain VH derived from the anti-VEGF antibody. Thus, the modified light chain of the anti-VEGF antibody comprises from N-terminal to C-terminal direction the domains VH-CL. Substitutions of the distinct amino acids in the CH1/CL interface are indicated in **Table 5b**.

In this example, multispecific antibodies of three general structures were generated:
i) bivalent multispecific Ang2-VEGF bispecific antibody of a CrossFabV_{H}-V_{L}-(Fab) format (general structure indicated Fig. 7C);
ii) trivalent multispecific Ang2-VEGF bispecific antibody of a (CrossFabV_{H}-V_{L})₂-Fab format (general structure indicated in Fig. 8C (neu));
iii) trivalent multispecific Ang2-VEGF bispecific antibody of a (Fab)₂-CrossFabV_{H}-V_{L} format;

For comparison also the wild type (wt) VH/VL domain exchange/replacement antibodies with no substitution in the CH1/CL interface are prepared. The multispecific antibodies are expressed using expression plasmids containing the nucleic acids encoding the amino acid sequences depicted in Table 5a.

**Table 5a: Amino acid sequences of light chains (LC) and heavy chains (HC) of anti-Ang2-VEGF multispecific antibodies with VH/VL domain exchange/replacement: wild type ("uncharged") and different combinations of charged amino acids substitutions ("charged")**

| **Antibody** | **LC Ang2** | **HC** | **LC VEGF** |
|---|---|---|---|
| xFab-Fab<ANG2-VEGF>-uncharged (Ang2VEGF-0452) | SEQ ID NO: 1 | SEQ ID NO: 40 | SEQ ID NO: 4 |
| xFab-Fab<ANG2-VEGF>-charged (Ang2VEGF-0447) | SEQ ID NO: 11 | SEQ ID NO: 41 | SEQ ID NO: 4 |
| xFab₂-Fab<ANG2-VEGF>-uncharged (Ang2VEGF-0453) | SEQ ID NO: 1 | SEQ ID NO: 42 | SEQ ID NO: 4 |
| xFab₂-Fab<ANG2-VEGF>-charged (Ang2VEGF-0448) | SEQ ID NO: 11 | SEQ ID NO: 43 | SEQ ID NO: 4 |
| Fab2-xFab<ANG2-VEGF>-uncharged | SEQ ID NO: 1 | SEQ ID NO: 38 | SEQ ID NO: 4 |
| Fab2-xFab<ANG2-VEGF>-charged | SEQ ID NO: 11 | SEQ ID NO: 39 | SEQ ID NO: 4 |

**Table 5b: Amino acid substitutions in the CH1/CL interface in antibodies according to the invention mentioned in Table 5a**

| | **Ang2** | | | | **VEGF** | |
|---|---|---|---|---|---|---|
| | **CL (position 124)** | **CL (position 123)** | **CH1 (position 147)** | **CH1 (position 213)** | **CH1** | **CL (position 124)** |
| xFab-Fab<ANG2-VEGF>-uncharged (Ang2VEGF-0452) | wt: Q124 | wt: E123 | wt: K147 | wt: K213 | wt | wt: Q124 |
| xFab-Fab<ANG2-VEGF>-charged (Ang2VEGF-0447) | **Q124R** | **E123K** | **K147E** | **K123E** | wt | wt |
| xFab₂-Fab<ANG2-VEGF>-uncharged (Ang2VEGF-0453) | wt: Q124 | wt: E123 | wt: K147 | wt: K213 | wt | wt: Q124 |
| xFab₂-Fab<ANG2-VEGF>-charged (Ang2VEGF-0448) | **Q124R** | **E123K** | **K147E** | **K123E** | wt | wt |
| Fab2-xFab<ANG2-VEGF>-uncharged | **wt: Q124** | **wt: E123** | **wt: K147** | **wt: K213** | wt | wt: Q124 |
| Fab2-xFab<ANG2-VEG F>-charged | **Q124R** | **E123K** | **K147E** | **K123E** | wt | wt |

### Example 4B:

### Production and expression of bivalent and trivalent multispecific antibodies which bind to ANG2 and VEGF, wherein the antibodies are devoid of an Fc fragments and include a VH/VL domain exchange/replacement in one binding arm and different charged amino acid substitutions in the CH1/CL interface

The secreted protein was purified by standard procedures using affinity purification.

Production yields after affinity purification and the fraction of the antibody molecule as determined by analytical size exclusion chromatography are indicated in **Table 5c**.

**Table 5c: Production yield and desired antibody fraction after affinity purification**

| **Antibody** | **Yield [mg/L]** | **Fraction [%] of antibody by analytical SEC** |
|---|---|---|
| Ang2VEGF-0452 | 37.8 | 64.1 |
| Ang2VEGF-0447 | 26.7 | 88.5 |
| Ang2VEGF-0453 | 4.2 | 88.5 |
| Ang2VEGF-0448 | 9.7 | 92.4 |

**Mass spectrometry:** The expected primary structures were analyzed by electrospray ionization mass spectrometry (ESI-MS) of the deglycosylated intact antibodies and deglycosylated/plasmin digested or alternatively deglycosylated/limited LysC digested antibodies .

The VH/VL Fab-CrossFab constructs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL Fab-CrossFabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

Due to a overlapping mass-range between the provided material and our MS-Methods the samples were aquired with two different methods to see potential side-products in a bigger mass range. While working in the larger mass range (1000-4000 m/z) the method includes CID voltage (in this case a cCID of 90), the measurement in the lower mass range (600-2000) uses no CID. With the application of CID there is a higher chance to aquire fragments which appear in order to in source fragmentation in the mass spectrometer.

Results are shown in **Table 5d**.

**Table 5d: Side products of indicated antibodies as analyzed by MS quantified relatively against the desired main molecule**

| **Antibody** | **Fraction of side product [%] by MS** | **Side product** |
|---|---|---|
| Ang2VEGF-0452 | 6% | mispaired side product with two Ang2 VL-CL light chains |
| Ang2VEGF-0447 | 0 | *not detected* |
| Ang2VEGF-0453 | 4.4 %; | mispaired side product with three Ang VL-CL light chains; |
| | 35.7% | mispaired side product with two Ang VL-CL light chains and one VEGF VH-CL chain |
| Ang2VEGF-0448 | 0 | *not detected* |

### Example 4C:

### Antigen binding properties of bivalent and trivalent multispecific antibodies which bind to ANG2 and VEGF, wherein the antibodies are devoid of an Fc fragments and include a VH/VL domain exchange/replacement in one binding arm and different charged amino acid substitutions in the CH1/CL interface

Binding of the multispecific antibodies of the previous examples 4A and 4B to their respective target antigens, i.e. ANG2 and VEGF, was assessed by Biacore®.

### VEGF binding was assessed according to the following procedure:

Binding of indicated antibodies to human VEGFA-121 was investigated by surface plasmon resonance using a BIACORE® T200 instrument (GE Healthcare). Aim for 50 RU of VEFGA-121-His were coupled on a Series S C1 chip (GE Healthcare BR-1005-35) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The association was measured by injection the indicated antibody in various concentrations in solution for 180 sec at a flow of 30 µl/min starting with 100 nM in 1:3 serial dilutions. The dissociation phase was monitored for up to 300 sec and triggered by switching from the sample solution to running buffer. The surface was regenerated by 30 sec washing with a 0.85% H₃PO₄ (phosphoric acid) solution at a flow rate of 30 µl/min. Bulk refractive index differences were corrected by subtracting the response obtained from a anti His antibody surface. Blank injections are also subtracted (= double referencing). For calculation of K_{D} and other kinetic parameters the Langmuir 1:1 model was used.

### Ang-2 binding was assessed according to the following procedure:

Binding of indicated antibodies to human Ang-2-RBD-Fc was investigated by surface plasmon resonance using a BIACORE® T200 instrument (GE Healthcare). Around 8000 (RU) of goat anti human F(ab')₂ (10 µg/ml anti human F(ab)'₂; Order Code: 28958325; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The bispecific antibody was captured by injecting a 5 nM solution for 25 sec at a flow of 5 µl/min. The association was measured by injection of human Ang2-RBD-Fc in various concentrations in solution for 120 sec at a flow of 30 µl/min starting with 100 nM in 1:3 serial dilutions. The dissociation phase was monitored for up to 180 sec and triggered by switching from the sample solution to running buffer. The surface was regenerated by 60 sec washing with a Glycine pH 2.1 solution at a flow rate of 30 µl/min. Bulk refractive index differences were corrected by subtracting the response obtained from a goat anti human F(ab')₂ surface. Blank injections are also subtracted (= double referencing). For calculation of apparent K_{D} and other kinetic parameters the Langmuir 1:1 model was used.

Results are indicated in **Tables 5e and 5f.**

**Table 5e: Affinity for VEGF of indicated antibodies**

| **Antibody** | **KD (nM)** |
|---|---|
| Ang2VEGF-0452 | 0.35 |
| Ang2VEGF-0447 | 0.36 |
| Ang2VEGF-0453 | 0.22 |
| Ang2VEGF-0448 | 0.18 |

**Table 5f: Affinity for Ang2 of indicated antibodies**

| **Antibody** | **KD (nM)** |
|---|---|
| Ang2VEGF-0452 | 3 |
| Ang2VEGF-0447 | 3 |
| Ang2VEGF-0453 | 5 |
| Ang2VEGF-0448 | 4 |

Antigen binding was not impaired by the mutations introduced into the CH1/CL interface of the Fc free antibodies.

### Example 5A:

### Production and expression of multispecific antibodies which bind to Angiopoietin-2 (ANG2) and VEGF with VH/VL domain exchange/replacement (CrossMAb^{Vh-VL}) in the VEGF-binding arm and with different charged amino acid substitutions in the CH1/CL interface of the VEGF-binding arm

In a further example multispecific antibodies which bind to human Angiopoietin-2 (ANG2) and human VEGF were generated as described in the general methods section by classical molecular biology techniques and is expressed transiently in HEK293 cells as described above. In these respective multispecific antibodies the substitution with different charged amino acids is present within the CH1/CL interface of the binding arm comprising the VH/VL domain exchange/replacement. For comparison also the wild type (wt) VH/VL domain exchange/replacement antibodies with no substitution in the CH1/CL interface was prepared. The multispecific antibodies were expressed using expression plasmids containing the nucleic acids encoding the amino acid sequences depicted in Table 6a.

**Table 6a: Amino acid sequences of light chains (LC) and heavy chains (HC) of anti-Ang2-VEGF multispecific antibodies Ang2VEGF-0273, Ang2VEGF-0425, and Ang2VEGF-0424 with VH/VL domain exchange/replacement (CrossMAb^{Vh-VL}): wild type (wt) and different combinations of charged amino acids substitutions**

| **Antibody** | **LC ANG-2** | **HC ANG-2** | **HC VEGF** | **LC VEGF** |
|---|---|---|---|---|
| Ang2VEGF-0273 | SEQ ID NO: 1 | SEQ ID NO: 2 | SEQ ID NO: 3 | SEQ ID NO: 4 |
| Ang2VEGF-0425 | SEQ ID NO: 1 | SEQ ID NO: 2 | SEQ ID NO: 44 | SEQ ID NO: 45 |
| Ang2VEGF-0424 | SEQ ID NO: 1 | SEQ ID NO: 2 | SEQ ID NO: 46 | SEQ ID NO: 47 |

For all constructs knobs into holes heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349C) (contained in the respective corresponding heavy chain (HC) sequences depicted above).

### Example 5B

### Purification and characterization of multispecific antibodies which bind to Angiopoietin-2 (ANG2) and VEGF with VH/VL domain exchange/replacement (CrossMAb^{Vh-VL}) in one binding arm and with different charged amino acid substitutions in the CH1/CL interface

The multispecific antibodies expressed above were purified from the supernatant by a combination of Protein A affinity chromatography and size exclusion chromatography. All multispecific antibodies can be produced in good yields and are stable.

The obtained products were characterized for identity by mass spectrometry and analytical properties such as purity by SDS-PAGE, monomer content and stability

### Mass spectrometry

The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

Results are shown in Table 6b.

**Table 6b: Side product profile (main Bence-Jones-type side product) by single charged amino acids substitutions in the CH1/CL interface within the binding arm comprising the VH/VL domain exchange/replacement**

| | **CL ANG-2** | **CH1 ANG-2** | **CH1 VEGF** | **CL VEGF** | **desired molecule** | **Main side product (Bence-Jones type mispairing) % by MS** |
|---|---|---|---|---|---|---|
| Ang2VEGF-0273 | wt (kappa) | wt | K147 K213 | wt E123 Q124 | ***n.d.*** | **∼20%** |
| Ang2VEGF-0425 | wt | wt | K147E | Q124K | **72%** | **22%** |
| Ang2VEGF-0424 | wt | wt | K147E K213E | E123K Q124K | **64%** | **26%** |

Results in Table 6b demonstrate that the side product profile (including the Bence-Jones type mispairing) could not be improved in the Ang2VEGF-bispecific antibodies with amino acid substitutions in the CH1/CL interface located within the binding arm comprising the VH/VL domain exchange/replacement.

## Claims

1. A multispecific antibody of human IgG1, human IgG2, human IgG3 or human IgG4 subclass, comprising:
a) a first light chain and a first heavy chain of a first antibody which specifically binds to a first antigen; and
b) a second light chain and a second heavy chain of a second antibody which specifically binds to a second antigen, and wherein the variable domains VL and VH in the second light chain and second heavy chain of the second antibody are replaced by each other; and
wherein
in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 147 or the amino acid at position 213 is substituted independently by glutamic acid (E) or aspartic acid (D) (numbering according to Kabat EU index).

2. The multispecific antibody according to claim 1,
wherein in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 147 is substituted independently by glutamic acid (E) or aspartic acid (D) (numbering according to Kabat EU index).

3. The multispecific antibody according to claim 1,
wherein in the constant domain CL of the first light chain under a) the amino acid at position 124 is substituted independently by lysine (K) or arginine (R) (numbering according to Kabat), and wherein in the constant domain CH1 of the first heavy chain under a) the amino acid at position 213 is substituted independently by glutamic acid (E) or aspartic acid (D) (numbering according to Kabat EU index).

4. The multispecific antibody according to one of claims 1 to 3,
wherein in the constant domain CL of the first light chain under a) the amino acid at position 124 is substituted independently by lysine (K) or arginine (R) (numbering according to Kabat) and the amino acid at position 123 is substituted independently by lysine (K) or arginine (R) (numbering according to Kabat), and wherein in the constant domain CH1 of the first heavy chain under a) the amino acid at position 147 is substituted independently by glutamic acid (E) or aspartic acid (D) (numbering according to Kabat EU index) and the amino acid at position 213 is substituted independently by glutamic acid (E) or aspartic acid (D) (numbering according to Kabat EU index).

5. The multispecific antibody according to one of claims 1 to 4,
wherein in the constant domain CL of the first light chain under a) the amino acid at position 124 is substituted by lysine (K) (numbering according to Kabat) and the amino acid at position 123 is substituted by lysine (K) (numbering according to Kabat),
and wherein in the constant domain CH1 of the first heavy chain under a) the amino acid at position 147 is substituted by glutamic acid (E) (numbering according to Kabat EU index) and the amino acid at position 213 is substituted by glutamic acid (E) (numbering according to Kabat EU index).

6. The antibody according to any one of the preceding claims, **characterized in that**
a first CH3 domain of the first heavy chain of the antibody under a) and a second CH3 domain of the second heavy chain of the antibody under b) each meet at an interface which comprises an original interface between the antibody CH3 domains,
wherein said interface is altered to promote the formation of the multispecific antibody, wherein the alteration is **characterized in that**:
i) the CH3 domain of one heavy chain is altered,
so that within the original interface of the CH3 domain of the one heavy chain that meets the original interface of the CH3 domain of the other heavy chain within the multispecific antibody,
an amino acid residue is replaced with an amino acid residue selected from the group consisting of arginine (R), phenylalanine (F), tyrosine (Y) and tryptophan (W), thereby generating a protuberance within the interface of the CH3 domain of the one heavy chain which is positionable in a cavity within the interface of the CH3 domain of the other heavy chain;
and
ii) the CH3 domain of the other heavy chain is altered,
so that within the original interface of the CH3 domain of the other heavy chain that meets the original interface of the CH3 domain of the one heavy chain within the multispecific antibody,
an amino acid residue is replaced with an amino acid residue selected from the group consisting of alanine (A), serine (S), threonine (T) and valine (V), thereby generating a cavity within the interface of the CH3 domain of the other heavy chain within which a protuberance within the interface of the CH3 domain of the one heavy chain is positionable.

7. The antibody according to claim 6, **characterized in that**
both CH3 domains are further altered by the introduction of cysteine (C) as amino acid in the corresponding positions of each CH3 domain such that a disulfide bridge between both CH3 domains can be formed.

8. A multispecific antibody according to any one of the preceding claims that specifically binds to human TWEAK and that specifically binds to human IL17, wherein
A) the multispecific antibody comprises
a variable heavy chain domain (VH) of SEQ ID NO:24, and a variable light chain domain (VL) of SEQ ID NO:25; and
B) the multispecific antibody comprises
a variable heavy chain domain (VH) of SEQ ID NO:26, and a variable light chain domain (VL) of SEQ ID NO:27.

9. A method for the preparation of the multispecific antibody according to any one of claims 1 to 8,
comprising the steps of
A) transforming a host cell with vectors comprising nucleic acid molecules encoding
a) the first light chain and the first heavy chain of the multispecific antibody according to any one of claims 1 to 9; and
b) the second light chain and the second heavy chain of the multispecific antibody according to any one of claims 1 to 9;
B) culturing the host cell under conditions that allow synthesis of said antibody molecule; and
C) recovering said antibody molecule from said culture.

10. A nucleic acid encoding the amino acid sequences of a multispecific antibody according to any one of claims 1 to 8.

11. An expression vector containing the nucleic acid according to claim 10 capable of expressing said nucleic acid in a host cell.

12. A pharmaceutical composition comprising an antibody according to any one of claims 1 to 8 and at least one pharmaceutically acceptable excipient.

## Patentansprüche

1. Multispezifischer Antikörper der humanen IgG1-, humanen IgG2-, humanen IgG3- oder humanen IgG4-Unterklasse, umfassend:
a) eine erste leichte Kette und eine erste schwere Kette eines ersten Antikörpers, der spezifisch an ein erstes Antigen bindet; und
b) eine zweite leichte Kette und eine zweite schwere Kette eines zweiten Antikörpers, der spezifisch an ein zweites Antigen bindet, und wobei die variablen Domänen VL und VH in der zweiten leichten Kette und der zweiten schweren Kette des zweiten Antikörpers untereinander ausgetauscht sind; und
wobei
in der konstanten Domäne CL der ersten leichten Kette unter a) die Aminosäure an Position 124 unabhängig voneinander durch Lysin (K) oder Arginin (R) substituiert ist (Nummerierung nach Kabat), und wobei in der konstanten Domäne CH1 der ersten schweren Kette unter a) die Aminosäure an Position 147 oder die Aminosäure an Position 213 unabhängig voneinander durch Glutaminsäure (E) oder Asparaginsäure (D) substituiert ist (Nummerierung nach Kabat-EU-Index).

2. Multispezifischer Antikörper nach Anspruch 1,
wobei in der konstanten Domäne CL der ersten leichten Kette unter a) die Aminosäure an Position 124 unabhängig voneinander durch Lysin (K) oder Arginin (R) substituiert ist (Nummerierung nach Kabat), und wobei in der konstanten Domäne CH1 der ersten schweren Kette unter a) die Aminosäure an Position 147 unabhängig voneinander durch Glutaminsäure (E) oder Asparaginsäure (D) substituiert ist (Nummerierung nach Kabat-EU-Index).

3. Multispezifischer Antikörper nach Anspruch 1,
wobei in der konstanten Domäne CL der ersten leichten Kette unter a) die Aminosäure an Position 124 unabhängig voneinander durch Lysin (K) oder Arginin (R) substituiert ist (Nummerierung nach Kabat), und wobei in der konstanten Domäne CH1 der ersten schweren Kette unter a) die Aminosäure an Position 213 unabhängig voneinander durch Glutaminsäure (E) oder Asparaginsäure (D) substituiert ist (Nummerierung nach Kabat-EU-Index).

4. Multispezifischer Antikörper nach einem der Ansprüche 1 bis 3,
wobei in der konstanten Domäne CL der ersten leichten Kette unter a) die Aminosäure an Position 124 unabhängig voneinander durch Lysin (K) oder Arginin (R) substituiert ist (Nummerierung nach Kabat) und die Aminosäure an Position 123 unabhängig voneinander durch Lysin (K) oder Arginin (R) substituiert ist (Nummerierung nach Kabat), und wobei in der konstanten Domäne CH1 der ersten schweren Kette unter a) die Aminosäure an Position 147 unabhängig voneinander durch Glutaminsäure (E) oder Asparaginsäure (D) substituiert ist (Nummerierung nach Kabat-EU-Index) und die Aminosäure an Position 213 unabhängig voneinander durch Glutaminsäure (E) oder Asparaginsäure (D) substituiert ist (Nummerierung nach Kabat-EU-Index).

5. Multispezifischer Antikörper nach einem der Ansprüche 1 bis 4,
wobei in der konstanten Domäne CL der ersten leichten Kette unter a) die Aminosäure an Position 124 durch Lysin (K) substituiert ist (Nummerierung nach Kabat) und die Aminosäure an Position 123 durch Lysin (K) substituiert ist (Nummerierung nach Kabat), und wobei in der konstanten Domäne CH1 der ersten schweren Kette unter a) die Aminosäure an Position 147 durch Glutaminsäure (E) substituiert ist (Nummerierung nach Kabat-EU-Index) und die Aminosäure an Position 213 durch Glutaminsäure (E) substituiert ist (Nummerierung nach Kabat-EU-Index).

6. Antikörper nach einem der vorhergehenden Ansprüche, **dadurch gekennzeichnet, dass** sich eine erste CH3-Domäne der ersten schweren Kette des Antikörpers unter a) und eine zweite CH3-Domäne der zweiten schweren Kette des Antikörpers unter b) jeweils an einer Grenzfläche treffen, die eine ursprüngliche Grenzfläche zwischen den CH3-Domänen des Antikörpers umfasst,
wobei die Grenzfläche so verändert ist, dass sie die Bildung des multispezifischen Antikörpers fördert, wobei die Veränderung **dadurch gekennzeichnet ist, dass**:
i) die CH3-Domäne einer schweren Kette verändert ist,
sodass innerhalb der ursprünglichen Grenzfläche der CH3-Domäne der einen schweren Kette, die die ursprüngliche Grenzfläche der CH3-Domäne der anderen schweren Kette in dem multispezifischen Antikörper trifft,
ein Aminosäurerest durch einen Aminosäurerest, ausgewählt aus der Gruppe, bestehend aus Arginin (R), Phenylalanin (F), Tyrosin (Y) und Tryptophan (W), ersetzt ist, wobei eine Protuberanz innerhalb der Grenzfläche der CH3-Domäne der einen schweren Kette erzeugt wird, die in einer Vertiefung innerhalb der Grenzfläche der CH3-Domäne der anderen schweren Kette positioniert werden kann;
und
ii) die CH3-Domäne der anderen schweren Kette verändert ist,
sodass innerhalb der ursprünglichen Grenzfläche der CH3-Domäne der anderen schweren Kette, die die ursprüngliche Grenzfläche der CH3-Domäne der einen schweren Kette innerhalb des multispezifischen Antikörpers trifft,
ein Aminosäurerest durch einen Aminosäurerest, ausgewählt aus der Gruppe, bestehend aus Alanin (A), Serin (S), Threonin (T) und Valin (V), ersetzt ist, wobei eine Vertiefung innerhalb der Grenzfläche der CH3-Domäne der anderen schweren Kette erzeugt wird, in der eine Protuberanz innerhalb der Grenzfläche der CH3-Domäne der einen schweren Kette positioniert werden kann.

7. Antikörper nach Anspruch 6, **dadurch gekennzeichnet, dass**
beide CH3-Domänen ferner durch die Einführung von Cystein (C) als Aminosäure an den entsprechenden Positionen jeder CH3-Domäne verändert sind, sodass eine Disulfidbrücke zwischen beiden CH3-Domänen gebildet werden kann.

8. Multispezifischer Antikörper nach einem der vorhergehenden Ansprüche, der spezifisch an humanes TWEAK bindet und der spezifisch an humanes IL17 bindet, wobei
A) der multispezifische Antikörper
eine variable Domäne der schweren Kette (VH) von SEQ ID NO:24 und eine variable Domäne der leichten Kette (VL) von SEQ ID NO:25 umfasst; und
B) der multispezifische Antikörper
eine variable Domäne der schweren Kette (VH) von SEQ ID NO:26 und eine variable Domäne der leichten Kette (VL) von SEQ ID NO:27 umfasst.

9. Verfahren zur Herstellung des multispezifischen Antikörpers nach einem der Ansprüche 1 bis 8, umfassend die folgenden Schritte
A) Transformieren einer Wirtszelle mit Vektoren, umfassend Nukleinsäuremoleküle, die Folgendes codieren
a) die erste leichte Kette und die erste schwere Kette des multispezifischen Antikörpers nach einem der Ansprüche 1 bis 9; und
b) die zweite leichte Kette und die zweite schwere Kette des multispezifischen Antikörpers nach einem der Ansprüche 1 bis 9;
B) Kultivieren der Wirtszelle unter Bedingungen, unter denen das Antikörpermolekül synthetisiert werden kann; und
C) Gewinnen des Antikörpermoleküls aus der Kultur.

10. Nukleinsäure, die die Aminosäuresequenzen eines multispezifischen Antikörpers nach einem der Ansprüche 1 bis 8 codiert.

11. Expressionsvektor, der die Nukleinsäure nach Anspruch 10 enthält und die Nukleinsäure in einer Wirtszelle exprimieren kann.

12. Pharmazeutische Zusammensetzung, umfassend einen Antikörper nach einem der Ansprüche 1 bis 8 und mindestens einen pharmazeutisch unbedenklichen Hilfsstoff.

## Revendications

1. Anticorps multispécifique de sous-classe IgG1 humaine, IgG2 humaine, IgG3 humaine ou IgG4 humaine, comprenant :
a) une première chaîne légère et une première chaîne lourde d'un premier anticorps qui se lie spécifiquement à un premier antigène ; et
b) une seconde chaîne légère et une seconde chaîne lourde d'un second anticorps qui se lie spécifiquement à un second antigène, et dans lequel les domaines variables VL et VH dans la seconde chaîne légère et la seconde chaîne lourde du second anticorps sont remplacés l'un par l'autre ; et
dans lequel
dans le domaine constant CL de la première chaîne légère sous a) l'acide aminé en position 124 est substitué indépendamment par de la lysine (K) ou de l'arginine (R) (numérotation selon Kabat), et dans lequel dans le domaine constant CH1 de la première chaîne lourde sous a) l'acide aminé en position 147 ou l'acide aminé en position 213 est substitué indépendamment par de l'acide glutamique (E) ou de l'acide aspartique (D) (numérotation selon l'indice EU de Kabat).

2. Anticorps multispécifique selon la revendication 1,
dans lequel dans le domaine constant CL de la première chaîne légère sous a) l'acide aminé en position 124 est substitué indépendamment par de la lysine (K) ou de l'arginine (R) (numérotation selon Kabat), et dans lequel dans le domaine constant CH1 de la première chaîne lourde sous a) l'acide aminé en position 147 est substitué indépendamment par de l'acide glutamique (E) ou de l'acide aspartique (D) (numérotation selon l'indice EU de Kabat).

3. Anticorps multispécifique selon la revendication 1,
dans lequel dans le domaine constant CL de la première chaîne légère sous a) l'acide aminé en position 124 est substitué indépendamment par de la lysine (K) ou de l'arginine (R) (numérotation selon Kabat), et dans lequel dans le domaine constant CH1 de la première chaîne lourde sous a) l'acide aminé en position 213 est substitué indépendamment par de l'acide glutamique (E) ou de l'acide aspartique (D) (numérotations selon l'indice EU de Kabat).

4. Anticorps multispécifique selon l'une des revendications 1 à 3,
dans lequel dans le domaine constant CL de la première chaîne légère sous a) l'acide aminé en position 124 est substitué indépendamment par de la lysine (K) ou de l'arginine (R) (numérotation selon Kabat) et l'acide aminé en position 123 est substitué indépendamment par de la lysine (K) ou de l'arginine (R) (numérotation selon Kabat), et dans lequel dans le domaine constant CH1 de la première chaîne lourde sous a) l'acide aminé en position 147 est substitué indépendamment par de l'acide glutamique (E) ou de l'acide aspartique (D) (numérotation selon l'indice EU de Kabat) et l'acide aminé en position 213 est substitué indépendamment par de l'acide glutamique (E) ou de l'acide aspartique (D) (numérotation selon l'indice EU de Kabat).

5. Anticorps multispécifique selon l'une des revendications 1 à 4,
dans lequel dans le domaine constant CL de la première chaîne légère sous a) l'acide aminé en position 124 est substitué par de la lysine (K) (numérotation selon Kabat) et l'acide aminé en position 123 est substitué par de la lysine (K) (numérotation selon Kabat),
et dans lequel dans le domaine constant CH1 de la première chaîne lourde sous a) l'acide aminé en position 147 est substitué par de l'acide glutamique (E) (numérotation selon l'indice EU de Kabat) et l'acide aminé en position 213 est substitué par de l'acide glutamique (E) (numérotation selon l'indice EU de Kabat).

6. Anticorps selon l'une quelconque des revendications précédentes, **caractérisé en ce que**
un premier domaine CH3 de la première chaîne lourde de l'anticorps sous a) et un second domaine CH3 de la seconde chaîne lourde de l'anticorps sous b) se rencontrent chacun à une interface qui comprend une interface originale entre les domaines CH3 d'anticorps,
dans lequel ladite interface est modifiée pour favoriser la formation de l'anticorps multispécifique, dans lequel la modification est **caractérisée en ce que** :
i) le domaine CH3 d'une chaîne lourde est modifié,
de sorte que dans l'interface originale du domaine CH3 de ladite chaîne lourde qui rencontre l'interface originale du domaine CH3 de l'autre chaîne lourde à l'intérieur de l'anticorps multispécifique,
un résidu d'acide aminé est remplacé par un résidu d'acide aminé sélectionné dans le groupe constitué par l'arginine (R), la phénylalanine (F), la tyrosine (Y) et le tryptophane (W), générant ainsi une protubérance à l'intérieur de l'interface du domaine CH3 de ladite chaîne lourde qui est positionnable dans une cavité à l'intérieur de l'interface du domaine CH3 de l'autre chaîne lourde ;
et
ii) le domaine CH3 de l'autre chaîne lourde est modifié,
de sorte qu'à l'intérieur de l'interface originale du domaine CH3 de l'autre chaîne lourde qui rencontre l'interface originale du domaine CH3 de ladite chaîne lourde à l'intérieur de l'anticorps multispécifique,
un résidu d'acide aminé est remplacé par un résidu d'acide aminé sélectionné dans le groupe constitué par l'alanine (A), la sérine (S), la thréonine (T) et la valine (V), générant ainsi une cavité à l'intérieur de l'interface du domaine CH3 de l'autre chaîne lourde à l'intérieur de laquelle une protubérance à l'intérieur de l'interface du domaine CH3 de ladite chaîne lourde est positionnable.

7. Anticorps selon la revendication 6, **caractérisé en ce que**
les deux domaines CH3 sont davantage modifiés par l'introduction de cystéine (C) en tant qu'acide aminé dans les positions correspondantes de chaque domaine CH3 de telle sorte qu'un pont disulfure peut être formé entre les deux domaines CH3.

8. Anticorps multispécifique selon l'une quelconque des revendications précédentes qui se lie spécifiquement au TWEAK humain et qui se lie spécifiquement à l'IL17 humaine, dans lequel
A) l'anticorps multispécifique comprend
un domaine de chaîne lourde variable (VH) de SEQ ID NO : 24, et un domaine de chaîne légère variable (VL) de SEQ ID NO : 25 ; et
B) l'anticorps multispécifique comprend
un domaine de chaîne lourde variable (VH) de SEQ ID NO : 26, et un domaine de chaîne légère variable (VL) de SEQ ID NO : 27.

9. Procédé de préparation de l'anticorps multispécifique selon l'une quelconque des revendications 1 à 8,
comprenant les étapes consistant à
A) transformer une cellule hôte avec des vecteurs comprenant des molécules d'acide nucléique codant pour
a) la première chaîne légère et la première chaîne lourde de l'anticorps multispécifique selon l'une quelconque des revendications 1 à 9 ; et
b) la seconde chaîne légère et la seconde chaîne lourde de l'anticorps multispécifique selon l'une quelconque des revendications 1 à 9 ;
B) cultiver la cellule hôte dans des conditions qui permettent la synthèse de ladite molécule d'anticorps ; et
C) récupérer ladite molécule d'anticorps à partir de ladite culture.

10. Acide nucléique codant pour les séquences d'acides aminés d'un anticorps multispécifique selon l'une quelconque des revendications 1 à 8.

11. Vecteur d'expression contenant l'acide nucléique selon la revendication 10 capable d'exprimer ledit acide nucléique dans une cellule hôte.

12. Composition pharmaceutique comprenant un anticorps selon l'un quelconque des revendications 1 à 8 et au moins un excipient pharmaceutiquement acceptable.


### SEQUENCE LISTING

<110> F. Hoffmann-La Roche AG
<120> Multispecific antibodies
<130> P32061-WO
<150> EP 14163165.5
   <151> 2014-04-02
<150> EP 14179034.5
   <151> 2014-07-30
<160> 47
<170> PatentIn version 3.5
<210> 1
   <211> 215
   <212> PRT
   <213> Artificial
<220>
   <223> light chain (LC) <Ang-2> wild type (wt)
<400> 1
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 2
   <211> 457
   <212> PRT
   <213> Artificial
<220>
   <223> heavy chain (HC) <Ang-2> wild type (wt)
<400> 2
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Lys Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Lys Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
   325 330 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro
   450 455
<210> 3
   <211> 437
   <212> PRT
   <213> Artificial
<220>
   <223> heavy chain (HC) <VEGF> with VH-VL exchange wild type (wt)
<400> 3
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Asn Ser Thr Tyr Arg Val Val Ser Val Leu Thr Val Leu His Gln Asp
   290 295 300
   Trp Leu Asn Gly Lys Glu Tyr Lys Cys Lys Val Ser Asn Lys Ala Leu
   305 310 315 320
   Pro Ala Pro Ile Glu Lys Thr Ile Ser Lys Ala Lys Gly Gln Pro Arg
   325 330 335
   Glu Pro Gln Val Cys Thr Leu Pro Pro Ser Arg Asp Glu Leu Thr Lys
   340 345 350
   Asn Gln Val Ser Leu Ser Cys Ala Val Lys Gly Phe Tyr Pro Ser Asp
   355 360 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Val Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro
   435
<210> 4
   <211> 230
   <212> PRT
   <213> Artificial
<220>
   <223> light chain (LC) <VEGF> with VH-VL exchange wild type (wt)
<400> 4
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
   20 25 30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Leu Val Thr Val Ser Ser Ala Ser Val Ala Ala
           115                 120                 125
   Pro Ser Val Phe Ile Phe Pro Pro Ser Asp Glu Gln Leu Lys Ser Gly
       130                 135                 140
   Thr Ala Ser Val Val Cys Leu Leu Asn Asn Phe Tyr Pro Arg Glu Ala
   145                 150                 155                 160
   Lys Val Gln Trp Lys Val Asp Asn Ala Leu Gln Ser Gly Asn Ser Gln
                   165                 170                 175
   Glu Ser Val Thr Glu Gln Asp Ser Lys Asp Ser Thr Tyr Ser Leu Ser
               180                 185                 190
   Ser Thr Leu Thr Leu Ser Lys Ala Asp Tyr Glu Lys His Lys Val Tyr
           195                 200                 205
   Ala Cys Glu Val Thr His Gln Gly Leu Ser Ser Pro Val Thr Lys Ser
       210                 215                 220
   Phe Asn Arg Gly Glu Cys
   225                 230
<210> 5
   <211> 215
   <212> PRT
   <213> Artificial
<220>
   <223> light chain (LC) <Ang-2> with Q124K substitution
<400> 5
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
               100                 105                 110
   Ala Pro Ser Val Phe Ile Phe Pro Pro Ser Asp Glu Lys Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
   210 215
<210> 6
   <211> 457
   <212> PRT
   <213> Artificial
<220>
   <223> heavy chain (HC) <Ang-2> with K147E substitution
<400> 6
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser Ala Ser Thr Lys Gly Pro Ser Val Phe Pro Leu Ala Pro Ser Ser
   130 135 140
   Lys Ser Thr Ser Gly Gly Thr Ala Ala Leu Gly Cys Leu Val Glu Asp
   145 150 155 160
   Tyr Phe Pro Glu Pro Val Thr Val Ser Trp Asn Ser Gly Ala Leu Thr
   165 170 175
   Ser Gly Val His Thr Phe Pro Ala Val Leu Gln Ser Ser Gly Leu Tyr
                   180                 185                 190
   Ser Leu Ser Ser Val Val Thr Val Pro Ser Ser Ser Leu Gly Thr Gln
               195                 200                 205
   Thr Tyr Ile Cys Asn Val Asn His Lys Pro Ser Asn Thr Lys Val Asp
           210                 215                 220
   Lys Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                       245                 250                 255
   Pro Lys Pro Lys Asp Thr Leu Met Ile Ser Arg Thr Pro Glu Val Thr
                   260                 265                 270
   Cys Val Val Val Asp Val Ser His Glu Asp Pro Glu Val Lys Phe Asn
               275                 280                 285
   Trp Tyr Val Asp Gly Val Glu Val His Asn Ala Lys Thr Lys Pro Arg
           290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                       325                 330                 335
   Asn Lys Ala Leu Pro Ala Pro Ile Glu Lys Thr Ile Ser Lys Ala Lys
                   340                 345                 350
   Gly Gln Pro Arg Glu Pro Gln Val Tyr Thr Leu Pro Pro Cys Arg Asp
               355                 360                 365
   Glu Leu Thr Lys Asn Gln Val Ser Leu Trp Cys Leu Val Lys Gly Phe
           370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                       405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 7
   <211> 457
   <212> PRT
   <213> Artificial
<220>
   <223> heavy chain (HC) <Ang-2> with K213E substitution
<400> 7
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Lys Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
       370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 8
   <211> 215
   <212> PRT
   <213> Artificial
<220>
   <223> light chain (LC) <Ang-2> with E123K substitution
<400> 8
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
   100 105 110
   Ala Pro Ser Val Phe Ile Phe Pro Pro Ser Asp Lys Gln Leu Lys Ser
   115 120 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 9
   <211> 215
   <212> PRT
   <213> Artificial
<220>
   <223> light chain (LC) <Ang-2> with Q124K substitution and E123K substitution
<400> 9
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Gln Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Arg Thr Val Ala
   100 105 110
   Ala Pro Ser Val Phe Ile Phe Pro Pro Ser Asp Lys Lys Leu Lys Ser
   115 120 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 10
   <211> 457
   <212> PRT
   <213> Artificial
<220>
   <223> heavy chain (HC) <Ang-2> with K147E substitution and K213E substitution
<400> 10
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
       370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 11
   <211> 215
   <212> PRT
   <213> Artificial
<220>
   <223> light chain (LC) <Ang-2> with Q124R substitution and E123K substitution
<400> 11
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
               100                 105                 110
   Ala Pro Ser Val Phe Ile Phe Pro Pro Ser Asp Lys Arg Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
   210 215
<210> 12
   <211> 230
   <212> PRT
   <213> Artificial
<220>
   <223> light chain (LC) <VEGF> with Q124E substitution
<400> 12
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Leu Val Thr Val Ser Ser Ala Ser Val Ala Ala
           115                 120                 125
   Pro Ser Val Phe Ile Phe Pro Pro Ser Asp Glu Glu Leu Lys Ser Gly
       130                 135                 140
   Thr Ala Ser Val Val Cys Leu Leu Asn Asn Phe Tyr Pro Arg Glu Ala
   145                 150                 155                 160
   Lys Val Gln Trp Lys Val Asp Asn Ala Leu Gln Ser Gly Asn Ser Gln
                   165                 170                 175
   Glu Ser Val Thr Glu Gln Asp Ser Lys Asp Ser Thr Tyr Ser Leu Ser
   180 185 190
   Ser Thr Leu Thr Leu Ser Lys Ala Asp Tyr Glu Lys His Lys Val Tyr
   195 200 205
   Ala Cys Glu Val Thr His Gln Gly Leu Ser Ser Pro Val Thr Lys Ser
   210 215 220
   Phe Asn Arg Gly Glu Cys
   225 230
<210> 13
   <211> 214
   <212> PRT
   <213> Artificial
<220>
   <223> light chain (LC) <Ang-2> with E124K substitution and E123K substitution
<400> 13
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Gln Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Gly Gln Pro Lys
   100 105 110
   Ala Ala Pro Ser Val Thr Leu Phe Pro Pro Ser Ser Lys Lys Leu Gln
   115 120 125
   Ala Asn Lys Ala Thr Leu Val Cys Leu Ile Ser Asp Phe Tyr Pro Gly
   130 135 140
   Ala Val Thr Val Ala Trp Lys Ala Asp Ser Ser Pro Val Lys Ala Gly
   145 150 155 160
   Val Glu Thr Thr Thr Pro Ser Lys Gln Ser Asn Asn Lys Tyr Ala Ala
   165 170 175
   Ser Ser Tyr Leu Ser Leu Thr Pro Glu Gln Trp Lys Ser His Arg Ser
   180 185 190
   Tyr Ser Cys Gln Val Thr His Glu Gly Ser Thr Val Glu Lys Thr Val
   195 200 205
   Ala Pro Thr Glu Cys Ser
   210
<210> 14
   <211> 457
   <212> PRT
   <213> Artificial
<220>
   <223> heavy chain (HC) <Ang-2> with K147E substitution and K213D substitution
<400> 14
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser Ala Ser Thr Lys Gly Pro Ser Val Phe Pro Leu Ala Pro Ser Ser
   130 135 140
   Lys Ser Thr Ser Gly Gly Thr Ala Ala Leu Gly Cys Leu Val Glu Asp
   145 150 155 160
   Tyr Phe Pro Glu Pro Val Thr Val Ser Trp Asn Ser Gly Ala Leu Thr
   165 170 175
   Ser Gly Val His Thr Phe Pro Ala Val Leu Gln Ser Ser Gly Leu Tyr
   180 185 190
   Ser Leu Ser Ser Val Val Thr Val Pro Ser Ser Ser Leu Gly Thr Gln
   195 200 205
   Thr Tyr Ile Cys Asn Val Asn His Lys Pro Ser Asn Thr Lys Val Asp
   210 215 220
   Asp Lys Val Glu Pro Lys Ser Cys Asp Lys Thr His Thr Cys Pro Pro
   225 230 235 240
   Cys Pro Ala Pro Glu Leu Leu Gly Gly Pro Ser Val Phe Leu Phe Pro
   245 250 255
   Pro Lys Pro Lys Asp Thr Leu Met Ile Ser Arg Thr Pro Glu Val Thr
   260 265 270
   Cys Val Val Val Asp Val Ser His Glu Asp Pro Glu Val Lys Phe Asn
   275 280 285
   Trp Tyr Val Asp Gly Val Glu Val His Asn Ala Lys Thr Lys Pro Arg
   290 295 300
   Glu Glu Gln Tyr Asn Ser Thr Tyr Arg Val Val Ser Val Leu Thr Val
   305 310 315 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
       370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 15
   <211> 219
   <212> PRT
   <213> Artificial
<220>
   <223> light chain (LC) <IL-17> wild type (wt)
<400> 15
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Gly Asp Thr Tyr Phe His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Thr
                   85                  90                  95
   Thr His Ala Pro Phe Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 16
   <211> 446
   <212> PRT
   <213> Artificial
<220>
   <223> heavy chain (HC) <IL-17> wild type (wt)
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Lys Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
   260 265 270
   Glu Val Gln Phe Asn Trp Tyr Val Asp Gly Val Glu Val His Asn Ala
   275 280 285
   Lys Thr Lys Pro Arg Glu Glu Gln Phe Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Gly Leu Pro Ser Ser Ile Glu Lys Thr
   325 330 335
   Ile Ser Lys Ala Lys Gly Gln Pro Arg Glu Pro Gln Val Cys Thr Leu
   340 345 350
   Pro Pro Ser Gln Glu Glu Met Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu
   435 440 445
<210> 17
   <211> 438
   <212> PRT
   <213> Artificial
<220>
   <223> heavy chain (HC) <TWEAK> with VH-VL exchange wild type (wt)
<400> 17
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Thr Ala Tyr Tyr Asn Ser Arg
                   85                  90                  95
   Pro Asp Thr Val Ala Phe Gly Gly Gly Thr Lys Val Glu Ile Lys Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Lys
               180                 185                 190
   Thr Tyr Thr Cys Asn Val Asp His Lys Pro Ser Asn Thr Lys Val Asp
           195                 200                 205
   Lys Arg Val Glu Ser Lys Tyr Gly Pro Pro Cys Pro Pro Cys Pro Ala
       210                 215                 220
   Pro Glu Phe Glu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser Gln Glu Asp Pro Glu Val Gln Phe Asn Trp Tyr Val
               260                 265                 270
   Asp Gly Val Glu Val His Asn Ala Lys Thr Lys Pro Arg Glu Glu Gln
           275                 280                 285
   Phe Asn Ser Thr Tyr Arg Val Val Ser Val Leu Thr Val Leu His Gln
       290                 295                 300
   Asp Trp Leu Asn Gly Lys Glu Tyr Lys Cys Lys Val Ser Asn Lys Gly
   305                 310                 315                 320
   Leu Pro Ser Ser Ile Glu Lys Thr Ile Ser Lys Ala Lys Gly Gln Pro
                   325                 330                 335
   Arg Glu Pro Gln Val Tyr Thr Leu Pro Pro Cys Gln Glu Glu Met Thr
               340                 345                 350
   Lys Asn Gln Val Ser Leu Trp Cys Leu Val Lys Gly Phe Tyr Pro Ser
           355                 360                 365
   Asp Ile Ala Val Glu Trp Glu Ser Asn Gly Gln Pro Glu Asn Asn Tyr
       370                 375                 380
   Lys Thr Thr Pro Pro Val Leu Asp Ser Asp Gly Ser Phe Phe Leu Tyr
   385                 390                 395                 400
   Ser Arg Leu Thr Val Asp Lys Ser Arg Trp Gln Glu Gly Asn Val Phe
                   405                 410                 415
   Ser Cys Ser Val Met His Glu Ala Leu His Asn His Tyr Thr Gln Lys
               420                 425                 430
   Ser Leu Ser Leu Ser Leu
           435
<210> 18
   <211> 227
   <212> PRT
   <213> Artificial
<220>
   <223> light chain (LC) <TWEAK> with VH-VL exchange wild type (wt)
<400> 18
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Asp Phe Ser Thr Tyr
               20                  25                  30
   Tyr Met Ser Trp Val Arg Gln Ala Pro Gly Gln Gly Leu Glu Trp Met
           35                  40                  45
   Gly Thr Val Tyr Val Arg Gln Gly Thr Thr Tyr Tyr Ala Ser Trp Leu
       50                  55                  60
   Asn Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Tyr Asn Tyr Asp Asp Ala Phe Val Ile Trp Gly Gln
               100                 105                 110
   Gly Thr Leu Val Thr Val Ser Ser Ala Ser Val Ala Ala Pro Ser Val
           115                 120                 125
   Phe Ile Phe Pro Pro Ser Asp Glu Gln Leu Lys Ser Gly Thr Ala Ser
       130                 135                 140
   Val Val Cys Leu Leu Asn Asn Phe Tyr Pro Arg Glu Ala Lys Val Gln
   145                 150                 155                 160
   Trp Lys Val Asp Asn Ala Leu Gln Ser Gly Asn Ser Gln Glu Ser Val
                   165                 170                 175
   Thr Glu Gln Asp Ser Lys Asp Ser Thr Tyr Ser Leu Ser Ser Thr Leu
               180                 185                 190
   Thr Leu Ser Lys Ala Asp Tyr Glu Lys His Lys Val Tyr Ala Cys Glu
           195                 200                 205
   Val Thr His Gln Gly Leu Ser Ser Pro Val Thr Lys Ser Phe Asn Arg
   210 215 220
   Gly Glu Cys
   225
<210> 19
   <211> 219
   <212> PRT
   <213> Artificial
<220>
   <223> light chain (LC) <IL-17> with Q124K substitution and E123R substitution
<400> 19
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val His Ser
   20 25 30
   Asn Gly Asp Thr Tyr Phe His Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Thr
   85 90 95
   Thr His Ala Pro Phe Thr Phe Gly Gln Gly Thr Lys Leu Glu Ile Lys
   100 105 110
   Arg Thr Val Ala Ala Pro Ser Val Phe Ile Phe Pro Pro Ser Asp Arg
   115 120 125
   Lys Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 20
   <211> 446
   <212> PRT
   <213> Artificial
<220>
   <223> heavy chain (HC) <IL-17> with K147E substitution and K213E substitution
<400> 20
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Asp Ser Tyr
   20 25 30
   Gly Val His Trp Val Arg Gln Ala Thr Gly Lys Gly Leu Glu Trp Val
   35 40 45
   Ser Val Ile Trp Ser Asp Gly Thr Thr Thr Tyr Asn Ser Ala Leu Lys
   50 55 60
   Ser Arg Phe Thr Ile Ser Arg Glu Asn Ala Lys Asn Ser Leu Tyr Leu
   65 70 75 80
   Gln Met Asn Ser Leu Arg Ala Gly Asp Thr Ala Val Tyr Tyr Cys Ala
   85 90 95
   Arg Asp Thr His Tyr Arg Leu Tyr Tyr Tyr Ala Met Asp Tyr Trp Gly
   100 105 110
   Gln Gly Thr Thr Val Thr Val Ser Ser Ala Ser Thr Lys Gly Pro Ser
               115                 120                 125
   Val Phe Pro Leu Ala Pro Cys Ser Arg Ser Thr Ser Glu Ser Thr Ala
       130                 135                 140
   Ala Leu Gly Cys Leu Val Glu Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Glu Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Gln Glu Glu Met Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu
   435 440 445
<210> 21
   <211> 227
   <212> PRT
   <213> Artificial
<220>
   <223> light chain (LC) <TWEAK> with Q124E substitution
<400> 21
   Gln Val Gln Leu Val Gln Ser Gly Ala Glu Val Lys Lys Pro Gly Ser
   1 5 10 15
   Ser Val Lys Val Ser Cys Lys Ala Ser Gly Phe Asp Phe Ser Thr Tyr
   20 25 30
   Tyr Met Ser Trp Val Arg Gln Ala Pro Gly Gln Gly Leu Glu Trp Met
   35 40 45
   Gly Thr Val Tyr Val Arg Gln Gly Thr Thr Tyr Tyr Ala Ser Trp Leu
   50 55 60
   Asn Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Gly Gly Tyr Asn Tyr Asp Asp Ala Phe Val Ile Trp Gly Gln
   100 105 110
   Gly Thr Leu Val Thr Val Ser Ser Ala Ser Val Ala Ala Pro Ser Val
   115 120 125
   Phe Ile Phe Pro Pro Ser Asp Glu Glu Leu Lys Ser Gly Thr Ala Ser
   130 135 140
   Val Val Cys Leu Leu Asn Asn Phe Tyr Pro Arg Glu Ala Lys Val Gln
   145 150 155 160
   Trp Lys Val Asp Asn Ala Leu Gln Ser Gly Asn Ser Gln Glu Ser Val
   165 170 175
   Thr Glu Gln Asp Ser Lys Asp Ser Thr Tyr Ser Leu Ser Ser Thr Leu
   180 185 190
   Thr Leu Ser Lys Ala Asp Tyr Glu Lys His Lys Val Tyr Ala Cys Glu
   195 200 205
   Val Thr His Gln Gly Leu Ser Ser Pro Val Thr Lys Ser Phe Asn Arg
   210 215 220
   Gly Glu Cys
   225
<210> 22
   <211> 446
   <212> PRT
   <213> Artificial
<220>
   <223> heavy chain (HC) <IL-17> with K147E substitution and K213D substitution
<400> 22
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Asp Ser Tyr
   20 25 30
   Gly Val His Trp Val Arg Gln Ala Thr Gly Lys Gly Leu Glu Trp Val
   35 40 45
   Ser Val Ile Trp Ser Asp Gly Thr Thr Thr Tyr Asn Ser Ala Leu Lys
   50 55 60
   Ser Arg Phe Thr Ile Ser Arg Glu Asn Ala Lys Asn Ser Leu Tyr Leu
   65 70 75 80
   Gln Met Asn Ser Leu Arg Ala Gly Asp Thr Ala Val Tyr Tyr Cys Ala
   85 90 95
   Arg Asp Thr His Tyr Arg Leu Tyr Tyr Tyr Ala Met Asp Tyr Trp Gly
   100 105 110
   Gln Gly Thr Thr Val Thr Val Ser Ser Ala Ser Thr Lys Gly Pro Ser
   115 120 125
   Val Phe Pro Leu Ala Pro Cys Ser Arg Ser Thr Ser Glu Ser Thr Ala
   130 135 140
   Ala Leu Gly Cys Leu Val Glu Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Lys Thr Tyr Thr Cys Asn Val Asp His
   195 200 205
   Lys Pro Ser Asn Thr Lys Val Asp Asp Arg Val Glu Ser Lys Tyr Gly
   210 215 220
   Pro Pro Cys Pro Pro Cys Pro Ala Pro Glu Phe Glu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser Gln Glu Asp Pro
   260 265 270
   Glu Val Gln Phe Asn Trp Tyr Val Asp Gly Val Glu Val His Asn Ala
   275 280 285
   Lys Thr Lys Pro Arg Glu Glu Gln Phe Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Gly Leu Pro Ser Ser Ile Glu Lys Thr
   325 330 335
   Ile Ser Lys Ala Lys Gly Gln Pro Arg Glu Pro Gln Val Cys Thr Leu
   340 345 350
   Pro Pro Ser Gln Glu Glu Met Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu
   435 440 445
<210> 23
   <211> 219
   <212> PRT
   <213> Artificial
<220>
   <223> light chain (LC) <IL-17> with Q124K substitution and E123K substitution
<400> 23
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val His Ser
   20 25 30
   Asn Gly Asp Thr Tyr Phe His Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Thr
   85 90 95
   Thr His Ala Pro Phe Thr Phe Gly Gln Gly Thr Lys Leu Glu Ile Lys
   100 105 110
   Arg Thr Val Ala Ala Pro Ser Val Phe Ile Phe Pro Pro Ser Asp Lys
   115 120 125
   Lys Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 24
   <211> 120
   <212> PRT
   <213> Artificial
<220>
   <223> variable heavy chain domain VH <TWEAK > 305-HC4
<400> 24
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Asp Phe Ser Thr Tyr
               20                  25                  30
   Tyr Met Ser Trp Val Arg Gln Ala Pro Gly Gln Gly Leu Glu Trp Met
           35                  40                  45
   Gly Thr Val Tyr Val Arg Gln Gly Thr Thr Tyr Tyr Ala Ser Trp Leu
       50                  55                  60
   Asn Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Tyr Asn Tyr Asp Asp Ala Phe Val Ile Trp Gly Gln
               100                 105                 110
   Gly Thr Leu Val Thr Val Ser Ser
           115                 120
<210> 25
   <211> 111
   <212> PRT
   <213> Artificial
<220>
   <223> variable light chain domain VL <TWEAK>305-LC2
<400> 25
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Thr Ala Tyr Tyr Asn Ser Arg
   85 90 95
   Pro Asp Thr Val Ala Phe Gly Gly Gly Thr Lys Val Glu Ile Lys
   100 105 110
<210> 26
   <211> 121
   <212> PRT
   <213> Artificial
<220>
   <223> variable heavy chain domain VH <IL-17> HC136
<400> 26
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Asp Ser Tyr
   20 25 30
   Gly Val His Trp Val Arg Gln Ala Thr Gly Lys Gly Leu Glu Trp Val
   35 40 45
   Ser Val Ile Trp Ser Asp Gly Thr Thr Thr Tyr Asn Ser Ala Leu Lys
   50 55 60
   Ser Arg Phe Thr Ile Ser Arg Glu Asn Ala Lys Asn Ser Leu Tyr Leu
   65 70 75 80
   Gln Met Asn Ser Leu Arg Ala Gly Asp Thr Ala Val Tyr Tyr Cys Ala
   85 90 95
   Arg Asp Thr His Tyr Arg Leu Tyr Tyr Tyr Ala Met Asp Tyr Trp Gly
   100 105 110
   Gln Gly Thr Thr Val Thr Val Ser Ser
   115 120
<210> 27
   <211> 112
   <212> PRT
   <213> Artificial
<220>
   <223> variable light chain domain VL <IL-17> LC136
<400> 27
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Gly Asp Thr Tyr Phe His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Thr
                   85                  90                  95
   Thr His Ala Pro Phe Thr Phe Gly Gln Gly Thr Lys Leu Glu Ile Lys
               100                 105                 110
<210> 28
   <211> 440
   <212> PRT
   <213> Artificial Sequence
<220>
   <223> heavy chain (HC) <TWEAK> with VH-VL exchange wild type (wt) (comprising terminal GK dipeptide)
<400> 28
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Thr Ala Tyr Tyr Asn Ser Arg
                   85                  90                  95
   Pro Asp Thr Val Ala Phe Gly Gly Gly Thr Lys Val Glu Ile Lys Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Lys
               180                 185                 190
   Thr Tyr Thr Cys Asn Val Asp His Lys Pro Ser Asn Thr Lys Val Asp
           195                 200                 205
   Lys Arg Val Glu Ser Lys Tyr Gly Pro Pro Cys Pro Pro Cys Pro Ala
       210                 215                 220
   Pro Glu Phe Glu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser Gln Glu Asp Pro Glu Val Gln Phe Asn Trp Tyr Val
               260                 265                 270
   Asp Gly Val Glu Val His Asn Ala Lys Thr Lys Pro Arg Glu Glu Gln
   275 280 285
   Phe Asn Ser Thr Tyr Arg Val Val Ser Val Leu Thr Val Leu His Gln
   290 295 300
   Asp Trp Leu Asn Gly Lys Glu Tyr Lys Cys Lys Val Ser Asn Lys Gly
   305 310 315 320
   Leu Pro Ser Ser Ile Glu Lys Thr Ile Ser Lys Ala Lys Gly Gln Pro
   325 330 335
   Arg Glu Pro Gln Val Tyr Thr Leu Pro Pro Cys Gln Glu Glu Met Thr
   340 345 350
   Lys Asn Gln Val Ser Leu Trp Cys Leu Val Lys Gly Phe Tyr Pro Ser
   355 360 365
   Asp Ile Ala Val Glu Trp Glu Ser Asn Gly Gln Pro Glu Asn Asn Tyr
   370 375 380
   Lys Thr Thr Pro Pro Val Leu Asp Ser Asp Gly Ser Phe Phe Leu Tyr
   385 390 395 400
   Ser Arg Leu Thr Val Asp Lys Ser Arg Trp Gln Glu Gly Asn Val Phe
   405 410 415
   Ser Cys Ser Val Met His Glu Ala Leu His Asn His Tyr Thr Gln Lys
   420 425 430
   Ser Leu Ser Leu Ser Leu Gly Lys
   435 440
<210> 29
   <211> 448
   <212> PRT
   <213> Artificial Sequence
<220>
   <223> heavy chain (HC) <IL-17> with K147E substitution and K213E substitution (comprising terminal GK dipeptide)
<400> 29
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Glu Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Glu Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Gln Glu Glu Met Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Arg Leu Thr Val Asp Lys Ser
                   405                 410                 415
   Arg Trp Gln Glu Gly Asn Val Phe Ser Cys Ser Val Met His Glu Ala
               420                 425                 430
   Leu His Asn His Tyr Thr Gln Lys Ser Leu Ser Leu Ser Leu Gly Lys
           435                 440                 445
<210> 30
   <211> 448
   <212> PRT
   <213> Artificial Sequence
<220>
   <223> heavy chain (HC) <IL-17> with K147E substitution and K213D substitution (comprising terminal GK dipeptide)
<400> 30
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Glu Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Asp Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Gln Glu Glu Met Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Arg Leu Thr Val Asp Lys Ser
                   405                 410                 415
   Arg Trp Gln Glu Gly Asn Val Phe Ser Cys Ser Val Met His Glu Ala
               420                 425                 430
   Leu His Asn His Tyr Thr Gln Lys Ser Leu Ser Leu Ser Leu Gly Lys
           435                 440                 445
<210> 31
   <211> 459
   <212> PRT
   <213> Artificial Sequence
<220>
   <223> heavy chain (HC) <Ang-2> wild type (wt) (comprising terminal GK dipeptide)
<400> 31
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Lys Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
   180 185 190
   Ser Leu Ser Ser Val Val Thr Val Pro Ser Ser Ser Leu Gly Thr Gln
   195 200 205
   Thr Tyr Ile Cys Asn Val Asn His Lys Pro Ser Asn Thr Lys Val Asp
   210 215 220
   Lys Lys Val Glu Pro Lys Ser Cys Asp Lys Thr His Thr Cys Pro Pro
   225 230 235 240
   Cys Pro Ala Pro Glu Leu Leu Gly Gly Pro Ser Val Phe Leu Phe Pro
   245 250 255
   Pro Lys Pro Lys Asp Thr Leu Met Ile Ser Arg Thr Pro Glu Val Thr
   260 265 270
   Cys Val Val Val Asp Val Ser His Glu Asp Pro Glu Val Lys Phe Asn
   275 280 285
   Trp Tyr Val Asp Gly Val Glu Val His Asn Ala Lys Thr Lys Pro Arg
   290 295 300
   Glu Glu Gln Tyr Asn Ser Thr Tyr Arg Val Val Ser Val Leu Thr Val
   305 310 315 320
   Leu His Gln Asp Trp Leu Asn Gly Lys Glu Tyr Lys Cys Lys Val Ser
   325 330 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro Gly Lys
   450 455
<210> 32
   <211> 439
   <212> PRT
   <213> Artificial Sequence
<220>
   <223> heavy chain (HC) <VEGF> with VH-VL exchange wild type (wt) (comprising terminal GK dipeptide)
<400> 32
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Thr Val Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Asn Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Cys Thr Leu Pro Pro Ser Arg Asp Glu Leu Thr Lys
               340                 345                 350
   Asn Gln Val Ser Leu Ser Cys Ala Val Lys Gly Phe Tyr Pro Ser Asp
           355                 360                 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Val Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro Gly Lys
   435
<210> 33
   <211> 459
   <212> PRT
   <213> Artificial Sequence
<220>
   <223> heavy chain (HC) <Ang-2> with K147E substitution (comprising terminal GK dipeptide)
<400> 33
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Lys Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro Gly Lys
   450 455
<210> 34
   <211> 459
   <212> PRT
   <213> Artificial Sequence
<220>
   <223> heavy chain (HC) <Ang-2> with K213E substitution (comprising terminal GK dipeptide)
<400> 34
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Lys Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305 310 315 320
   Leu His Gln Asp Trp Leu Asn Gly Lys Glu Tyr Lys Cys Lys Val Ser
   325 330 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro Gly Lys
   450 455
<210> 35
   <211> 459
   <212> PRT
   <213> Artificial Sequence
<220>
   <223> heavy chain (HC) <Ang-2> with K147E substitution and K213E substitution (comprising terminal GK dipeptide)
<400> 35
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
       370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro Gly Lys
       450                 455
<210> 36
   <211> 459
   <212> PRT
   <213> Artificial Sequence
<220>
   <223> heavy chain (HC) <Ang-2> with K147E substitution and K213D substitution (comprising terminal GK dipeptide)
<400> 36
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Asp Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
       370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro Gly Lys
   450 455
<210> 37
   <211> 448
   <212> PRT
   <213> Artificial Sequence
<220>
   <223> heavy chain (HC) <IL-17> wild type (wt) (comprising terminal GK dipeptide)
<400> 37
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Asp Ser Tyr
   20 25 30
   Gly Val His Trp Val Arg Gln Ala Thr Gly Lys Gly Leu Glu Trp Val
   35 40 45
   Ser Val Ile Trp Ser Asp Gly Thr Thr Thr Tyr Asn Ser Ala Leu Lys
   50 55 60
   Ser Arg Phe Thr Ile Ser Arg Glu Asn Ala Lys Asn Ser Leu Tyr Leu
   65 70 75 80
   Gln Met Asn Ser Leu Arg Ala Gly Asp Thr Ala Val Tyr Tyr Cys Ala
   85 90 95
   Arg Asp Thr His Tyr Arg Leu Tyr Tyr Tyr Ala Met Asp Tyr Trp Gly
   100 105 110
   Gln Gly Thr Thr Val Thr Val Ser Ser Ala Ser Thr Lys Gly Pro Ser
   115 120 125
   Val Phe Pro Leu Ala Pro Cys Ser Arg Ser Thr Ser Glu Ser Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Lys Thr Tyr Thr Cys Asn Val Asp His
   195 200 205
   Lys Pro Ser Asn Thr Lys Val Asp Lys Arg Val Glu Ser Lys Tyr Gly
   210 215 220
   Pro Pro Cys Pro Pro Cys Pro Ala Pro Glu Phe Glu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser Gln Glu Asp Pro
   260 265 270
   Glu Val Gln Phe Asn Trp Tyr Val Asp Gly Val Glu Val His Asn Ala
   275 280 285
   Lys Thr Lys Pro Arg Glu Glu Gln Phe Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Gly Leu Pro Ser Ser Ile Glu Lys Thr
   325 330 335
   Ile Ser Lys Ala Lys Gly Gln Pro Arg Glu Pro Gln Val Cys Thr Leu
   340 345 350
   Pro Pro Ser Gln Glu Glu Met Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu Gly Lys
   435 440 445
<210> 38
   <211> 688
   <212> PRT
   <213> Artificial Sequence
<220>
   <223> Fab2-CrossFab heavy chain (HC) including two heavy chains (HC) <Ang-2> wild type (wt) coupled to one heavy chain (HC) <VEGF> with VL-VH domain exchange wild type (wt) via glycine-serine-linkers
<400> 38
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser Ala Ser Thr Lys Gly Pro Ser Val Phe Pro Leu Ala Pro Ser Ser
   130 135 140
   Lys Ser Thr Ser Gly Gly Thr Ala Ala Leu Gly Cys Leu Val Lys Asp
   145 150 155 160
   Tyr Phe Pro Glu Pro Val Thr Val Ser Trp Asn Ser Gly Ala Leu Thr
   165 170 175
   Ser Gly Val His Thr Phe Pro Ala Val Leu Gln Ser Ser Gly Leu Tyr
   180 185 190
   Ser Leu Ser Ser Val Val Thr Val Pro Ser Ser Ser Leu Gly Thr Gln
   195 200 205
   Thr Tyr Ile Cys Asn Val Asn His Lys Pro Ser Asn Thr Lys Val Asp
   210 215 220
   Lys Lys Val Glu Pro Lys Ser Cys Ser Gly Gly Gly Gly Ser Gln Val
   225 230 235 240
   Gln Leu Val Glu Ser Gly Ala Glu Val Lys Lys Pro Gly Ala Ser Val
   245 250 255
   Lys Val Ser Cys Lys Ala Ser Gly Tyr Thr Phe Thr Gly Tyr Tyr Met
   260 265 270
   His Trp Val Arg Gln Ala Pro Gly Gln Gly Leu Glu Trp Met Gly Trp
   275 280 285
   Ile Asn Pro Asn Ser Gly Gly Thr Asn Tyr Ala Gln Lys Phe Gln Gly
   290 295 300
   Arg Val Thr Met Thr Arg Asp Thr Ser Ile Ser Thr Ala Tyr Met Glu
   305 310 315 320
   Leu Ser Arg Leu Arg Ser Asp Asp Thr Ala Val Tyr Tyr Cys Ala Arg
   325 330 335
   Ser Pro Asn Pro Tyr Tyr Tyr Asp Ser Ser Gly Tyr Tyr Tyr Pro Gly
   340 345 350
   Ala Phe Asp Ile Trp Gly Gln Gly Thr Met Val Thr Val Ser Ser Ala
   355 360 365
   Ser Thr Lys Gly Pro Ser Val Phe Pro Leu Ala Pro Ser Ser Lys Ser
       370                 375                 380
   Thr Ser Gly Gly Thr Ala Ala Leu Gly Cys Leu Val Lys Asp Tyr Phe
   385                 390                 395                 400
   Pro Glu Pro Val Thr Val Ser Trp Asn Ser Gly Ala Leu Thr Ser Gly
                   405                 410                 415
   Val His Thr Phe Pro Ala Val Leu Gln Ser Ser Gly Leu Tyr Ser Leu
               420                 425                 430
   Ser Ser Val Val Thr Val Pro Ser Ser Ser Leu Gly Thr Gln Thr Tyr
           435                 440                 445
   Ile Cys Asn Val Asn His Lys Pro Ser Asn Thr Lys Val Asp Lys Lys
       450                 455                 460
   Val Glu Pro Lys Ser Cys Gly Ser Gly Gly Gly Ser Asp Ile Gln Met
   465                 470                 475                 480
   Thr Gln Ser Pro Ser Ser Leu Ser Ala Ser Val Gly Asp Arg Val Thr
                   485                 490                 495
   Ile Thr Cys Ser Ala Ser Gln Asp Ile Ser Asn Tyr Leu Asn Trp Tyr
               500                 505                 510
   Gln Gln Lys Pro Gly Lys Ala Pro Lys Val Leu Ile Tyr Phe Thr Ser
           515                 520                 525
   Ser Leu His Ser Gly Val Pro Ser Arg Phe Ser Gly Ser Gly Ser Gly
       530                 535                 540
   Thr Asp Phe Thr Leu Thr Ile Ser Ser Leu Gln Pro Glu Asp Phe Ala
   545                 550                 555                 560
   Thr Tyr Tyr Cys Gln Gln Tyr Ser Thr Val Pro Trp Thr Phe Gly Gln
                   565                 570                 575
   Gly Thr Lys Val Glu Ile Lys Ser Ser Ala Ser Thr Lys Gly Pro Ser
               580                 585                 590
   Val Phe Pro Leu Ala Pro Ser Ser Lys Ser Thr Ser Gly Gly Thr Ala
   595 600 605
   Ala Leu Gly Cys Leu Val Lys Asp Tyr Phe Pro Glu Pro Val Thr Val
   610 615 620
   Ser Trp Asn Ser Gly Ala Leu Thr Ser Gly Val His Thr Phe Pro Ala
   625 630 635 640
   Val Leu Gln Ser Ser Gly Leu Tyr Ser Leu Ser Ser Val Val Thr Val
   645 650 655
   Pro Ser Ser Ser Leu Gly Thr Gln Thr Tyr Ile Cys Asn Val Asn His
   660 665 670
   Lys Pro Ser Asn Thr Lys Val Asp Lys Lys Val Glu Pro Lys Ser Cys
   675 680 685
<210> 39
   <211> 688
   <212> PRT
   <213> Artificial Sequence
<220>
   <223> Fab2-CrossFab heavy chain (HC) including two heavy chains (HC) <Ang-2> with K147E and K213E substitutions coupled to one heavy chain (HC) <VEGF> with VL-VH domain exchange wild type (wt) via glycine-serine-linkers
<400> 39
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser Ala Ser Thr Lys Gly Pro Ser Val Phe Pro Leu Ala Pro Ser Ser
   130 135 140
   Lys Ser Thr Ser Gly Gly Thr Ala Ala Leu Gly Cys Leu Val Glu Asp
   145 150 155 160
   Tyr Phe Pro Glu Pro Val Thr Val Ser Trp Asn Ser Gly Ala Leu Thr
   165 170 175
   Ser Gly Val His Thr Phe Pro Ala Val Leu Gln Ser Ser Gly Leu Tyr
   180 185 190
   Ser Leu Ser Ser Val Val Thr Val Pro Ser Ser Ser Leu Gly Thr Gln
   195 200 205
   Thr Tyr Ile Cys Asn Val Asn His Lys Pro Ser Asn Thr Lys Val Asp
   210 215 220
   Glu Lys Val Glu Pro Lys Ser Cys Ser Gly Gly Gly Gly Ser Gln Val
   225 230 235 240
   Gln Leu Val Glu Ser Gly Ala Glu Val Lys Lys Pro Gly Ala Ser Val
   245 250 255
   Lys Val Ser Cys Lys Ala Ser Gly Tyr Thr Phe Thr Gly Tyr Tyr Met
   260 265 270
   His Trp Val Arg Gln Ala Pro Gly Gln Gly Leu Glu Trp Met Gly Trp
   275 280 285
   Ile Asn Pro Asn Ser Gly Gly Thr Asn Tyr Ala Gln Lys Phe Gln Gly
   290 295 300
   Arg Val Thr Met Thr Arg Asp Thr Ser Ile Ser Thr Ala Tyr Met Glu
   305 310 315 320
   Leu Ser Arg Leu Arg Ser Asp Asp Thr Ala Val Tyr Tyr Cys Ala Arg
   325 330 335
   Ser Pro Asn Pro Tyr Tyr Tyr Asp Ser Ser Gly Tyr Tyr Tyr Pro Gly
   340 345 350
   Ala Phe Asp Ile Trp Gly Gln Gly Thr Met Val Thr Val Ser Ser Ala
   355 360 365
   Ser Thr Lys Gly Pro Ser Val Phe Pro Leu Ala Pro Ser Ser Lys Ser
   370 375 380
   Thr Ser Gly Gly Thr Ala Ala Leu Gly Cys Leu Val Glu Asp Tyr Phe
   385 390 395 400
   Pro Glu Pro Val Thr Val Ser Trp Asn Ser Gly Ala Leu Thr Ser Gly
   405 410 415
   Val His Thr Phe Pro Ala Val Leu Gln Ser Ser Gly Leu Tyr Ser Leu
   420 425 430
   Ser Ser Val Val Thr Val Pro Ser Ser Ser Leu Gly Thr Gln Thr Tyr
   435 440 445
   Ile Cys Asn Val Asn His Lys Pro Ser Asn Thr Lys Val Asp Glu Lys
   450 455 460
   Val Glu Pro Lys Ser Cys Gly Ser Gly Gly Gly Ser Asp Ile Gln Met
   465 470 475 480
   Thr Gln Ser Pro Ser Ser Leu Ser Ala Ser Val Gly Asp Arg Val Thr
   485 490 495
   Ile Thr Cys Ser Ala Ser Gln Asp Ile Ser Asn Tyr Leu Asn Trp Tyr
   500 505 510
   Gln Gln Lys Pro Gly Lys Ala Pro Lys Val Leu Ile Tyr Phe Thr Ser
   515 520 525
   Ser Leu His Ser Gly Val Pro Ser Arg Phe Ser Gly Ser Gly Ser Gly
   530 535 540
   Thr Asp Phe Thr Leu Thr Ile Ser Ser Leu Gln Pro Glu Asp Phe Ala
   545                 550                 555                 560
   Thr Tyr Tyr Cys Gln Gln Tyr Ser Thr Val Pro Trp Thr Phe Gly Gln
                   565                 570                 575
   Gly Thr Lys Val Glu Ile Lys Ser Ser Ala Ser Thr Lys Gly Pro Ser
               580                 585                 590
   Val Phe Pro Leu Ala Pro Ser Ser Lys Ser Thr Ser Gly Gly Thr Ala
           595                 600                 605
   Ala Leu Gly Cys Leu Val Lys Asp Tyr Phe Pro Glu Pro Val Thr Val
       610                 615                 620
   Ser Trp Asn Ser Gly Ala Leu Thr Ser Gly Val His Thr Phe Pro Ala
   625                 630                 635                 640
   Val Leu Gln Ser Ser Gly Leu Tyr Ser Leu Ser Ser Val Val Thr Val
                   645                 650                 655
   Pro Ser Ser Ser Leu Gly Thr Gln Thr Tyr Ile Cys Asn Val Asn His
               660                 665                 670
   Lys Pro Ser Asn Thr Lys Val Asp Lys Lys Val Glu Pro Lys Ser Cys
           675                 680                 685
<210> 40
   <211> 450
   <212> PRT
   <213> Artificial Sequence
<220>
   <223> CrossFab-Fab heavy chain (HC) including one heavy chain (HC) <VEGF> with VL-VH domain exchange wild type (wt) coupled to one heavy chain (HC) <Ang-2> wild type (wt) via glycine-serine-linkers
<400> 40
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Thr Val Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Ser Gly Gly Gly Gly Ser Gln Val Gln Leu Val Glu
   210 215 220
   Ser Gly Ala Glu Val Lys Lys Pro Gly Ala Ser Val Lys Val Ser Cys
   225 230 235 240
   Lys Ala Ser Gly Tyr Thr Phe Thr Gly Tyr Tyr Met His Trp Val Arg
   245 250 255
   Gln Ala Pro Gly Gln Gly Leu Glu Trp Met Gly Trp Ile Asn Pro Asn
   260 265 270
   Ser Gly Gly Thr Asn Tyr Ala Gln Lys Phe Gln Gly Arg Val Thr Met
   275 280 285
   Thr Arg Asp Thr Ser Ile Ser Thr Ala Tyr Met Glu Leu Ser Arg Leu
   290 295 300
   Arg Ser Asp Asp Thr Ala Val Tyr Tyr Cys Ala Arg Ser Pro Asn Pro
   305 310 315 320
   Tyr Tyr Tyr Asp Ser Ser Gly Tyr Tyr Tyr Pro Gly Ala Phe Asp Ile
   325 330 335
   Trp Gly Gln Gly Thr Met Val Thr Val Ser Ser Ala Ser Thr Lys Gly
   340 345 350
   Pro Ser Val Phe Pro Leu Ala Pro Ser Ser Lys Ser Thr Ser Gly Gly
   355 360 365
   Thr Ala Ala Leu Gly Cys Leu Val Lys Asp Tyr Phe Pro Glu Pro Val
   370 375 380
   Thr Val Ser Trp Asn Ser Gly Ala Leu Thr Ser Gly Val His Thr Phe
   385 390 395 400
   Pro Ala Val Leu Gln Ser Ser Gly Leu Tyr Ser Leu Ser Ser Val Val
   405 410 415
   Thr Val Pro Ser Ser Ser Leu Gly Thr Gln Thr Tyr Ile Cys Asn Val
   420 425 430
   Asn His Lys Pro Ser Asn Thr Lys Val Asp Lys Lys Val Glu Pro Lys
   435 440 445
   Ser Cys
   450
<210> 41
   <211> 450
   <212> PRT
   <213> Artificial Sequence
<220>
   <223> CrossFab-Fab heavy chain (HC) including one heavy chain (HC) <VEGF> with VL-VH domain exchange wild type (wt) coupled to one heavy chain (HC) <Ang-2> with K147E and K213E substitutions via glycine-serine-linkers
<400> 41
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Ser Gly Gly Gly Gly Ser Gln Val Gln Leu Val Glu
       210                 215                 220
   Ser Gly Ala Glu Val Lys Lys Pro Gly Ala Ser Val Lys Val Ser Cys
   225                 230                 235                 240
   Lys Ala Ser Gly Tyr Thr Phe Thr Gly Tyr Tyr Met His Trp Val Arg
                   245                 250                 255
   Gln Ala Pro Gly Gln Gly Leu Glu Trp Met Gly Trp Ile Asn Pro Asn
               260                 265                 270
   Ser Gly Gly Thr Asn Tyr Ala Gln Lys Phe Gln Gly Arg Val Thr Met
           275                 280                 285
   Thr Arg Asp Thr Ser Ile Ser Thr Ala Tyr Met Glu Leu Ser Arg Leu
       290                 295                 300
   Arg Ser Asp Asp Thr Ala Val Tyr Tyr Cys Ala Arg Ser Pro Asn Pro
   305                 310                 315                 320
   Tyr Tyr Tyr Asp Ser Ser Gly Tyr Tyr Tyr Pro Gly Ala Phe Asp Ile
                   325                 330                 335
   Trp Gly Gln Gly Thr Met Val Thr Val Ser Ser Ala Ser Thr Lys Gly
               340                 345                 350
   Pro Ser Val Phe Pro Leu Ala Pro Ser Ser Lys Ser Thr Ser Gly Gly
           355                 360                 365
   Thr Ala Ala Leu Gly Cys Leu Val Glu Asp Tyr Phe Pro Glu Pro Val
       370                 375                 380
   Thr Val Ser Trp Asn Ser Gly Ala Leu Thr Ser Gly Val His Thr Phe
   385                 390                 395                 400
   Pro Ala Val Leu Gln Ser Ser Gly Leu Tyr Ser Leu Ser Ser Val Val
                   405                 410                 415
   Thr Val Pro Ser Ser Ser Leu Gly Thr Gln Thr Tyr Ile Cys Asn Val
               420                 425                 430
   Asn His Lys Pro Ser Asn Thr Lys Val Asp Glu Lys Val Glu Pro Lys
           435                 440                 445
   Ser Cys
   450
<210> 42
   <211> 667
   <212> PRT
   <213> Artificial Sequence
<220>
   <223> CrossFab2-Fab heavy chain (HC) including two heavy chains (HC) <VEGF> with VL-VH domain exchange wild type (wt) coupled to one heavy chain (HC) <Ang-2> wild type (wt) via glycine-serinee-linkers
<400> 42
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Ser Gly Gly Gly Gly Ser Asp Ile Gln Met Thr Gln
   210 215 220
   Ser Pro Ser Ser Leu Ser Ala Ser Val Gly Asp Arg Val Thr Ile Thr
   225 230 235 240
   Cys Ser Ala Ser Gln Asp Ile Ser Asn Tyr Leu Asn Trp Tyr Gln Gln
   245 250 255
   Lys Pro Gly Lys Ala Pro Lys Val Leu Ile Tyr Phe Thr Ser Ser Leu
   260 265 270
   His Ser Gly Val Pro Ser Arg Phe Ser Gly Ser Gly Ser Gly Thr Asp
   275 280 285
   Phe Thr Leu Thr Ile Ser Ser Leu Gln Pro Glu Asp Phe Ala Thr Tyr
   290 295 300
   Tyr Cys Gln Gln Tyr Ser Thr Val Pro Trp Thr Phe Gly Gln Gly Thr
   305 310 315 320
   Lys Val Glu Ile Lys Ser Ser Ala Ser Thr Lys Gly Pro Ser Val Phe
   325 330 335
   Pro Leu Ala Pro Ser Ser Lys Ser Thr Ser Gly Gly Thr Ala Ala Leu
   340 345 350
   Gly Cys Leu Val Lys Asp Tyr Phe Pro Glu Pro Val Thr Val Ser Trp
   355 360 365
   Asn Ser Gly Ala Leu Thr Ser Gly Val His Thr Phe Pro Ala Val Leu
   370 375 380
   Gln Ser Ser Gly Leu Tyr Ser Leu Ser Ser Val Val Thr Val Pro Ser
   385 390 395 400
   Ser Ser Leu Gly Thr Gln Thr Tyr Ile Cys Asn Val Asn His Lys Pro
                   405                 410                 415
   Ser Asn Thr Lys Val Asp Lys Lys Val Glu Pro Lys Ser Cys Gly Gly
               420                 425                 430
   Gly Gly Ser Gln Val Gln Leu Val Glu Ser Gly Ala Glu Val Lys Lys
           435                 440                 445
   Pro Gly Ala Ser Val Lys Val Ser Cys Lys Ala Ser Gly Tyr Thr Phe
       450                 455                 460
   Thr Gly Tyr Tyr Met His Trp Val Arg Gln Ala Pro Gly Gln Gly Leu
   465                 470                 475                 480
   Glu Trp Met Gly Trp Ile Asn Pro Asn Ser Gly Gly Thr Asn Tyr Ala
                   485                 490                 495
   Gln Lys Phe Gln Gly Arg Val Thr Met Thr Arg Asp Thr Ser Ile Ser
               500                 505                 510
   Thr Ala Tyr Met Glu Leu Ser Arg Leu Arg Ser Asp Asp Thr Ala Val
           515                 520                 525
   Tyr Tyr Cys Ala Arg Ser Pro Asn Pro Tyr Tyr Tyr Asp Ser Ser Gly
       530                 535                 540
   Tyr Tyr Tyr Pro Gly Ala Phe Asp Ile Trp Gly Gln Gly Thr Met Val
   545                 550                 555                 560
   Thr Val Ser Ser Ala Ser Thr Lys Gly Pro Ser Val Phe Pro Leu Ala
                   565                 570                 575
   Pro Ser Ser Lys Ser Thr Ser Gly Gly Thr Ala Ala Leu Gly Cys Leu
               580                 585                 590
   Val Lys Asp Tyr Phe Pro Glu Pro Val Thr Val Ser Trp Asn Ser Gly
           595                 600                 605
   Ala Leu Thr Ser Gly Val His Thr Phe Pro Ala Val Leu Gln Ser Ser
       610                 615                 620
   Gly Leu Tyr Ser Leu Ser Ser Val Val Thr Val Pro Ser Ser Ser Leu
   625                 630                 635                 640
   Gly Thr Gln Thr Tyr Ile Cys Asn Val Asn His Lys Pro Ser Asn Thr
   645 650 655
   Lys Val Asp Lys Lys Val Glu Pro Lys Ser Cys
   660 665
<210> 43
   <211> 667
   <212> PRT
   <213> Artificial Sequence
<220>
   <223> CrossFab2-Fab heavy chain (HC) including two heavy chains (HC) <VEGF> with VL-VH domain exchange wild type (wt) coupled to one heavy chain (HC) <Ang-2> with K147E and K231E substitutions via glycine-serine-linkers
<400> 43
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Thr Val Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Ser Gly Gly Gly Gly Ser Asp Ile Gln Met Thr Gln
   210 215 220
   Ser Pro Ser Ser Leu Ser Ala Ser Val Gly Asp Arg Val Thr Ile Thr
   225 230 235 240
   Cys Ser Ala Ser Gln Asp Ile Ser Asn Tyr Leu Asn Trp Tyr Gln Gln
   245 250 255
   Lys Pro Gly Lys Ala Pro Lys Val Leu Ile Tyr Phe Thr Ser Ser Leu
   260 265 270
   His Ser Gly Val Pro Ser Arg Phe Ser Gly Ser Gly Ser Gly Thr Asp
   275 280 285
   Phe Thr Leu Thr Ile Ser Ser Leu Gln Pro Glu Asp Phe Ala Thr Tyr
   290 295 300
   Tyr Cys Gln Gln Tyr Ser Thr Val Pro Trp Thr Phe Gly Gln Gly Thr
   305 310 315 320
   Lys Val Glu Ile Lys Ser Ser Ala Ser Thr Lys Gly Pro Ser Val Phe
   325 330 335
   Pro Leu Ala Pro Ser Ser Lys Ser Thr Ser Gly Gly Thr Ala Ala Leu
   340 345 350
   Gly Cys Leu Val Lys Asp Tyr Phe Pro Glu Pro Val Thr Val Ser Trp
   355 360 365
   Asn Ser Gly Ala Leu Thr Ser Gly Val His Thr Phe Pro Ala Val Leu
       370                 375                 380
   Gln Ser Ser Gly Leu Tyr Ser Leu Ser Ser Val Val Thr Val Pro Ser
   385                 390                 395                 400
   Ser Ser Leu Gly Thr Gln Thr Tyr Ile Cys Asn Val Asn His Lys Pro
                   405                 410                 415
   Ser Asn Thr Lys Val Asp Lys Lys Val Glu Pro Lys Ser Cys Gly Gly
               420                 425                 430
   Gly Gly Ser Gln Val Gln Leu Val Glu Ser Gly Ala Glu Val Lys Lys
           435                 440                 445
   Pro Gly Ala Ser Val Lys Val Ser Cys Lys Ala Ser Gly Tyr Thr Phe
       450                 455                 460
   Thr Gly Tyr Tyr Met His Trp Val Arg Gln Ala Pro Gly Gln Gly Leu
   465                 470                 475                 480
   Glu Trp Met Gly Trp Ile Asn Pro Asn Ser Gly Gly Thr Asn Tyr Ala
                   485                 490                 495
   Gln Lys Phe Gln Gly Arg Val Thr Met Thr Arg Asp Thr Ser Ile Ser
               500                 505                 510
   Thr Ala Tyr Met Glu Leu Ser Arg Leu Arg Ser Asp Asp Thr Ala Val
           515                 520                 525
   Tyr Tyr Cys Ala Arg Ser Pro Asn Pro Tyr Tyr Tyr Asp Ser Ser Gly
       530                 535                 540
   Tyr Tyr Tyr Pro Gly Ala Phe Asp Ile Trp Gly Gln Gly Thr Met Val
   545                 550                 555                 560
   Thr Val Ser Ser Ala Ser Thr Lys Gly Pro Ser Val Phe Pro Leu Ala
                   565                 570                 575
   Pro Ser Ser Lys Ser Thr Ser Gly Gly Thr Ala Ala Leu Gly Cys Leu
               580                 585                 590
   Val Glu Asp Tyr Phe Pro Glu Pro Val Thr Val Ser Trp Asn Ser Gly
           595                 600                 605
   Ala Leu Thr Ser Gly Val His Thr Phe Pro Ala Val Leu Gln Ser Ser
   610 615 620
   Gly Leu Tyr Ser Leu Ser Ser Val Val Thr Val Pro Ser Ser Ser Leu
   625 630 635 640
   Gly Thr Gln Thr Tyr Ile Cys Asn Val Asn His Lys Pro Ser Asn Thr
   645 650 655
   Lys Val Asp Glu Lys Val Glu Pro Lys Ser Cys
   660 665
<210> 44
   <211> 439
   <212> PRT
   <213> Artificial Sequence
<220>
   <223> heavy chain (HC) <VEGF> with VH-VL exchange with K147E substitution
<400> 44
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Thr Val Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Glu Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
           275                 280                 285
   Asn Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Ala Leu
   305                 310                 315                 320
   Pro Ala Pro Ile Glu Lys Thr Ile Ser Lys Ala Lys Gly Gln Pro Arg
                   325                 330                 335
   Glu Pro Gln Val Cys Thr Leu Pro Pro Ser Arg Asp Glu Leu Thr Lys
               340                 345                 350
   Asn Gln Val Ser Leu Ser Cys Ala Val Lys Gly Phe Tyr Pro Ser Asp
   355 360 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Val Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro Gly Lys
   435
<210> 45
   <211> 230
   <212> PRT
   <213> Artificial Sequence
<220>
   <223> light chain (LC) <VEGF> with VH-VL exchange with Q124K substitution
<400> 45
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
   20 25 30
   Gly Met Asn Trp Val Arg Gln Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Ser Leu Arg Ala Glu Asp Thr Ala Val Tyr Tyr Cys
   85 90 95
   Ala Lys Tyr Pro His Tyr Tyr Gly Ser Ser His Trp Tyr Phe Asp Val
   100 105 110
   Trp Gly Gln Gly Thr Leu Val Thr Val Ser Ser Ala Ser Val Ala Ala
   115 120 125
   Pro Ser Val Phe Ile Phe Pro Pro Ser Asp Glu Lys Leu Lys Ser Gly
   130 135 140
   Thr Ala Ser Val Val Cys Leu Leu Asn Asn Phe Tyr Pro Arg Glu Ala
   145 150 155 160
   Lys Val Gln Trp Lys Val Asp Asn Ala Leu Gln Ser Gly Asn Ser Gln
   165 170 175
   Glu Ser Val Thr Glu Gln Asp Ser Lys Asp Ser Thr Tyr Ser Leu Ser
   180 185 190
   Ser Thr Leu Thr Leu Ser Lys Ala Asp Tyr Glu Lys His Lys Val Tyr
   195 200 205
   Ala Cys Glu Val Thr His Gln Gly Leu Ser Ser Pro Val Thr Lys Ser
   210 215 220
   Phe Asn Arg Gly Glu Cys
   225 230
<210> 46
   <211> 439
   <212> PRT
   <213> Artificial Sequence
<220>
   <223> heavy chain (HC) <VEGF> with VH-VL exchange with K147E, and K213E substitution
<400> 46
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Glu Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Glu Lys Val Glu
           195                 200                 205
   Pro Lys Ser Cys Asp Lys Thr His Thr Cys Pro Pro Cys Pro Ala Pro
       210                 215                 220
   Glu Leu Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser His Glu Asp Pro Glu Val Lys Phe Asn Trp Tyr Val Asp
               260                 265                 270
   Gly Val Glu Val His Asn Ala Lys Thr Lys Pro Arg Glu Glu Gln Tyr
   275 280 285
   Asn Ser Thr Tyr Arg Val Val Ser Val Leu Thr Val Leu His Gln Asp
   290 295 300
   Trp Leu Asn Gly Lys Glu Tyr Lys Cys Lys Val Ser Asn Lys Ala Leu
   305 310 315 320
   Pro Ala Pro Ile Glu Lys Thr Ile Ser Lys Ala Lys Gly Gln Pro Arg
   325 330 335
   Glu Pro Gln Val Cys Thr Leu Pro Pro Ser Arg Asp Glu Leu Thr Lys
   340 345 350
   Asn Gln Val Ser Leu Ser Cys Ala Val Lys Gly Phe Tyr Pro Ser Asp
   355 360 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Val Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro Gly Lys
   435
<210> 47
   <211> 230
   <212> PRT
   <213> Artificial Sequence
<220>
   <223> light chain (LC) <VEGF> with VH-VL exchange with E123K, and Q124K substitution
<400> 47
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Leu Val Thr Val Ser Ser Ala Ser Val Ala Ala
           115                 120                 125
   Pro Ser Val Phe Ile Phe Pro Pro Ser Asp Lys Lys Leu Lys Ser Gly
       130                 135                 140
   Thr Ala Ser Val Val Cys Leu Leu Asn Asn Phe Tyr Pro Arg Glu Ala
   145                 150                 155                 160
   Lys Val Gln Trp Lys Val Asp Asn Ala Leu Gln Ser Gly Asn Ser Gln
                   165                 170                 175
   Glu Ser Val Thr Glu Gln Asp Ser Lys Asp Ser Thr Tyr Ser Leu Ser
               180                 185                 190
   Ser Thr Leu Thr Leu Ser Lys Ala Asp Tyr Glu Lys His Lys Val Tyr
           195                 200                 205
   Ala Cys Glu Val Thr His Gln Gly Leu Ser Ser Pro Val Thr Lys Ser
       210                 215                 220
   Phe Asn Arg Gly Glu Cys
   225                 230